# EUROPÄISCHE PATENTSCHRIFT

(11) **EP 0 691 950 B1**
(45) Veröffentlichungstag und Bekanntmachung des Hinweises auf die Patenterteilung: **03.09.1997**
(21) Anmeldenummer: 94912490.3
(22) Anmeldetag: 19.03.1994
(51) Int. Cl.: C07C 251/48, C07C 251/86, C07D 217/22, A01N 37/50

(54) **IMINO-SUBSTITUIERTE PHENYLESSIGSÄUREAMIDE, IHRE HERSTELLUNG UND DIESE ENTHALTENDE FUNGIZIDE**
IMINO-SUBSTITUTED PHENYLACETIC ACID AMIDES, THEIR PREPARATION AND FUNGICIDES CONTAINING THEM
AMIDES D'ACIDE PHENYLACETIQUE IMINO-SUBSTITUES, LEUR PREPARATION ET FONGICIDES LES CONTENANT

(30) Priorität: 29.03.1993 DE 4310143
(43) Veröffentlichungstag der Anmeldung: 17.01.1996
(73) Patentinhaber: BASF AKTIENGESELLSCHAFT, 67056 Ludwigshafen (DE)
(72) Erfinder: DOETZER, Reinhard, D-69469 Weinheim (DE); GRAMMENOS, Wassilios, D-67063 Ludwigshafen (DE); SAUTER, Hubert, D-68167 Mannheim (DE); HARREUS, Albrecht, D-67063 Ludwigshafen (DE); WINGERT, Horst, D-68159 Mannheim (DE); AMMERMANN, Eberhard, D-64646 Heppenheim (DE); LORENZ, Gisela, D-67434 Neustadt (DE)
(86) Internationale Anmeldenummer: EP9400877
(87) Internationale Veröffentlichungsnummer: WO9422812

(56) Entgegenhaltungen:
- EP-A- 0 331 061
- EP-A- 0 398 692
- EP-A- 0 463 488
- EP-A- 0 477 631
- EP-A- 0 499 823
- WO-A-92/13830

## Beschreibung

Die vorliegende Erfindung betrifft substituierte Phenylessigsäureamide der Formel I 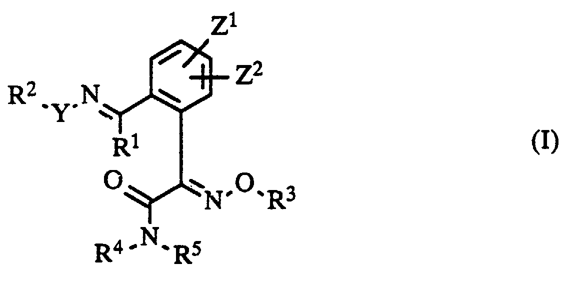 in der die Substituenten die folgende Bedeutung haben:
Y
   Sauerstoff oder die Gruppe NR⁶;
Z¹ und Z²
   unabhängig voneinander Wasserstoff, Halogen, Alkyl, Alkenyl, Alkinyl, Alkoxy, Alkenyloxy, Halogenalkyl, Halogenalkoxy, Halogenalkenyloxy, Cyano oder Nitro;
R¹
   Wasserstoff, Alkyl, Halogenalkyl oder Aryl;
R²
   Wasserstoff, oder ggf. subst. Alkyl, Alkenyl, Alkinyl, Cycloalkyl, Heteroarylalkyl, Heterocyclyl, Cycloalkenyl, Arylalkyl, Aryl, Heteroaryl, Arylalkenyl, Heteroarylalkenyl, Aryloxyalkyl, Heteroaryloxyalkyl, Acyl, Arylcarbonyl, Heteroarylcarbonyl oder Alkoxycarbonyl;
R³, R⁴, R⁵ und R⁶
   unabhängig voneinander Wasserstoff oder Alkyl;
oder R⁶ und R² gemeinsam mit dem Stickstoff, dessen Substituenten sie sind, einen Ring bedeuten können.

Außerdem betrifft die Erfindung Verfahren und Zwischenprodukte zur Herstellung dieser Verbindungen.

Es ist bekannt, iminosubstituierte Phenylessigsäurederivate als Fungizide (EP 499 823) zu verwenden. Ihre Wirkung ist jedoch in vielen Fällen unbefriedigend.

Der vorliegenden Erfindung lagen daher Verbindungen mit verbesserter Wirkung und verbreitetem Wirkungsspektrum als Aufgabe zugrunde.

Demgemäß wurden die eingangs definierten Verbindungen I gefunden.

Außerdem wurde gefunden, daß Verbindungen der Formel I eine gute insektizide, nematizide und akarizide Wirkung haben. Die fungizide Wirkung wird bevorzugt.

Im Hinblick auf ihre biologische Wirksamkeit zur Bekämpfung von Schadpilzen kommen Verbindungen I in Betracht, in denen die Substituenten z.B. die folgende Bedeutung haben:
Z¹ und Z²
   Wasserstoff, Halogen wie Fluor, Chlor, Brom und Iod oder z.B. Methyl, Methoxy, Cyano und Nitro;
R³, R⁴, R⁵ und R⁶ Wasserstoff
   C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylphenyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
R¹
   Wasserstoff
   C₁-C₄-Alkyl wie Methyl, Ethyl, Propyl, 1-Methylethyl, Butyl, 1-Methylpropyl, 2-Methylpropyl und 1,1-Dimethylethyl;
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
   ggf. subst. Aryl wie Phenyl, Naphthyl und Anthryl, bevorzugt Phenyl und Naphthyl, insbesondere Phenyl;
R²
   Wasserstoff;
   C₁-C₄-Alkyl wie vorstehend genannt; ggf. subst. C₃-C₁₅-Alkenyl besonders C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl;
   ggf. subst. C₃-C₈-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl und 1-Ethyl-1-methyl-2-propinyl;
   ggf. subst. C₃-C₈-Cycloalkyl wie Cyclopropyl, Cyclobutyl, Cyclopentyl, Cyclohexyl, Cycloheptyl und Cyclooctyl;
   C₁-C₄-Alkoxycarbonyl-C₁-C₄-Alkyl wie Methoxycarbonylmethyl, Ethoxycarbonylmethyl, Propyloxycarbonylmethyl, 1-Methylethyloxycarbonylmethyl, Butyloxycarbonylmethyl, 1-Methylpropyloxycarbonylmethyl und 1,1-Dimethylethyloxycarbonylmethyl;
   ggf. subst. C₅-C₈-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-1-enyl;
   ggf. subst. Aryl (z.B. Phenyl, Naphthyl, Anthryl);
   ggf. subst. Aryl-C₁-C₄-alkyl (z.B. Benzyl, 1-Phenethyl, 2-Phenethyl, 1-Phenylpropyl, 2-Phenylpropyl, 3-Phenpropyl, 2-Methyl-3-Phenylpropyl, 2-Methyl-2-Phenylpropyl, 4-Phenylbutyl);
   ggf. subst. Acyl, z.B. C₁-C₄-Alkylcarbonyl wie Acetyl, Propionyl, Butyryl, Phenylacetyl und Chloracetyl;
   ggf. subst. Arylcarbonyl wie Benzoyl und 2,4-Dichlorbenzoyl;
   ggf. subst. Heteroarylcarbonyl wie 2-Pyridylcarbonyl;
   ggf. subst. Aryl-C₁-C₄-alkenyl (z.B. Phenyl-1-ethenyl, 2-Phenyl-1-propenyl, 2,2-Diphenylethenyl, 1-Phenyl-1-propen-2-yl und 1-Phenylbutenyl);
   ggf. subst. Aryloxy-C₁-C₄-Alkyl wie Phenoxymethyl, Phenoxyethyl und Phenoxypropyl;
   ggf. subst. Heteroaryl (z.B. 2-Pyridyl, 3-pyridyl, 4-Pyridyl, 3-Pyridazinyl, 4-Pyridazinyl, 1-Pyrazolyl, 3-Pyrazolyl, 4-Pyrazolyl, 2-Pyrazinyl, 2-Pyrimidyl, 4-Pyrimidyl, 5-Pyrimidyl, 1-Indazolyl, 3-Indazolyl, 2-Furyl, 3-Furyl, 1-Pyrrolyl, 2-Pyrrolyl, 3-Pyrrolyl, 2-Thiophenyl, 3-Thiophenyl, 1-Imidazolyl, 2-Imidazolyl, 4-Imidazolyl, 5-Imidazolyl, 2-Isoindolyl, 1-Indolyl, 1-Indazolyl, 1,2-Benzothiazol-3-yl, 1,3-Benzothiazol-2-yl, 3-Isoxazolyl, 4-Isoxazolyl, 5-Isoxazolyl, 3-Isothiazolyl, 4-Isothiazolyl, 5-Isothiazolyl, 1,2-Benzoxazol-3-yl, 1,3-Benzoxazol-2-yl, 1,2,4-Triazolyl, 1,3,4-Triazolyl, 7-Purinyl, 2-Chinolyl, 3-Chinolyl, 4-Chinolyl, 1-Isochinolyl, 3-Isochinolyl, 4-Isochinolyl, 2-Benzofuranyl, 3-Benzofuranyl, 1-Isobenzofuranyl, 1,2,4-Triazin-3-yl und 1,3,5-Triazin-2-yl;
   ggf. subst. Heteroaryl-C₁-C₄-alkyl (z.B. 2-Pyridylmethyl, 3-Pyridylmethyl);
   ggf. subst. Heteroaryl-C₂-C₄-alkenyl (z.B. 2'-Furyl-2-ethenyl, 2'-Thienyl-2-ethenyl, 3'-Pyridyl-2-ethenyl):
   ggf. subst. Heterocyclyl (z.B. Oxiranyl, 1-Aziridinyl, 1-Azetidinyl, 1-Pyrrolidinyl, 2-Tetrahydrofuranyl, 2-Tetrahydropyranyl, 3-Tetrahydropyranyl, 1-Piperidinyl, 1-Morpholinyl, 1-Piperazinyl, 1,3-Dioxanyl, 3-Tetrahydrothiopyranyl);
   Die mit "ggf. substituiert" bezeichneten Reste enthalten neben Wasserstoff z.B. die folgenden Reste: Halogen wie Fluor, Chlor, Brom und Jod;
   C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
   C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 3-Methylbutyloxy, 1,1-Dimethylpropyloxy, 1,2-Dimethylpropyloxy, 2,2-Dimethylpropyloxy, 1-Ethylpropyloxy, Hexyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
   C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
   C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
   C₂-C₆-Alkenyloxy wie Ethenyloxy, 1-Propenyloxy, 2-Propenyloxy, 1-Methylethenyloxy, 1-Butenyloxy, 2-Butenyloxy, 3-Butenyloxy, 1-Methyl-1-propenyloxy, 2-Methyl-1-propenyloxy, 1-Methyl-2-propenyloxy, 2-Methyl-2-propenyloxy, 1-Pentenyloxy, 2-Pentenyloxy, 3-Pentenyloxy, 4-Pentenyloxy, 1-Methyl-1-butenyloxy, 2-Methyl-1-butenyloxy, 3-Methyl-1-butenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyl-1-pentenyloxy, 2-Methyl-1-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-2-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy;
   Cyano; Cyanato, Thiocyanato; Nitro; Amino; Hydroxy; Carboxyl;
   Di-C₁-C₆-alkylamino, besonders Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)-amino, N-Butyl-N-ethylamino, N-Ethyl-N-(1-methylpropyl)amino, N-Ethyl-N-(2-methylpropyl)amino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Di-methylethyl)-N-(1-methylethyl)amino, N-Butyl-N-(1-methyl-propyl)amino, N-Butyl-N-(2-methylpropyl)-amino, N-Butyl-N-(1,1-dimethylethyl)amino, N-(1-Methylpropyl)-N-(2-methyl-propyl)amino, N-(1,1-Dimethylethyl)-N-(1-methylpropyl)amino und N-(1,1-Dimethylethyl)-N-(2-methylpropyl)amino;
   Phenyl, Phenoxy, Phenylthio und Phenylamino, welche ihrerseits ein bis fünf Halogenatome wie vorstehend genannt;
   und/oder eine bis fünf C₁-C₆-Alkoxygruppen wie vorstehend genannt;
   und/oder eine bis fünf C₁-C₆-Alkylthiogruppen wie vorstehend genannt;
   und/oder eine bis fünf C₁-C₄-Halogenalkylgruppen wie vorstehend genannt;
   und/oder eine bis vier C₁-C₆-Alkoxyiminomethylgruppen wie Methoxyiminomethyl, Ethoxyiminomethyl, n-Propoxyiminomethyl, n-Butoxyiminomethyl, n-Pentoxyiminomethyl, n-Hexoxyiminomethyl, Allyloxyiminomethyl, Benzyloxyiminomethyl, iso-Propoxyiminomethyl, iso-Butoxyiminomethyl und tert.-Butoxyiminomethyl, und/oder eine bis vier C₁-C₆-Alkoxyiminoethylgruppen wie Methoxyiminoethyl, Ethoxyimino-1-ethyl, n-Propoxyimino-1-ethyl, n-Butoxyimino-1-ethyl, n-Pentoxyimino-1-ethyl, n-Hexoxyimino-1-ethyl, Allyloxyimino-1-ethyl, Benzyloxyimino-1-ethyl; und/oder Phenyl, Phenoxy und Benzyloxy;
   und/oder eine C₁-C₄-Alkyliminomethylgruppe wie Methyliminomethyl und Ethyliminomethyl; und/oder eine bis vier C₃-C₆-Cycloalkylgruppen wie Cyclopropyl, Cyclobutyl, Cyclopentyl und Cyclohexyl, tragen können.

Die neuen Verbindungen der allgemeinen Formel I können bei der Herstellung aufgrund der C=C- bzw. C=N-Doppelbindungen als E/Z-Isomerengemische anfallen. Diese können in üblicher Weise durch Kristallisation oder Chromatographie in die einzelnen Komponenten aufgetrennt werden.

Sowohl die einzelnen isomeren Verbindungen als auch ihre Gemische werden von der Erfindung umfaßt und sind als Fungizide brauchbar.

Besonders bevorzugt sind Verbindungen der Formel I, in der
Y
   Sauerstoff oder die Gruppe N-R⁶ bedeutet,
Z¹ und Z²
   unabhängig voneinander Wasserstoff, Methyl, Methoxy, Chlor, Brom oder Trifluormethyl bedeuten,
R¹
   Wasserstoff, Methyl oder Trifluormethyl bedeutet,
R²
   Wasserstoff, oder ggf. subst. Alkyl, Alkenyl, Alkinyl, Cycloalkyl, Arylalkyl, Heteroarylalkyl, Aryloxyalkyl oder Heteroaryloxyalkyl bedeutet
   und
R³, R⁴, R⁵ und R⁶
   Wasserstoff, Methyl oder Ethyl bedeuten.

Außerdem werden Verbindungen der Formel I bevorzugt, in denen
Y Sauerstoff bedeutet,
Z¹ und Z² für Wasserstoff stehen,
R³ und R⁴ für Methyl stehen,
R⁵ Wasserstoff bedeutet
und
in der
R¹ Wasserstoff oder Methyl und
R¹ Wasserstoff, Alkyl, Allyl, Propargyl, Arylalkyl, Heteroarylalkyl, Aryloxyalkyl oder Heteroaryloxyalkyl bedeutet.

Des weiteren werden Verbindungen I bevorzugt, in der R¹ Wasserstoff und R² (3-Trifluormethyl-)benzyl bedeutet.

Daneben werden Verbindungen I bevorzugt, in der R¹ Methyl und R² (3-Trifluormethyl-)benzyl bedeutet.

Die Herstellung der Verbindungen der allgemeinen Formel I gemäß Anspruch 1 erfolgt beispielsweise wie in Schema 1 beschrieben (Z¹, Z² bedeutet Wasserstoff).

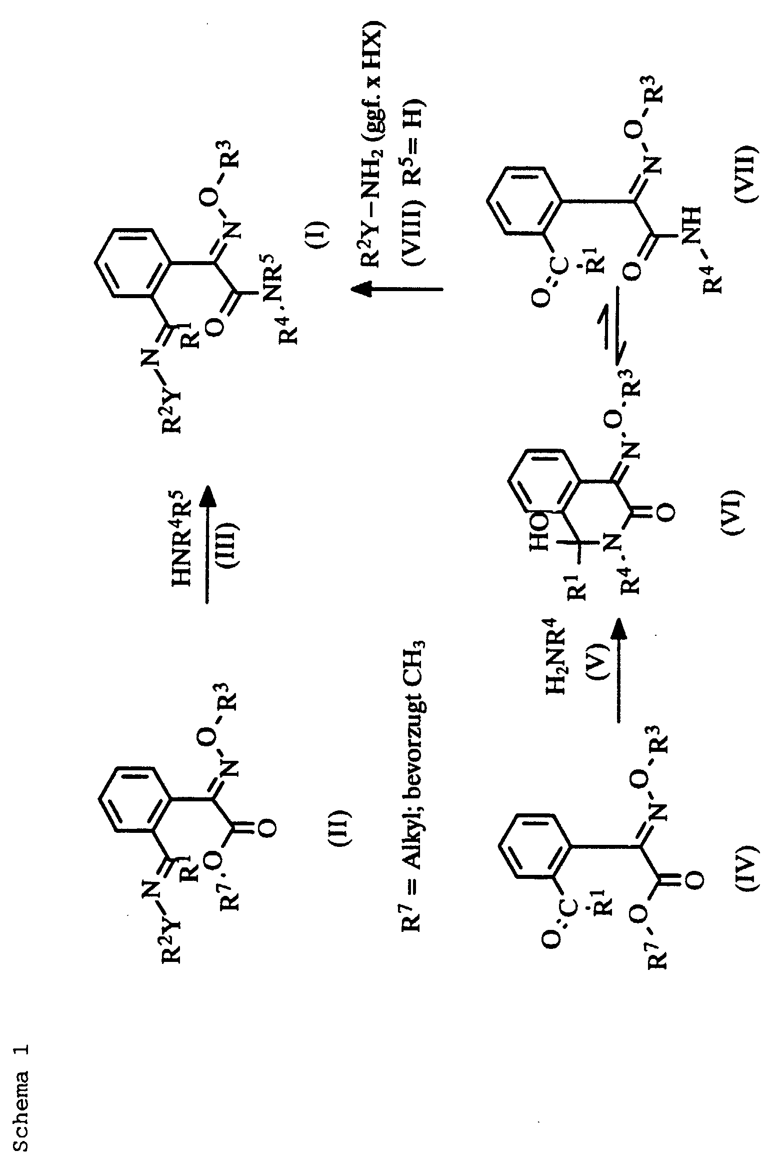

Die Verbindungen der allgemeinen Formel I lassen sich beispielsweise aus den Estern der allgemeinen Formel II (Herstellung bekannt aus EP 499 823) herstellen, indem man II mit Ammoniak oder Aminen (III) umsetzt.

Verbindungen der allgemeinen Formel I, in denen R⁴ H bedeutet, lassen sich darstellen, indem man eine Carbonylverbindung der allgemeinen Formel IV (Darstellung s. EP 393 428, R¹ = H und EP 499 823, R¹ verschieden von H) mit einem Monoalkylamin V zu einem cyclischen Halbaminal VI umsetzt, welches mit seiner Aldehyd- bzw. Ketoform VII im Gleichgewicht steht und mit einem Hydroxylamin- bzw. Hydrazinderivat VIII oder einem ihrer Säureadditionssalze als Carbonylverbindung VII reagiert, wobei sich die Substanzen der allgemeinen Formel I (mit R⁴ = Alkyl und R⁵ = H) bilden.

Die Zwischenprodukte der Formel VI bzw. VII können auch dargestellt werden aus der Verbindung X (Schema 2), (die in an sich bekannter Weise aus Phenylglyoxylsäureestern durch Umsetzung mit Alkoxyamin-Hydrochlorid und Monoalkylamin zugänglich ist,) indem man X zweifach metalliert (zu XI), wozu Metallalkyle wie Butyllithium (J. Org. Chem. 29 (1964), 853) oder Metallamide wie z.B. Magnesium-diisopropylamid (EP 418 013) geeignet sind, und XI mit einem Carbonsäurederivat XII, z.B. einem Carbonsäurechlorid, -anhydrid oder -ester umsetzt oder XI mit einem Aldehyd XIII in das Carbinol XIV überführt, der anschließend zur Carbonylverbindung VII (bzw. ihrer cyclischen Form VI) in an sich bekannter Weise oxidiert wird.

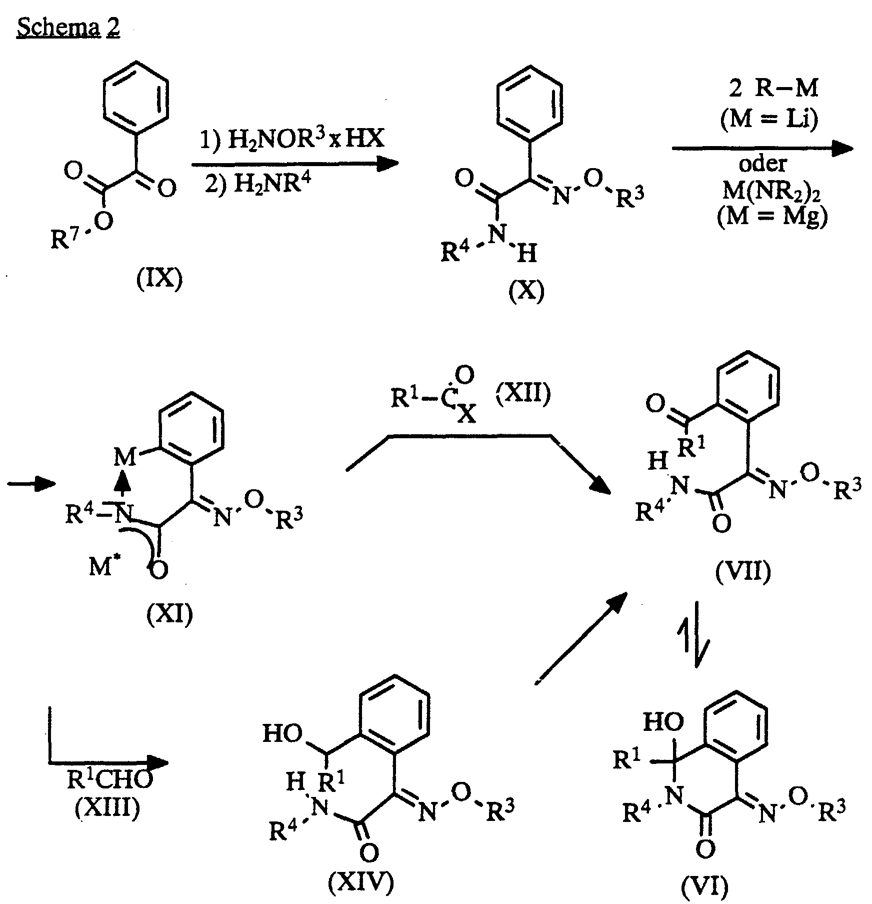

Hydroxylaminderivate der allgemeinen Formel VIII (Y = O) sind entweder bekannt oder können nach bekannten Methoden analog Houben-Weyl X/1, S. 1192 und 1183, hergestellt werden.

Hydrazinderivate der allgemeinen Formel VIII (Y = NR⁶) sind entweder bekannt oder können, ebenso nach bekannten Methoden, analog Houben-Weyl X/2, S. 271, 280 und 740 hergestellt werden.

Amine (III und V), Phenylglyoxylsäureester (IX), Carbonsäurederivate (XII) und Aldehyde (XIII) sind allgemein bekannt.

Die folgenden Vorschriften und Beispiele sollen die Herstellung der neuen Wirkstoffe und Zwischenprodukte erläutern.

### Beispiel 1

a) Darstellung von 1-Hydroxy-2-methyl-4-methoxyimino-1,2,3,4-tetrahydroisochinolin-3-on
   (Verbindung der Formel VI in Schema 1; R¹=H, R³=R⁴=CH₃)
   11 ,0 g (50 mmol) des Aldehyds der Formel IV [(R¹=H, R³=R⁷=CH₃), Darstellung s. EP 393 428)] werden in 200 ml THF gelöst und mit 10 ml 40 %iger wäßriger Methylaminlösung (112 mmol) 30 min bei 25°C gerührt. Die entstandene Verbindung VI fällt teilweise aus; der Niederschlag wird abgesaugt, mit Ether gewaschen und getrocknet. Weiteres Produkt wird durch Einengen der Mutterlauge, Verreiben des Rückstands mit Diisopropylether und Absaugen gewonnen. Farblose Kristalle, Smp. 116 - 168°C, Ausbeute 9,0 g (82 % d. Th.).
   ¹H-NMR (CDCl₃,D-Skala): 2,2 - 2,5.(br, OH), 3,26 (s, 3H, NCH₃); 4.06 (s, 3H, OCH₃); 4,06 (s, 3H, OCH₃); 5,71 (s, 1H, CH-OH); 7,2 - 7,5 (m, 3H) und 8,41 (dd, 1H; Aromatenprotonen)
   ¹³C-NMR (CDCl₃,D-Skala): 33,0 (q, NCH₃); 64,0 (q, OCH₃); 82,6 (d, HC-CH; 125,0 (s, C-C=N); 127,6/128,7/130,2/130,6 (4d, tertiäre Aromaten-C); 135,6 (s, C-CHOH); 141,6 (s, C=N); 161,4 (s, C=O) IR (KBr,ν in cm⁻¹): 3295, 1648, 1576, 1021, 765.
b) Darstellung von 2- (O-3-Brombenzyl-oximinoethyl)-α-Methoxyimino-phenylessigsäuremonomethylamid
   (Verbindung Nr. 58 in Tabelle 1)
   1,7 g (7,7 mmol) Halbaminal der Formel VI wie oben gemäß a) hergestellt werden mit 2,3 g (7,7 mmol) 3-Brombenzyloxyamin-Hydrochlorid der Formel VIII (R²-Y=3-BrC₆H₄CH₂-O) in 150 ml THF 1 h lang am Rückfluß gekocht. Nach dem Erkalten wird das Reaktionsgemisch 2 mal mit gesättigter Kochsalzlösung gewaschen, über Magnesiumsulfat getrocknet und eingeengt. Das verbleibende Öl wird mit Cyclohexan/Essigester 90:10 bis 70:30 an Kieselgel chromatographiert und das ölige Produkt durch Verreiben mit Diisopropylether zur Kristallisation gebracht. Fast farblose Kristalle, Smp. 103 - 105°C, Ausbeute 1,1 g (38 % d. Th.).
   ¹H-NMR (CDCl₃, D-Skala): 2,88 (d, 3H, NCH₃); 3,90 (s, 3H, OCH₃); 5,10 (s, 2H, OCH₂); 6,73 (br 2, 1H, NH); 7,1 - 7,8 (m, 8H, Aromatenprotonen); 7,94 (s, 1H, CH=N-O).

### Beispiel 2

Darstellung von 2-(O-3-Methylbenzyl-oximinomethyl)-α-Methoxyimino-phenylessigsäure-monomethylamid
(Verbindung Nr. 36 in Tabelle 1)

13,6 g (40 mmol) 2-(O-3-Methylbenzyl-oximinomethyl)-α-Methoxyimino-phenylessig-säuremethylester (Darstellung s. EP 499 823) werden in 100 ml Tetrahydrofuran gelöst, 12 ml 40 %iger wäßriger Methylaminlösung (= 150 mmol CH₃NH₂) zugesetzt und die Mischung 7 h bei 40°C gerührt.

Man läßt auf 25°C abkühlen, setzt Diethylether zu, trennt die Phasen, wäscht die organische Phase mit Wasser, trocknet über Natriumsulfat und engt im Vakuum ein.
Gelbliches Öl, Ausbeute 13,0 g = 96 % d. Th.

IR (Film, ν in cm⁻¹): 1669, 1527, 1411, 1035, 980, 785, 756.

Beispiele für in der beschriebenen Weise darstellbare Wirkstoffe geben die nachstehenden Tabellen:

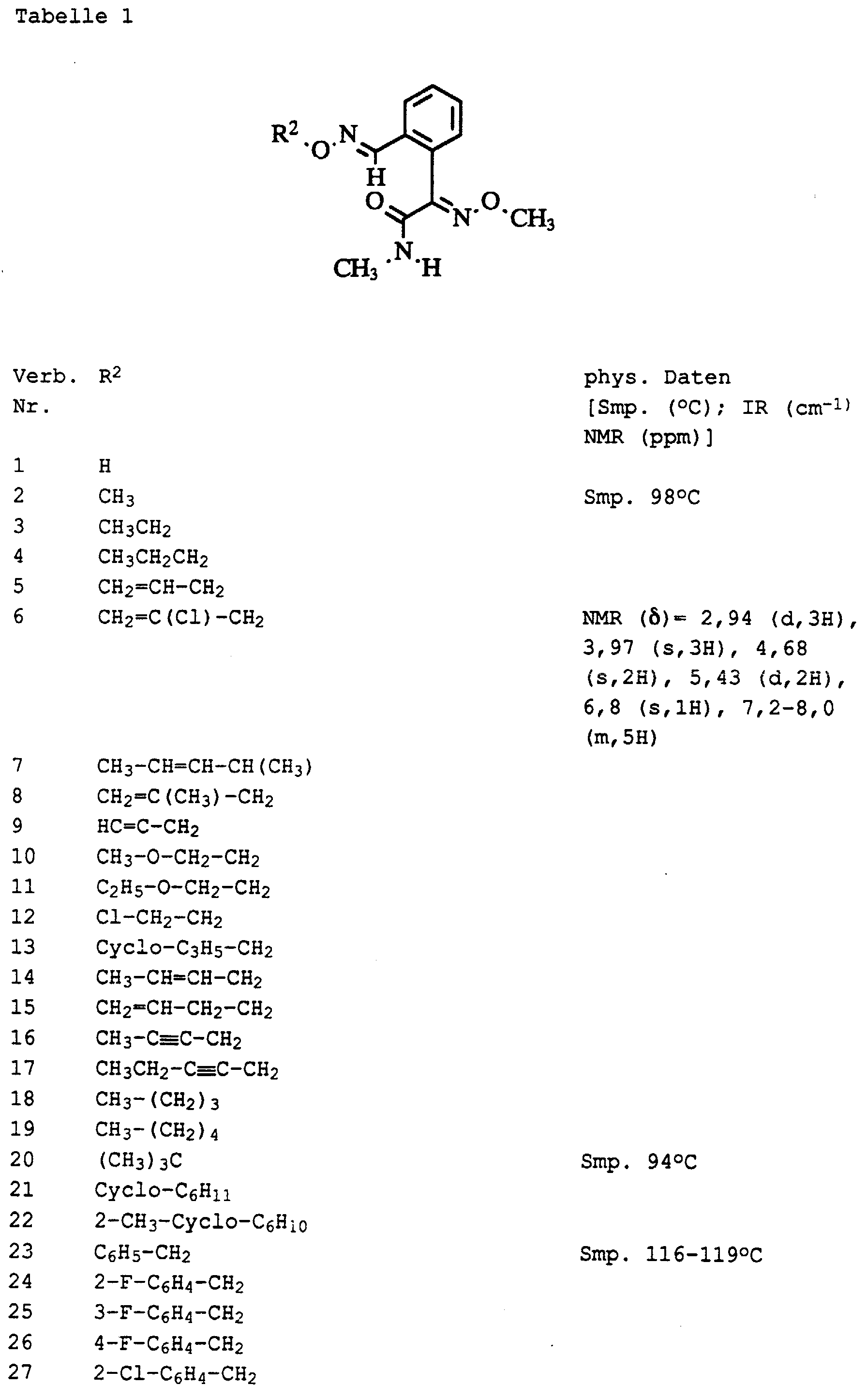

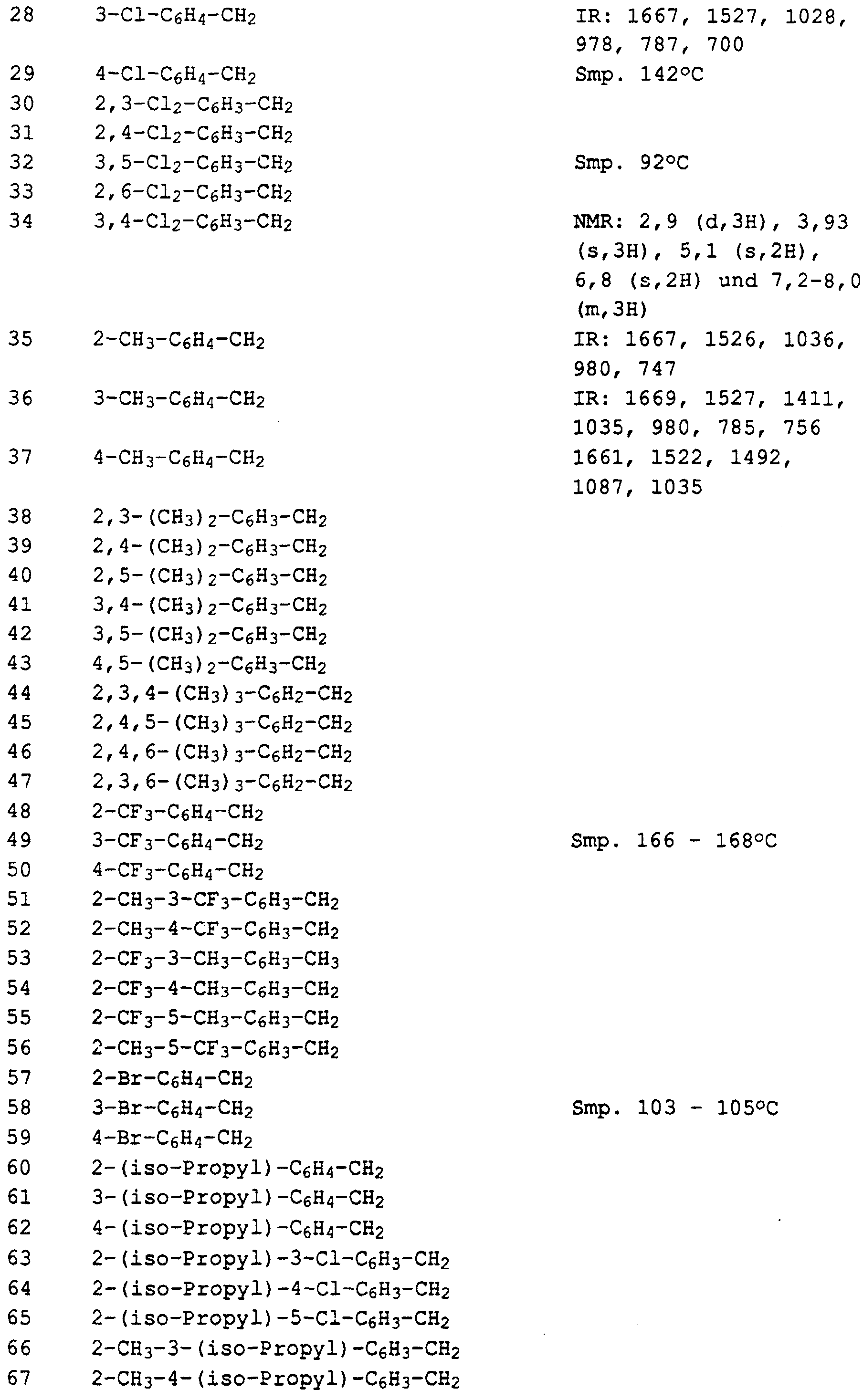

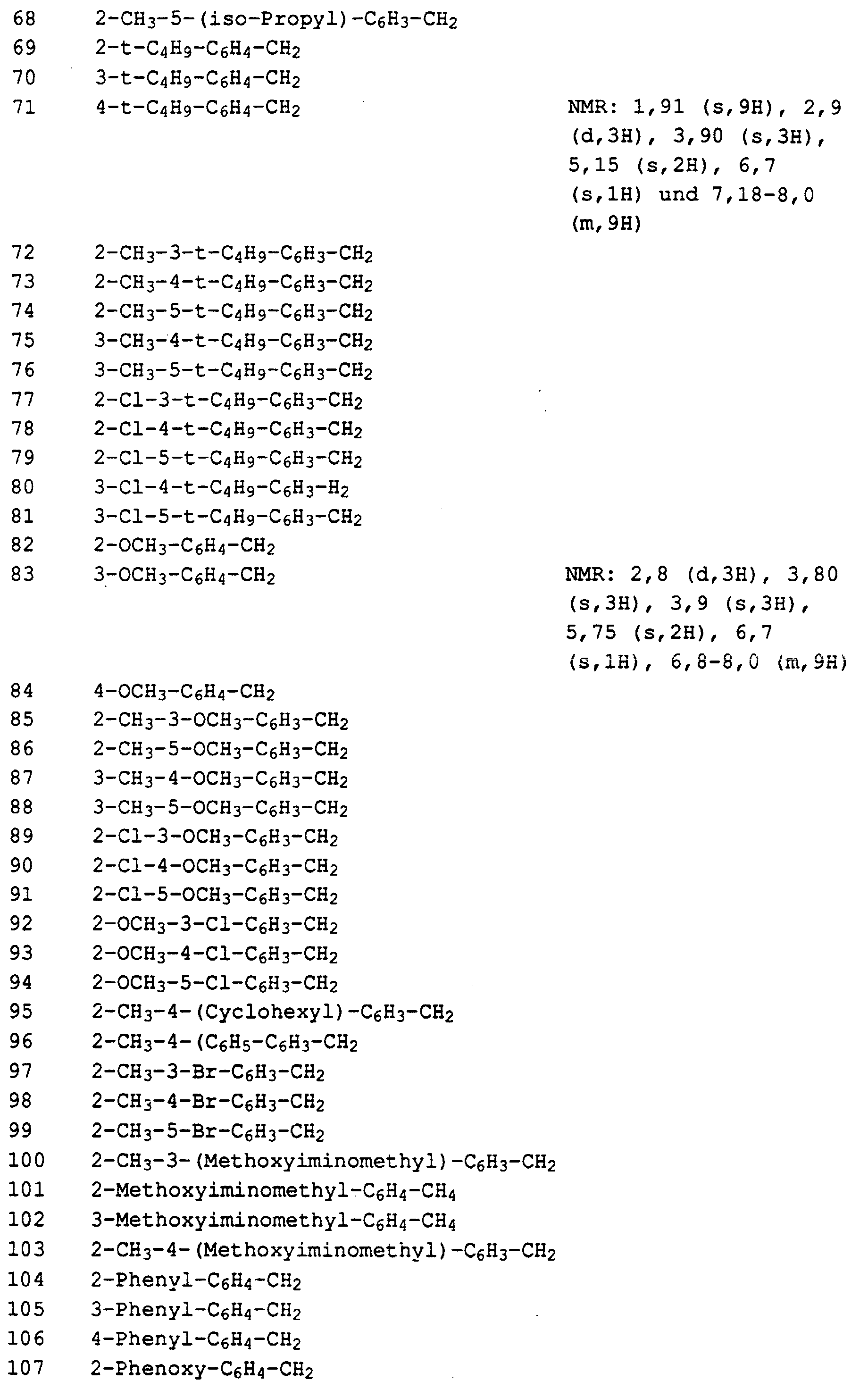

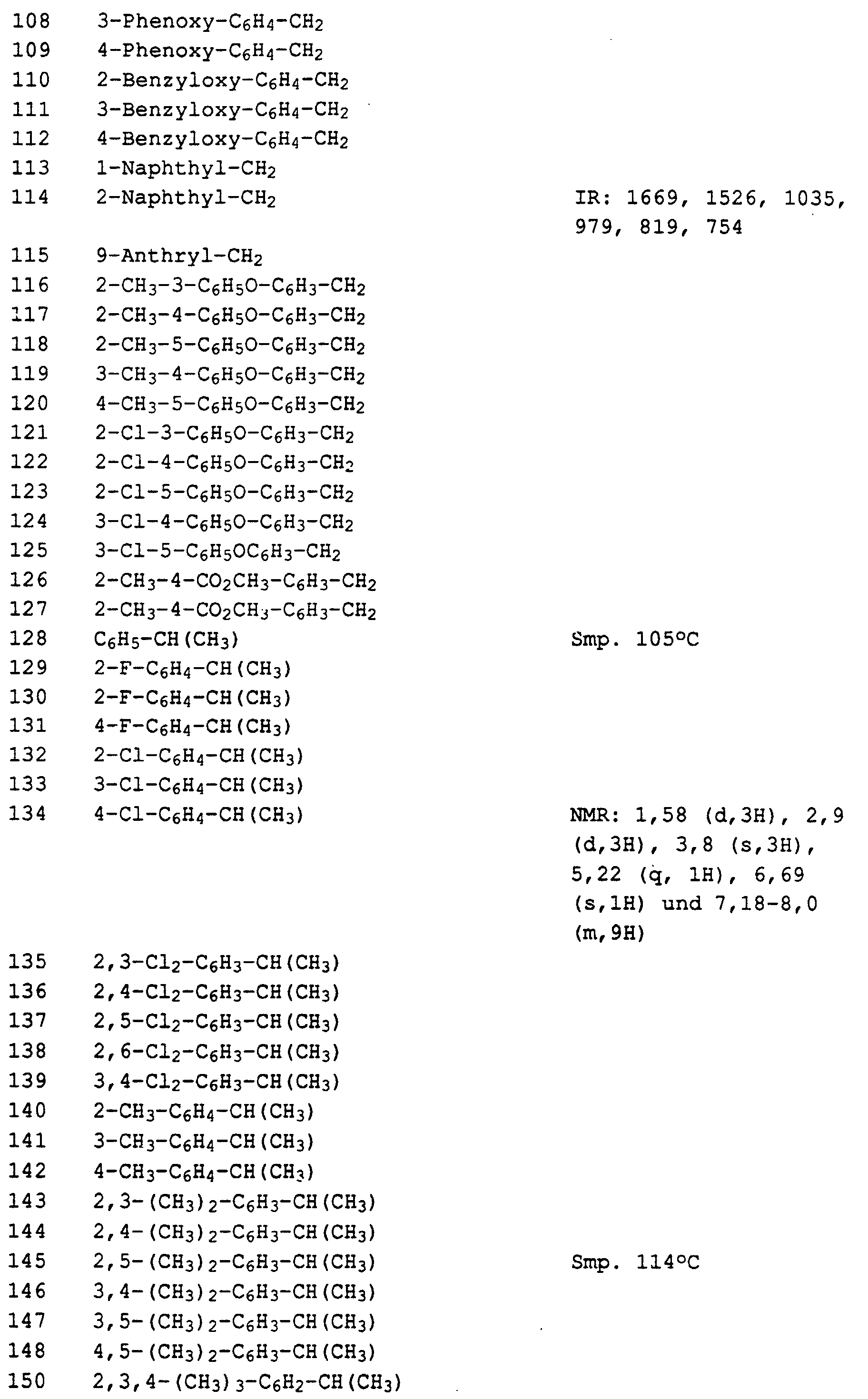

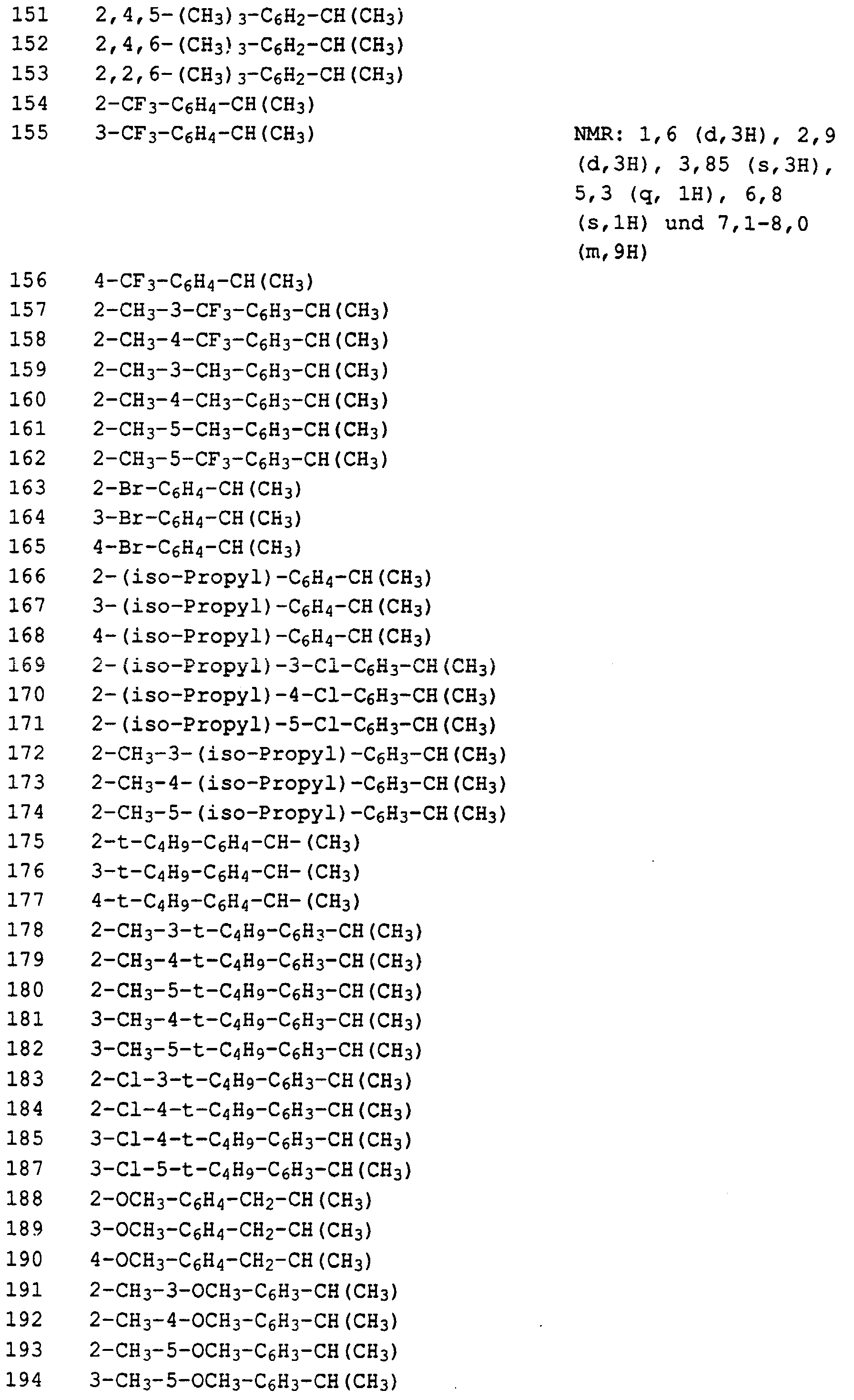

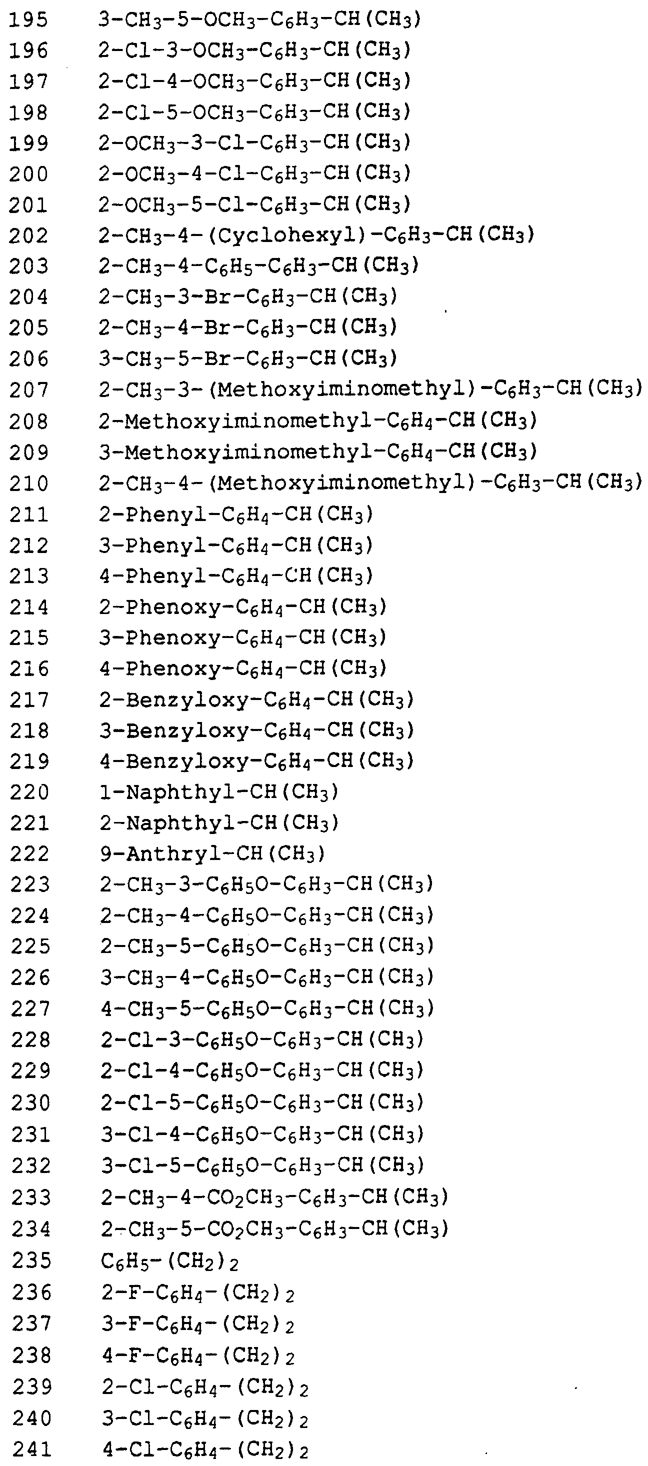

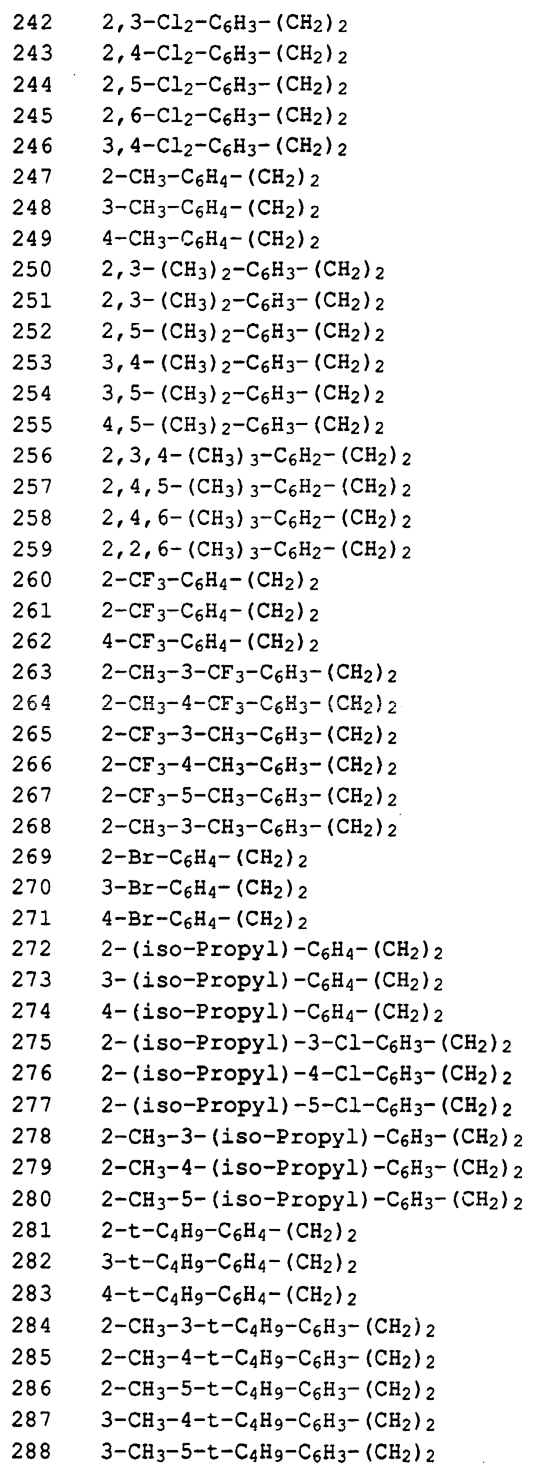

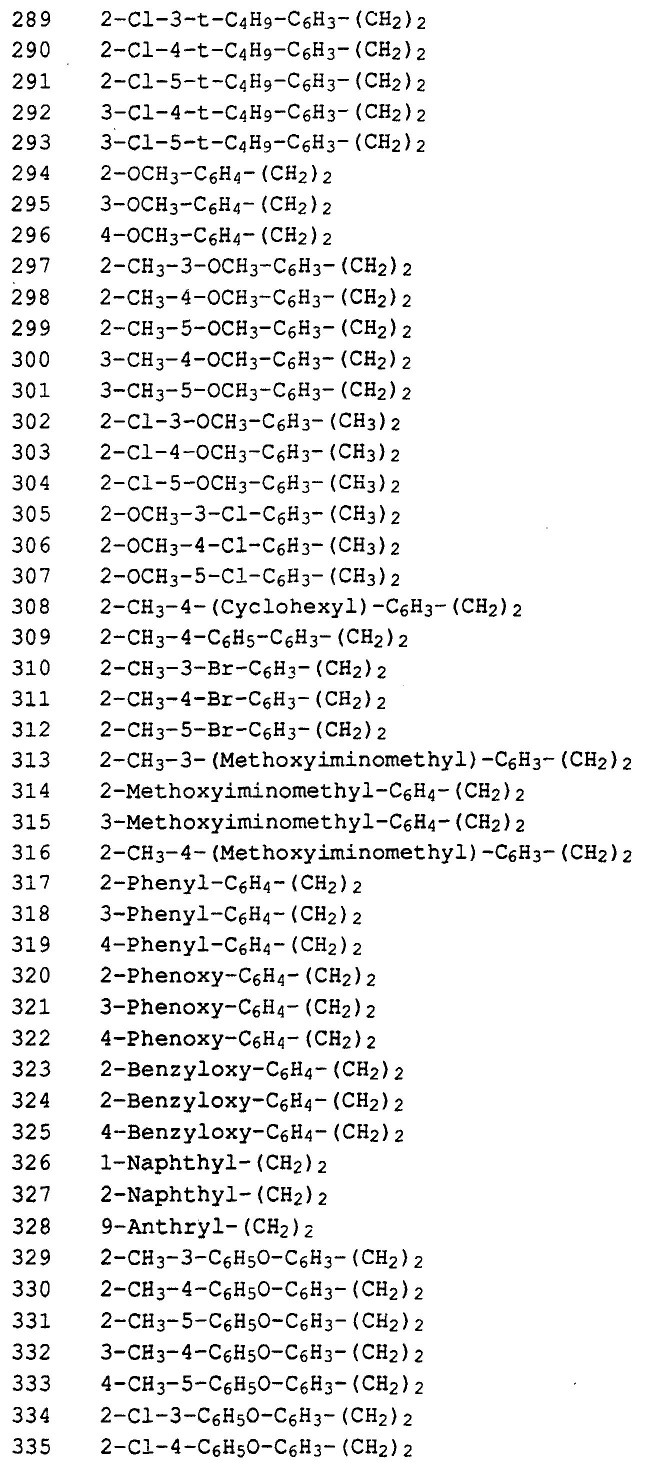

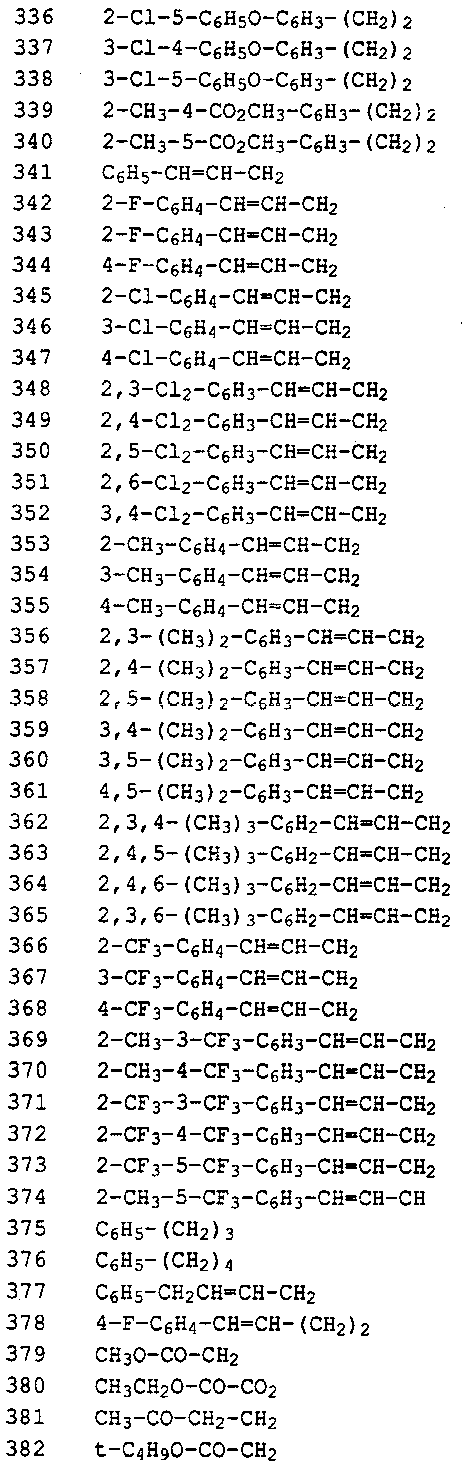

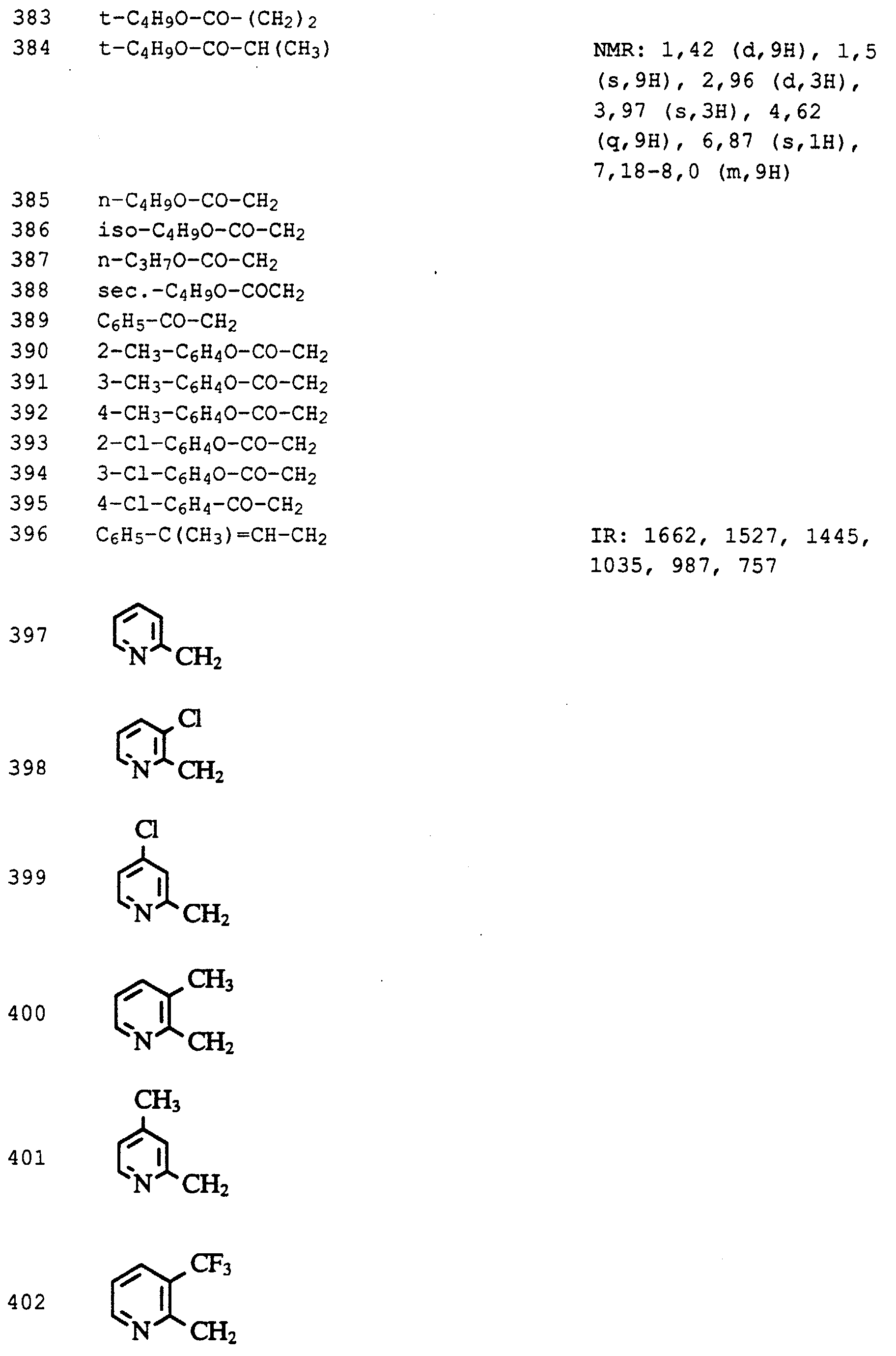

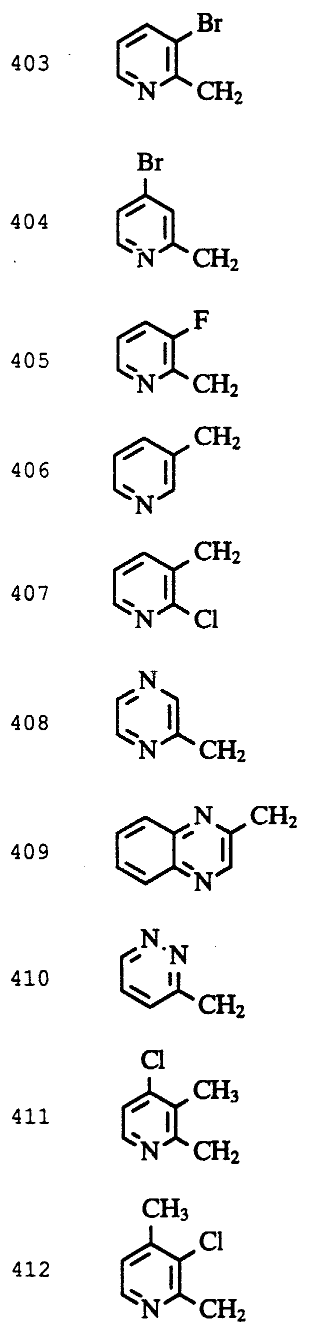

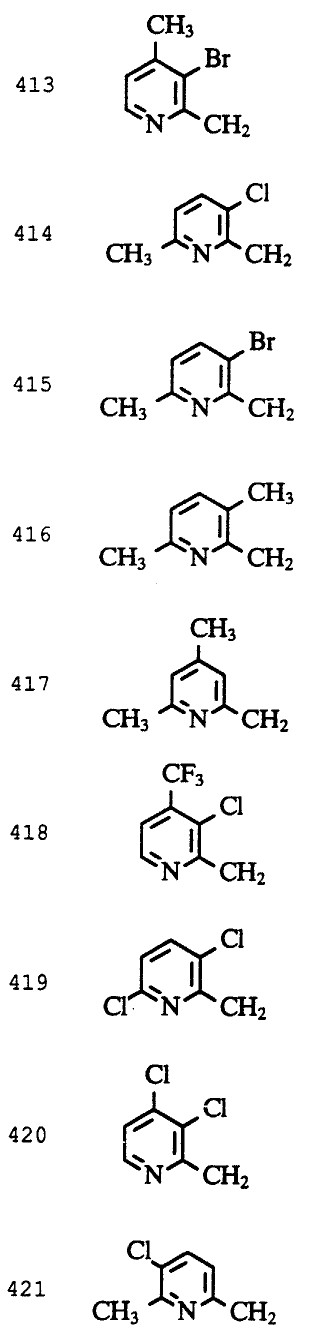

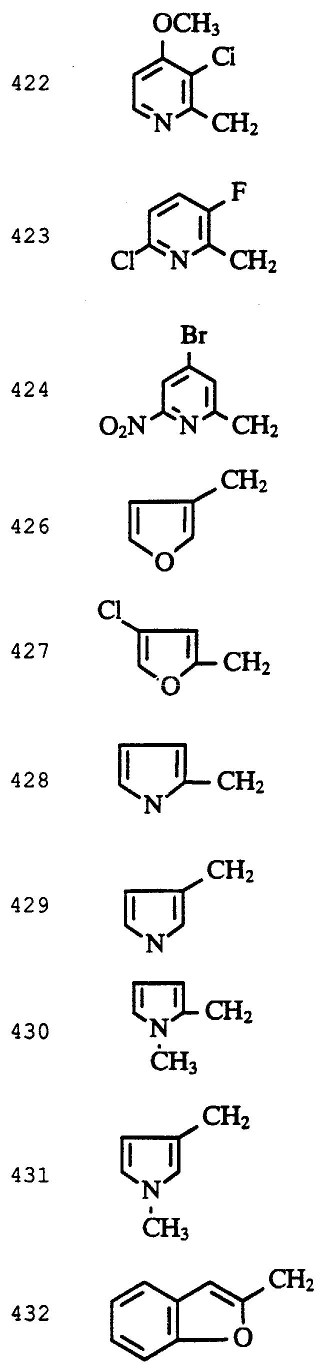

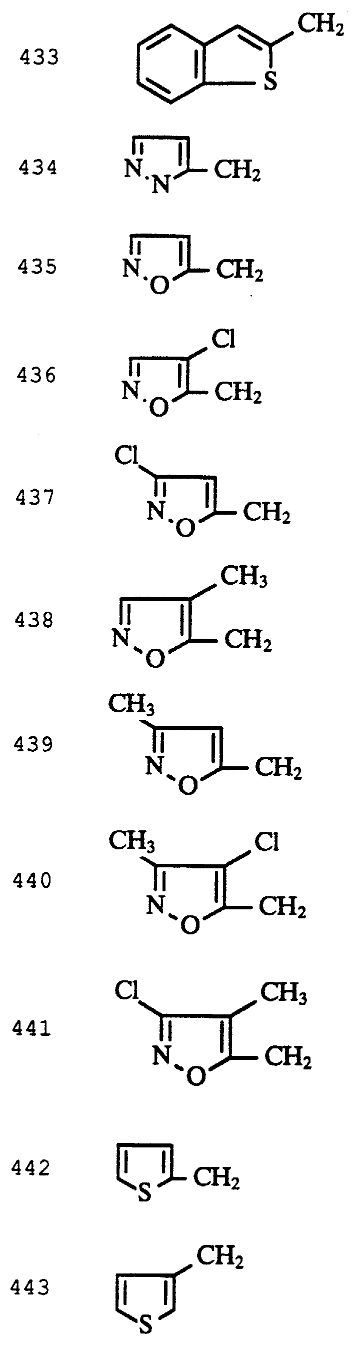

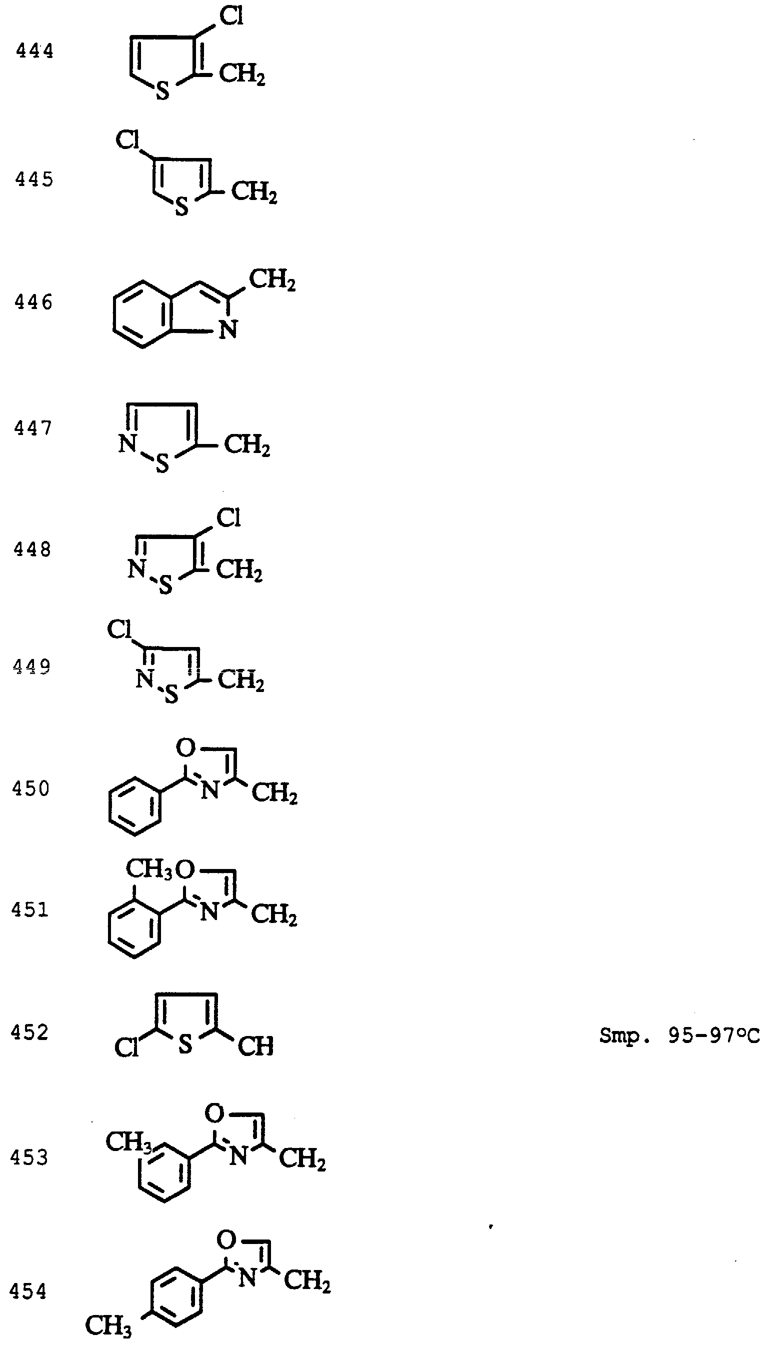

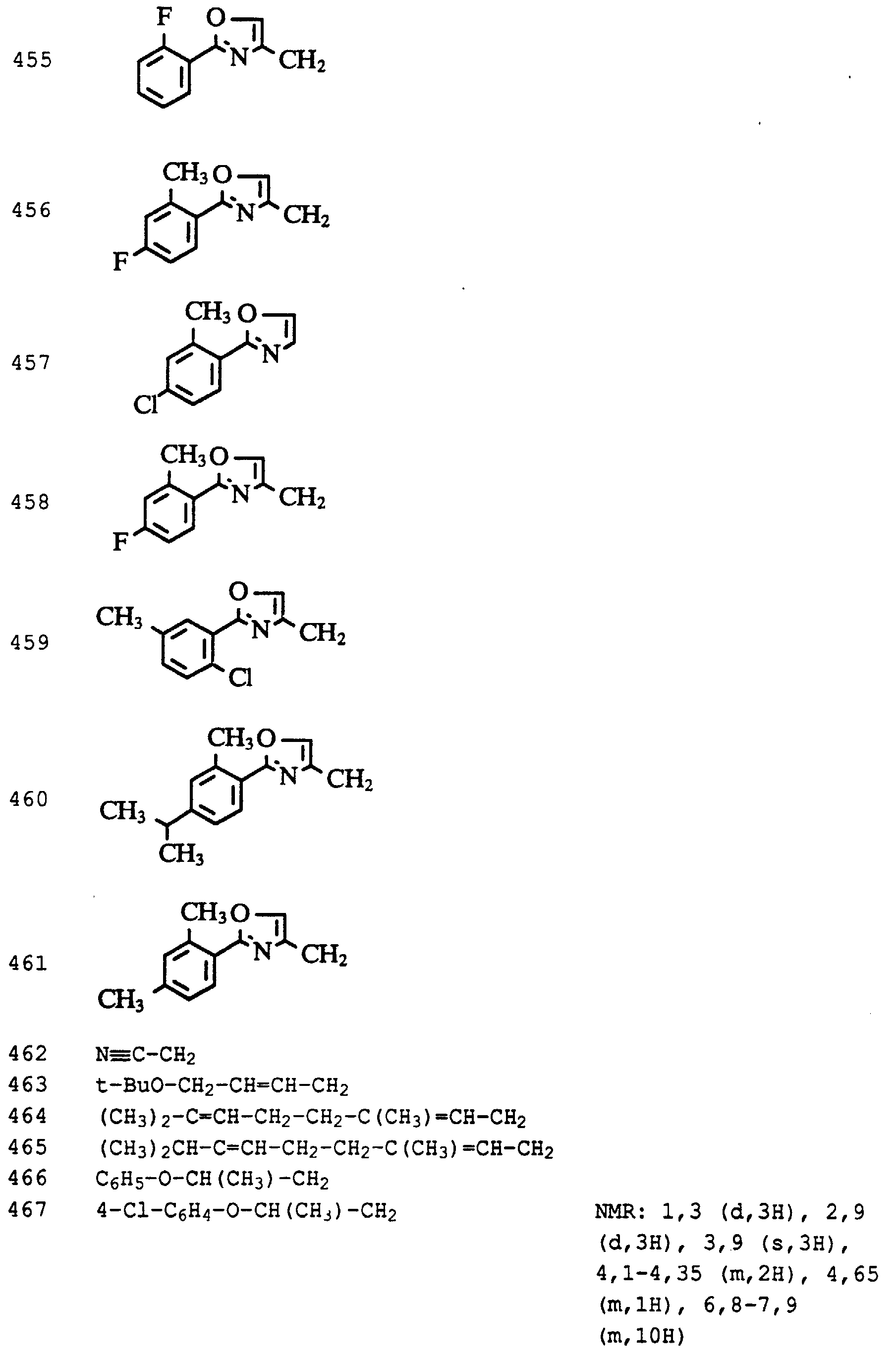

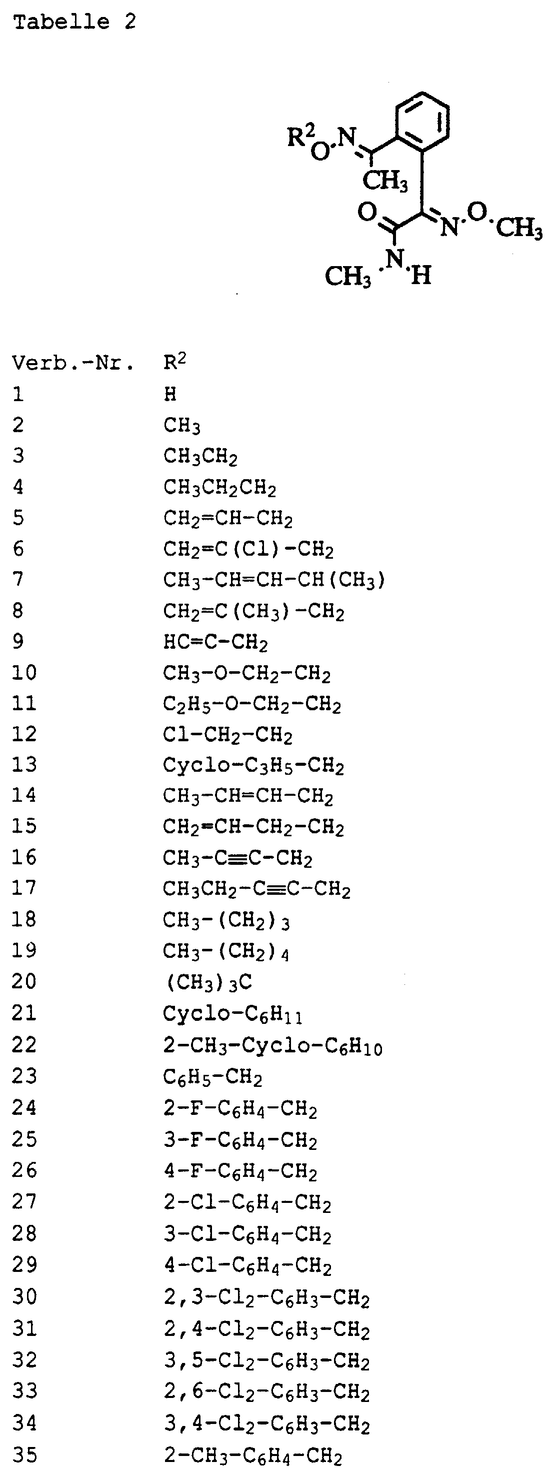

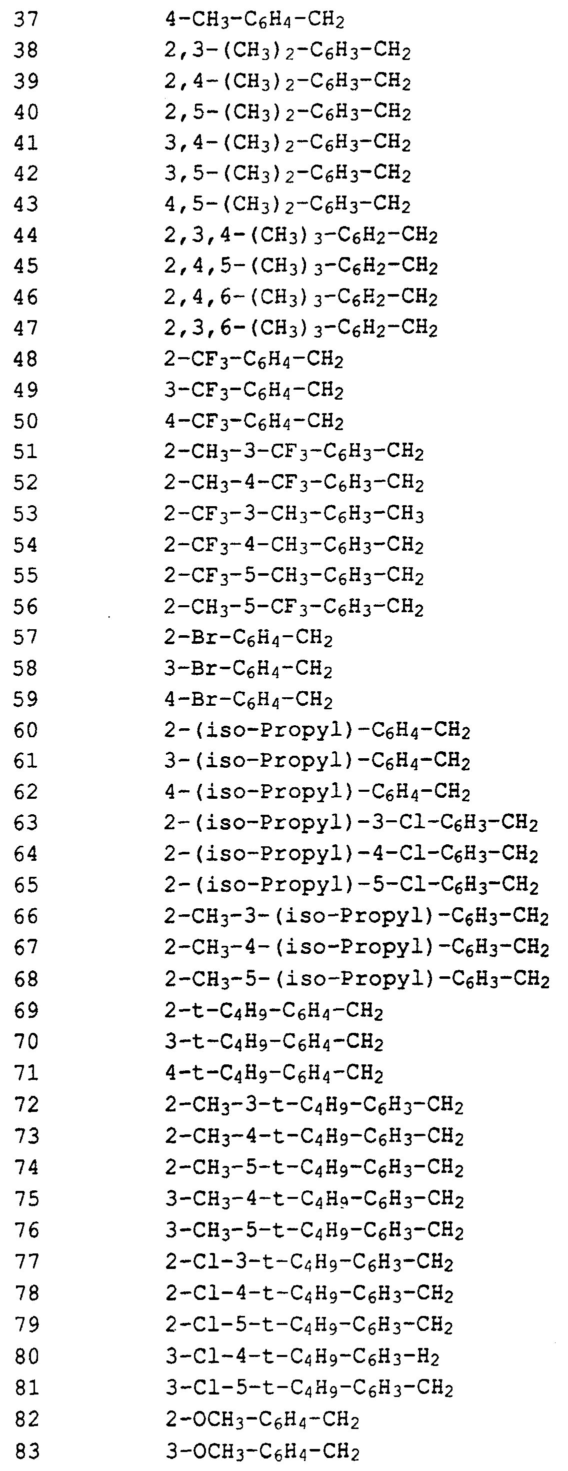

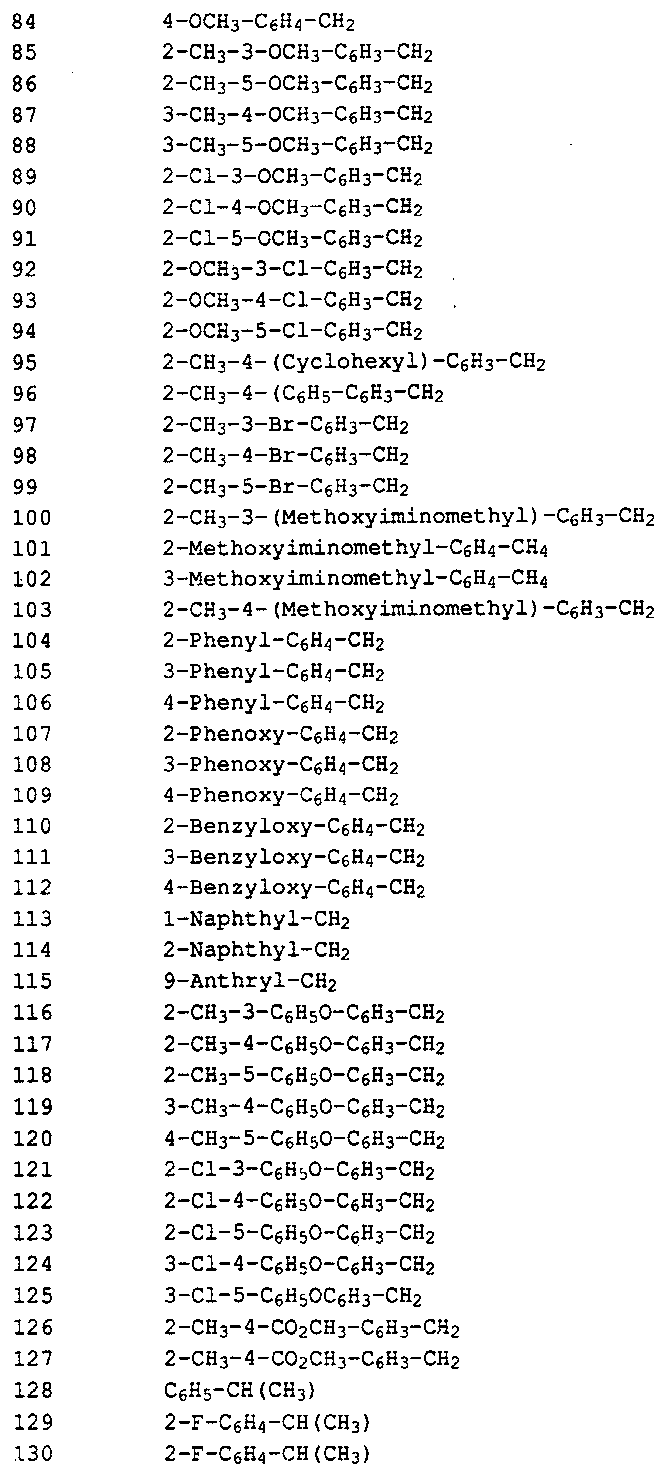

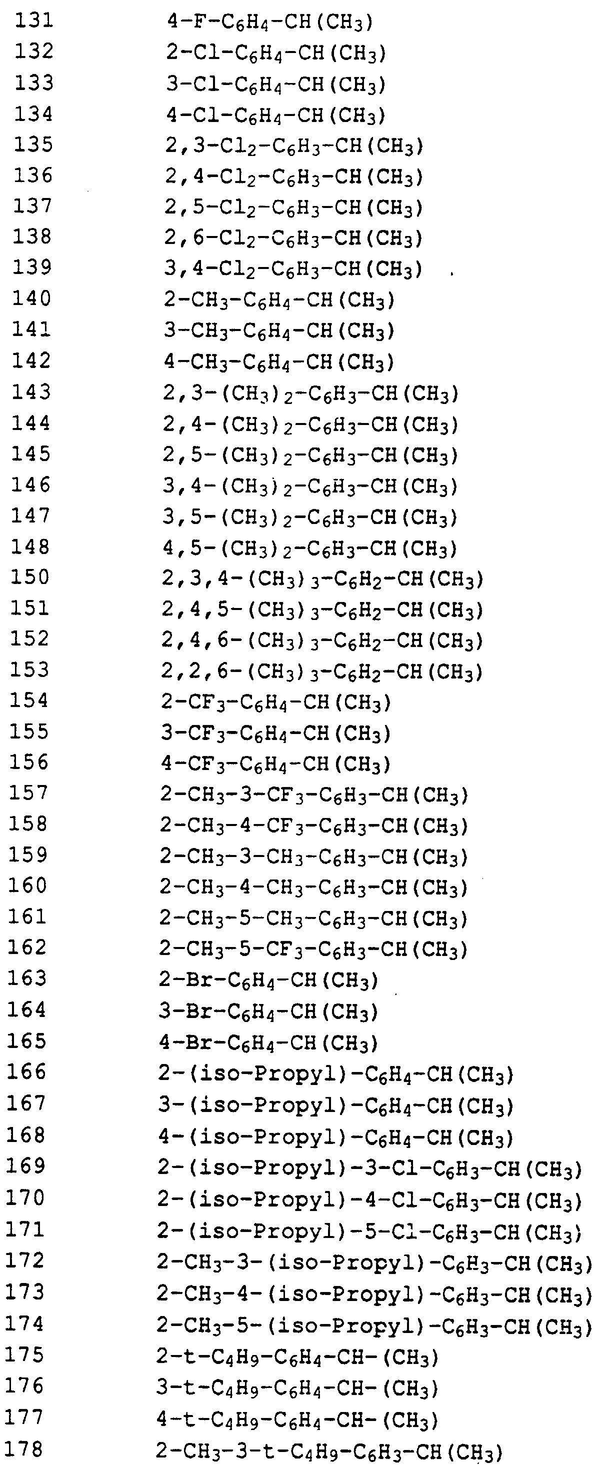

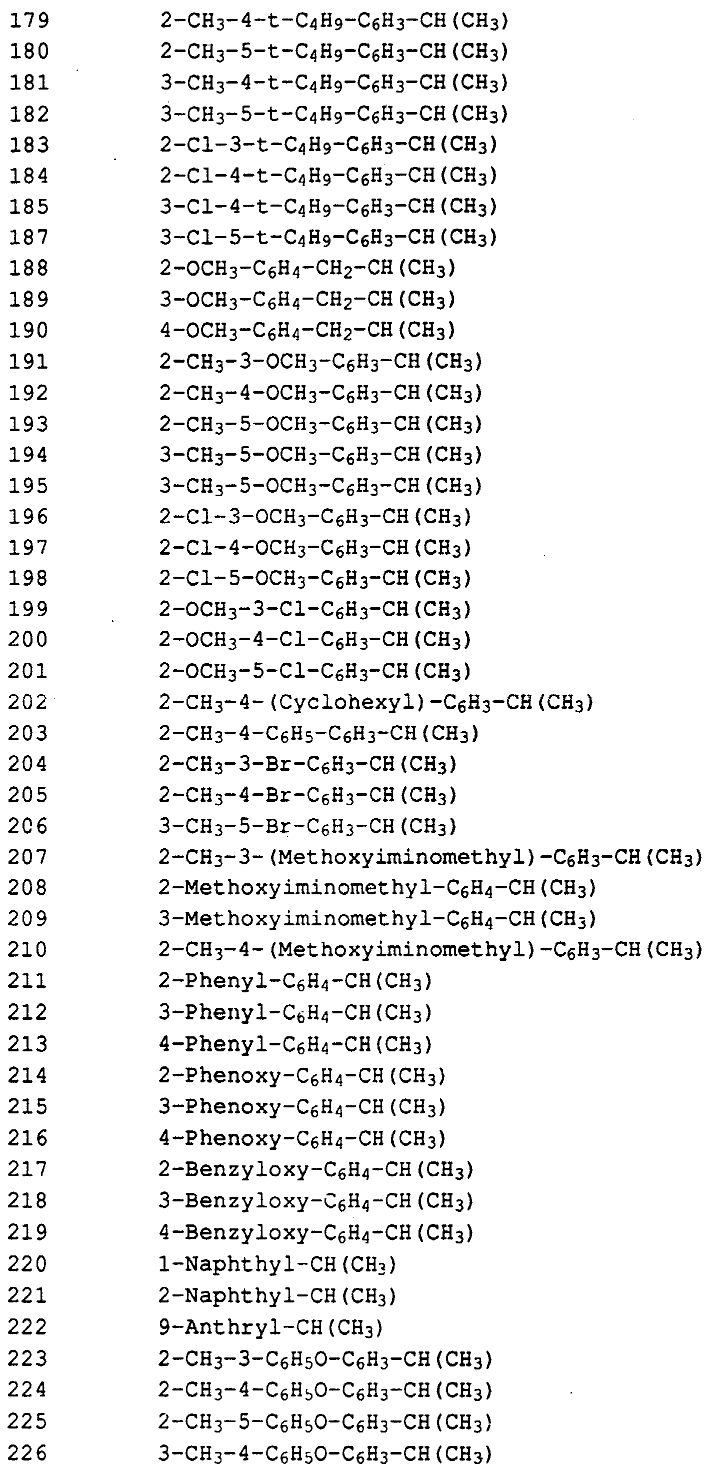

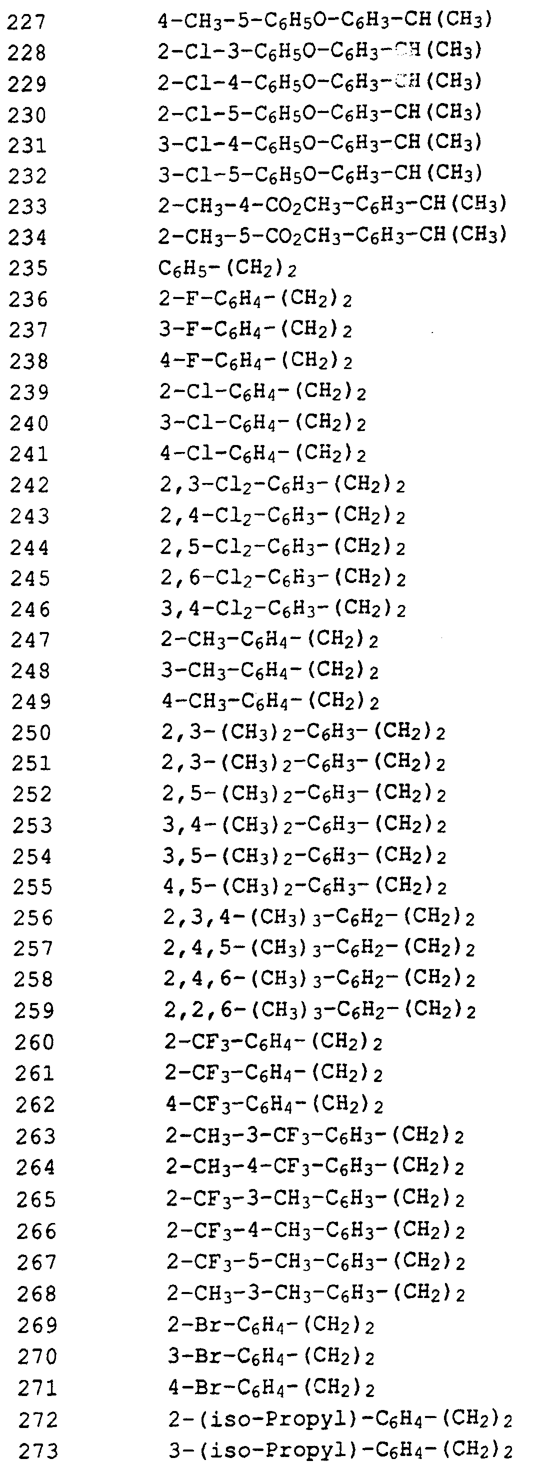

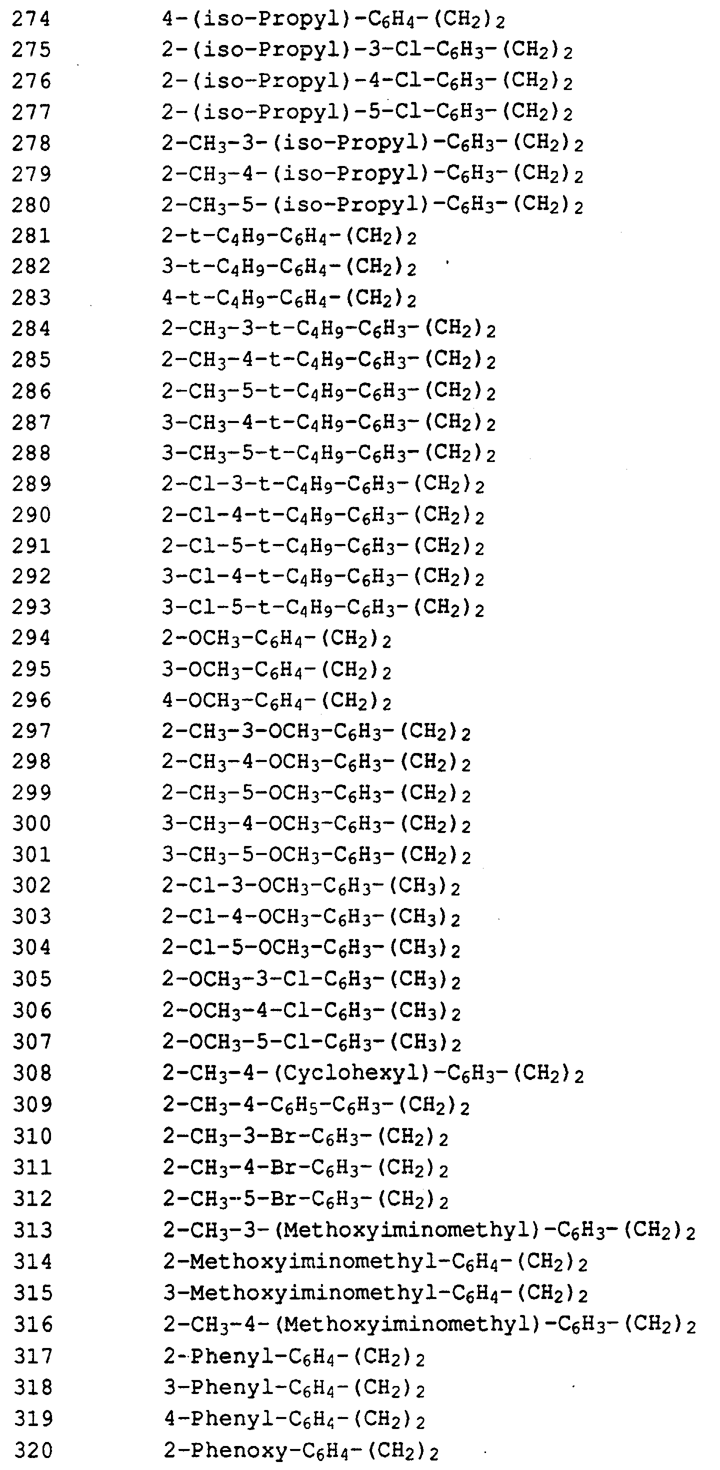

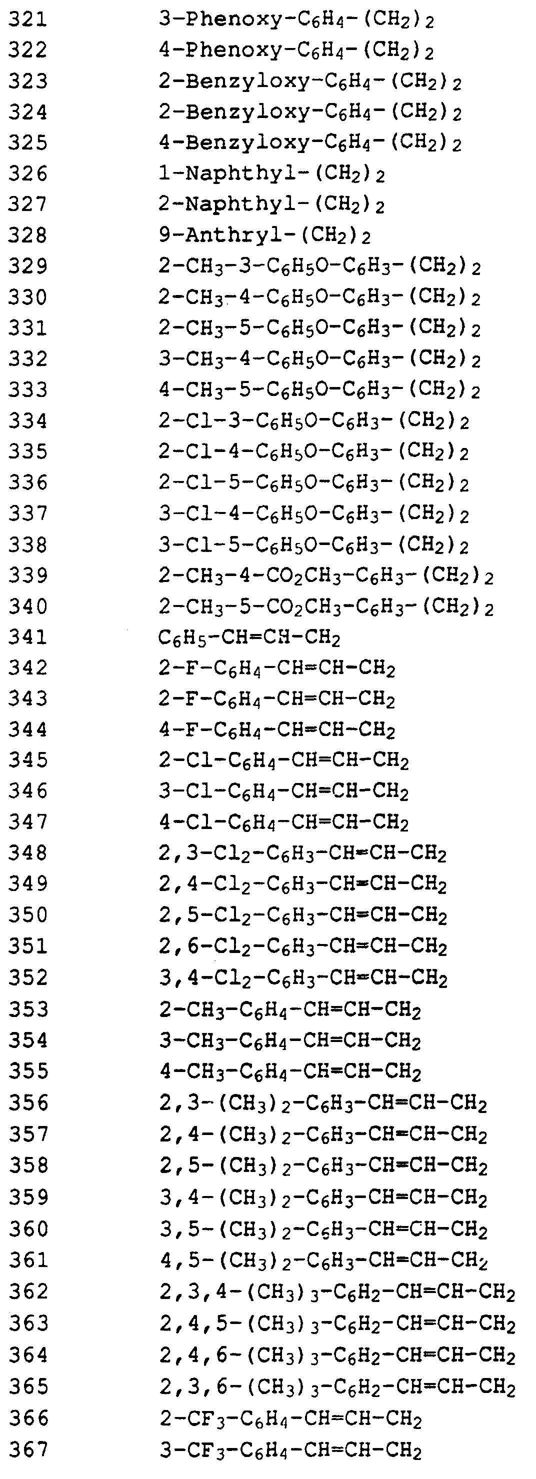

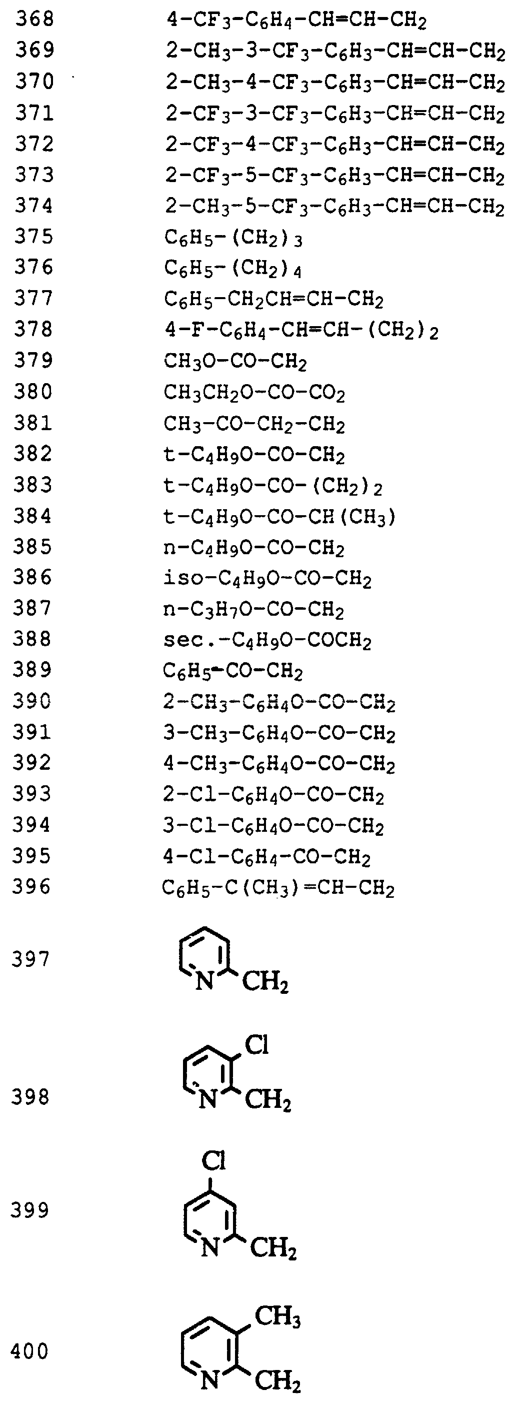

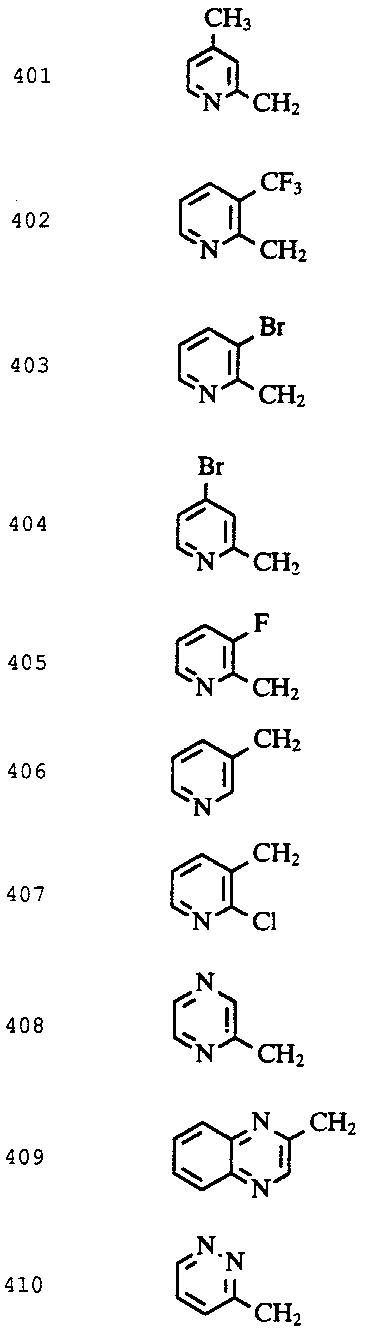

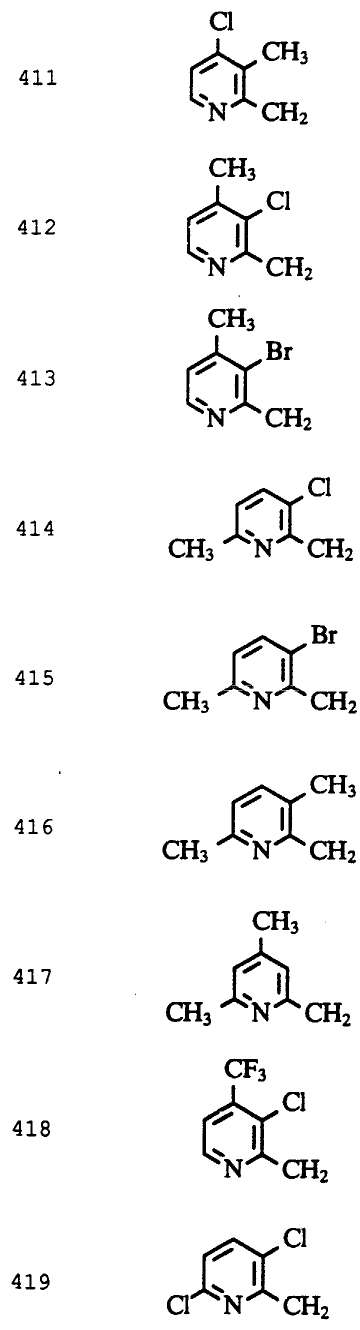

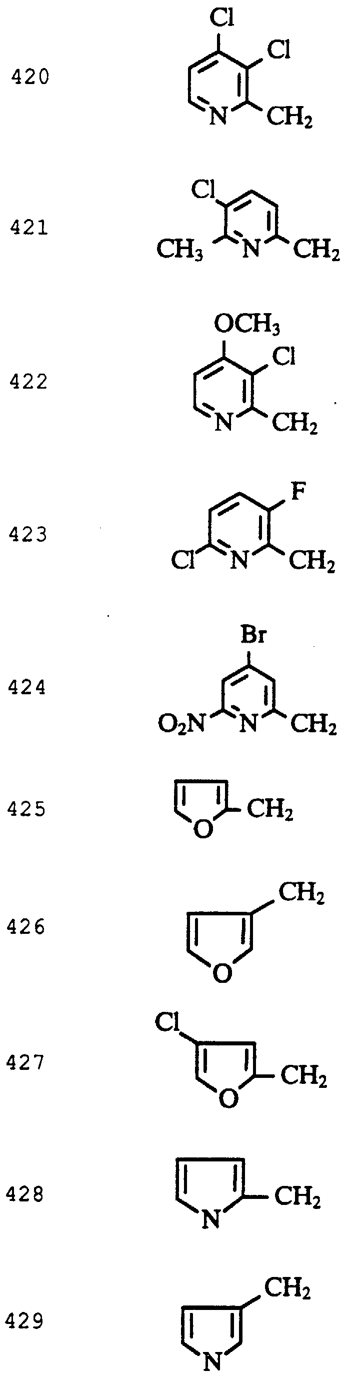

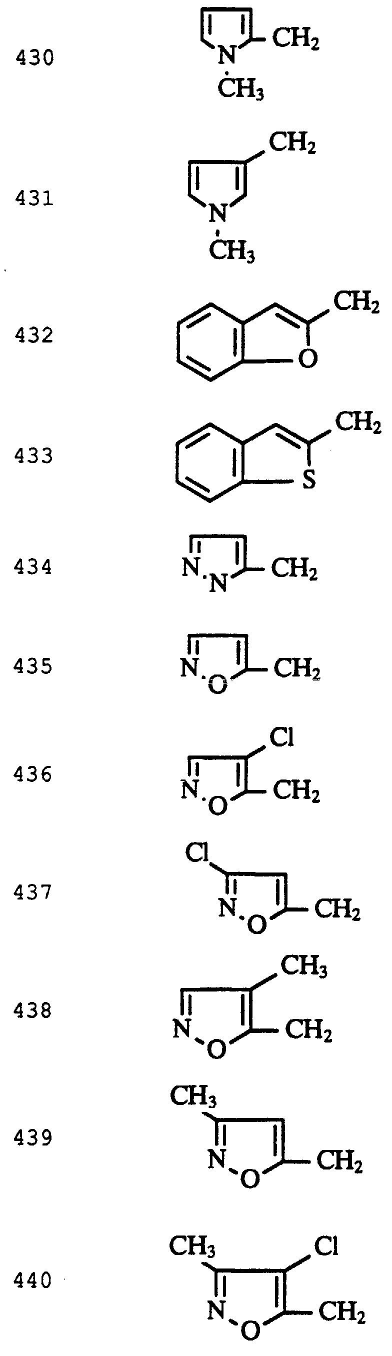

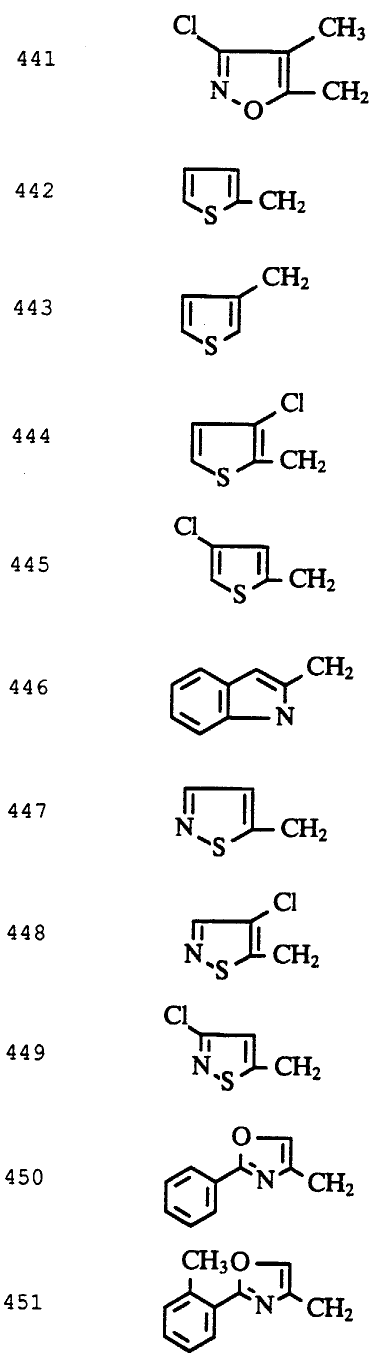

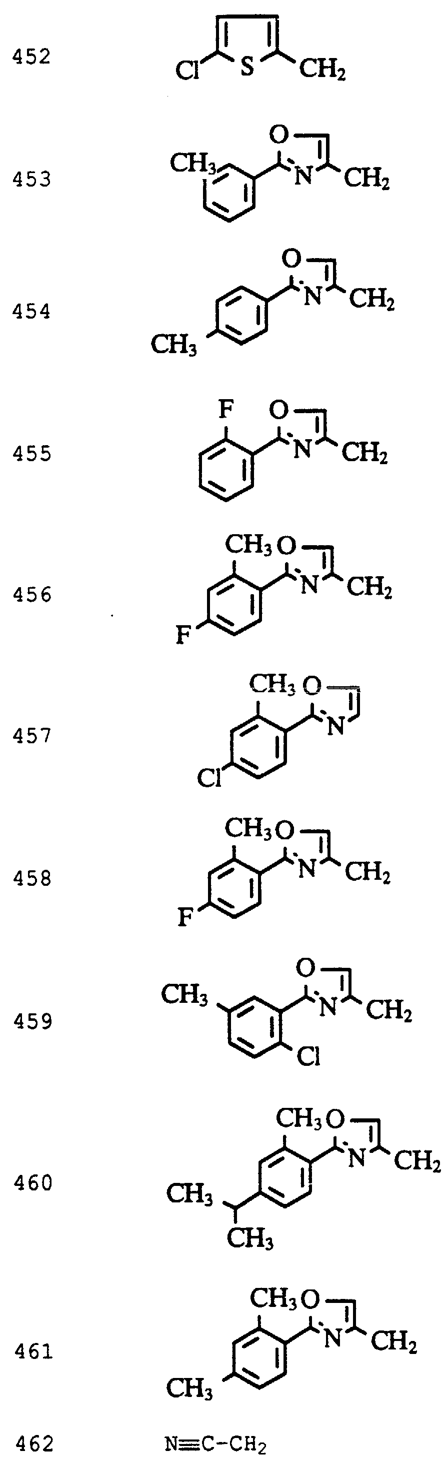

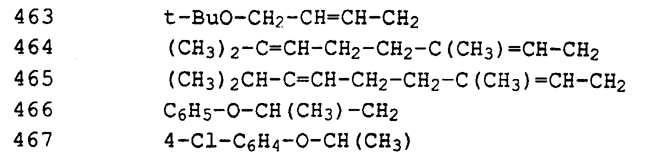

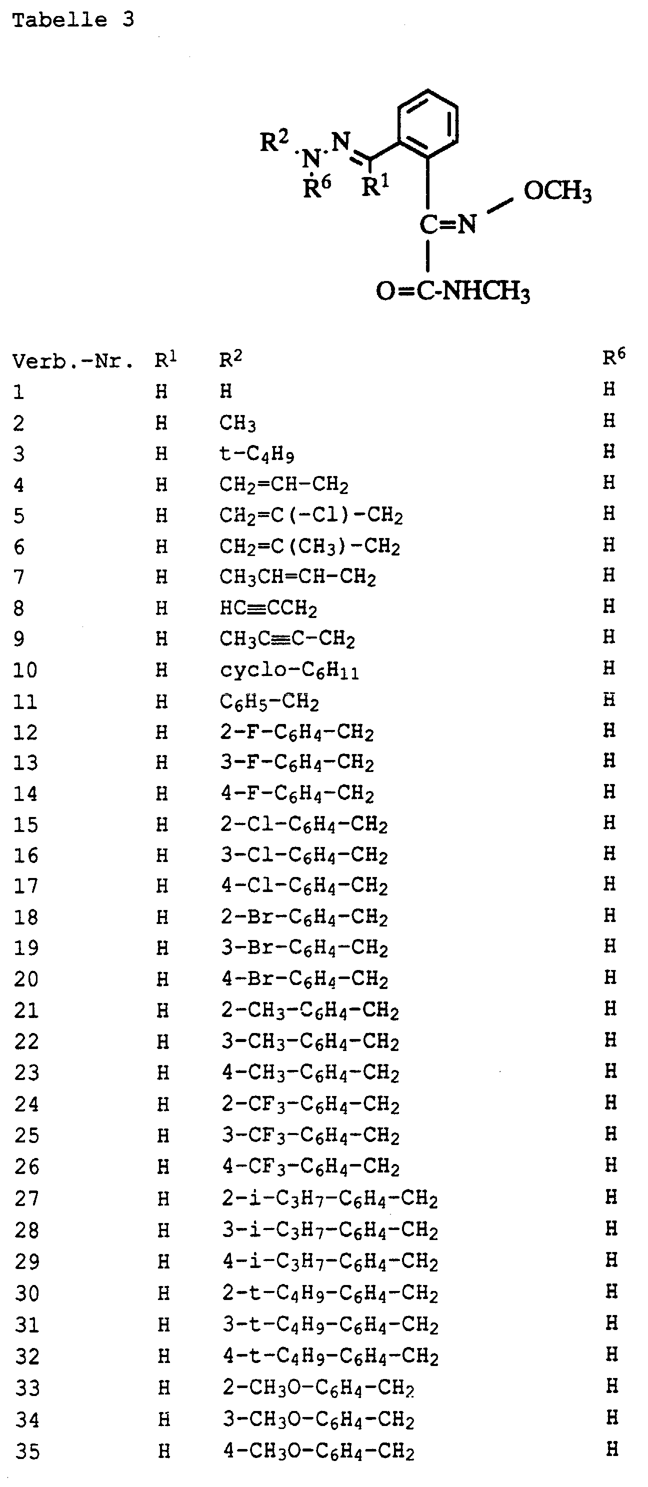

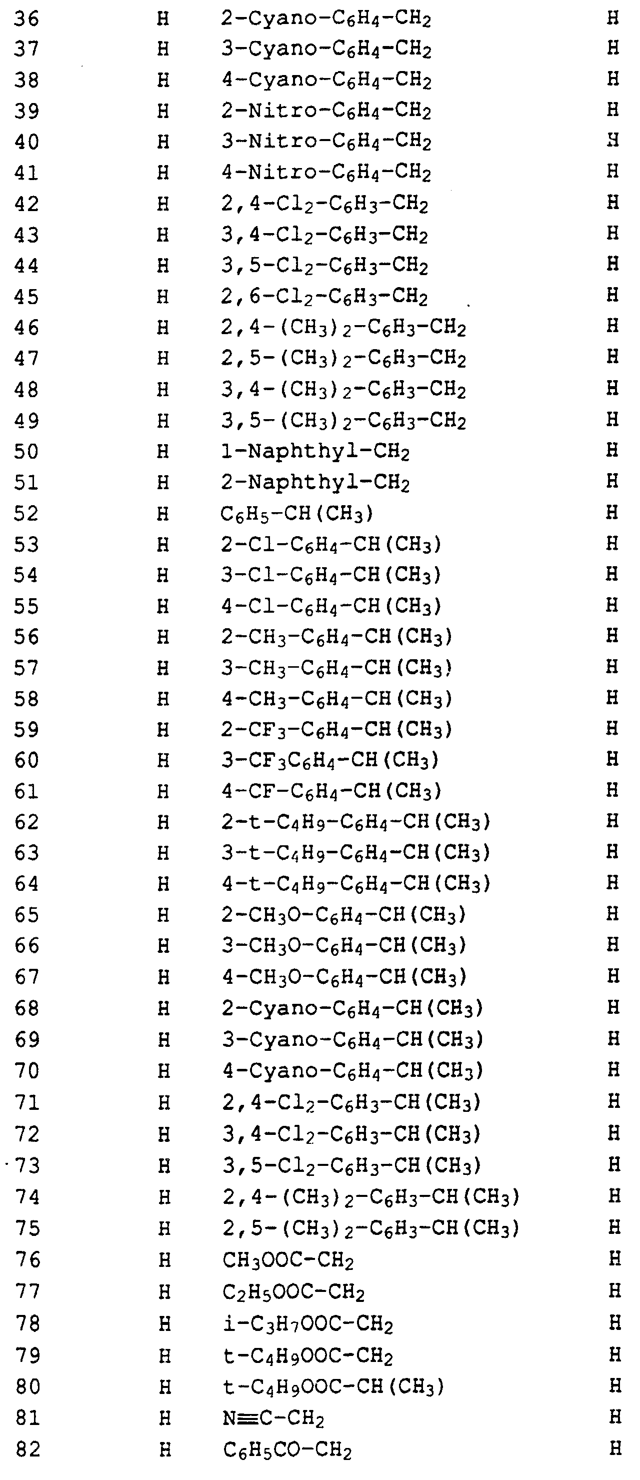

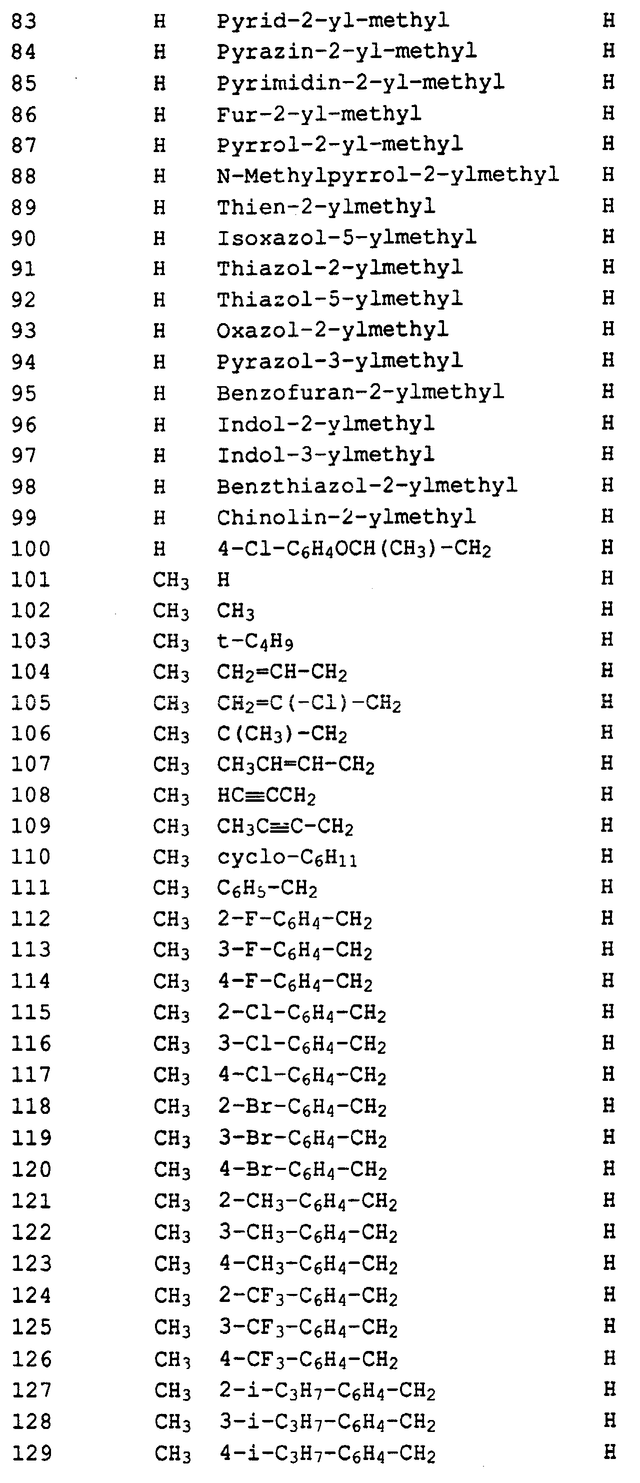

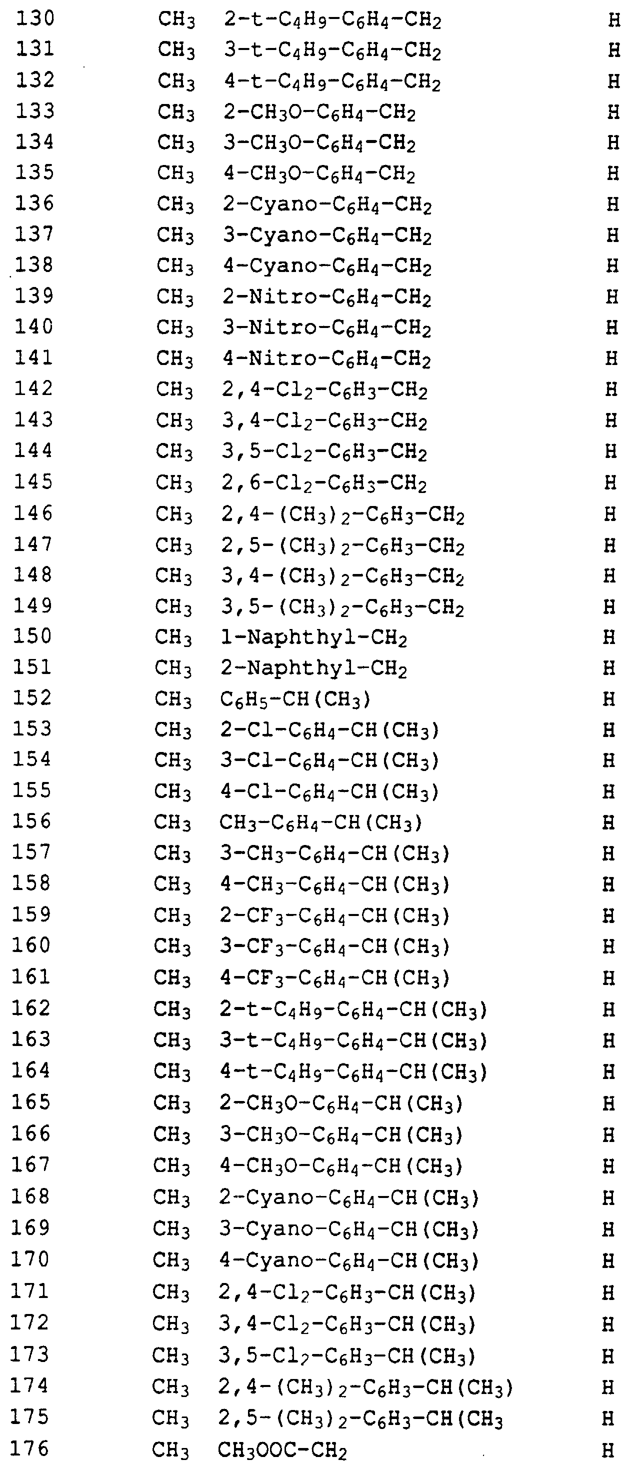

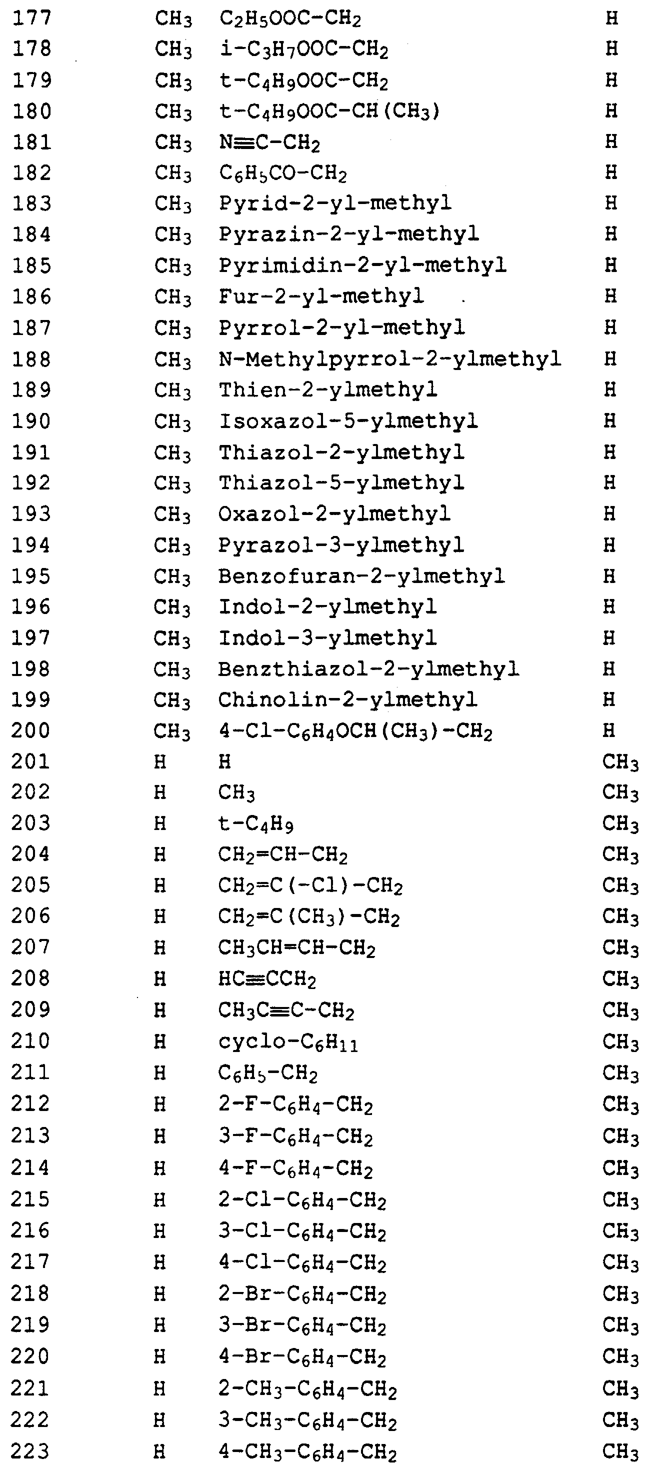

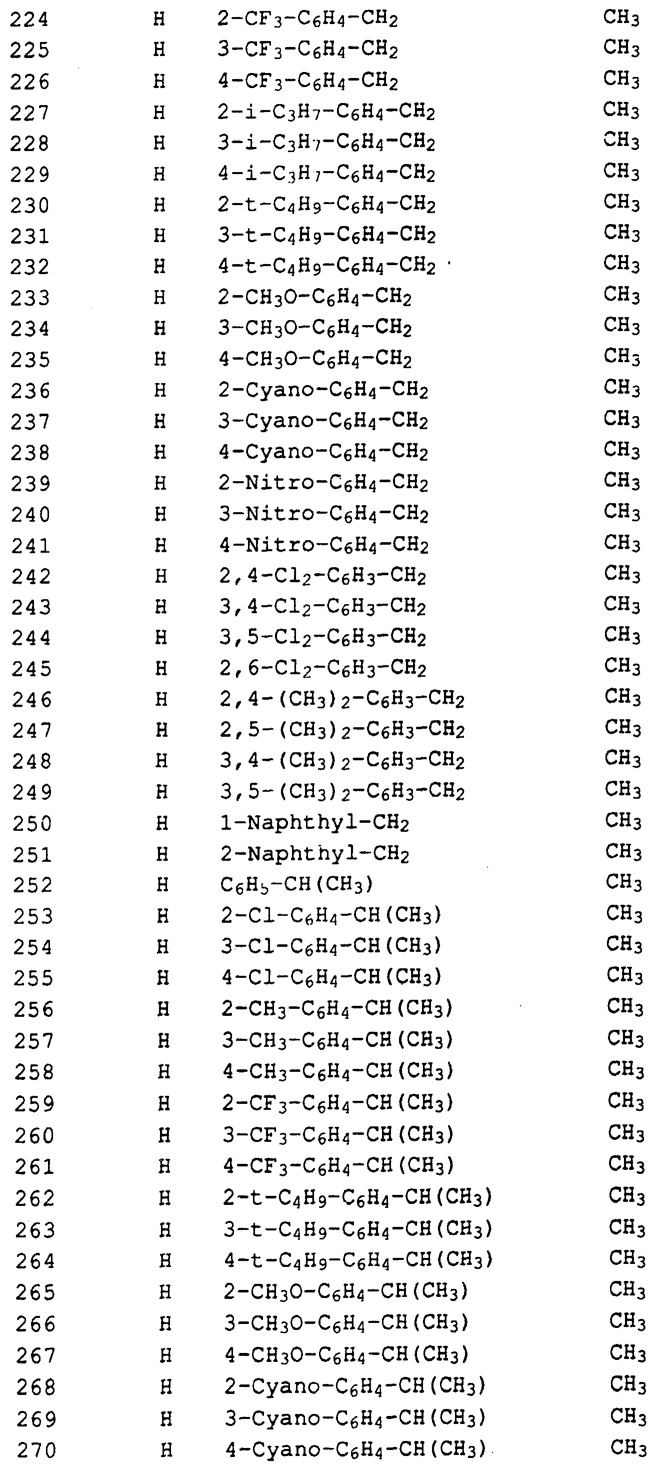

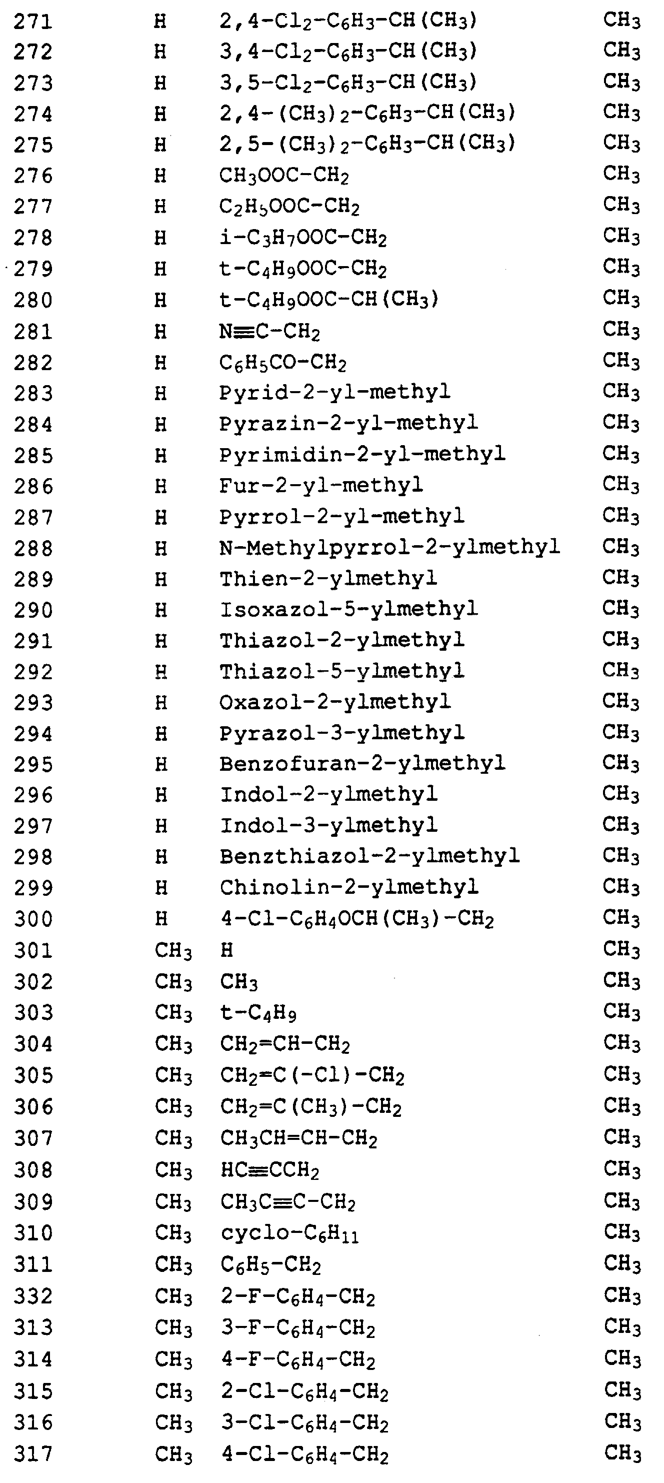

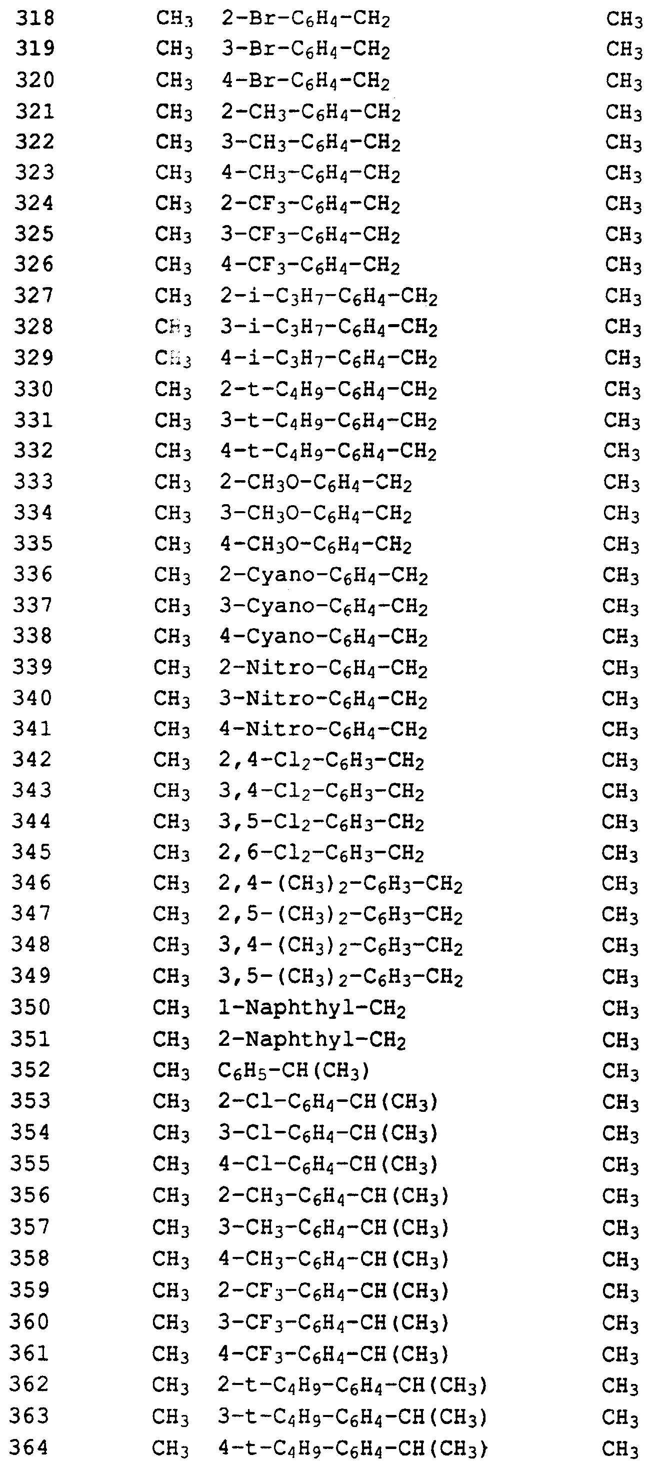

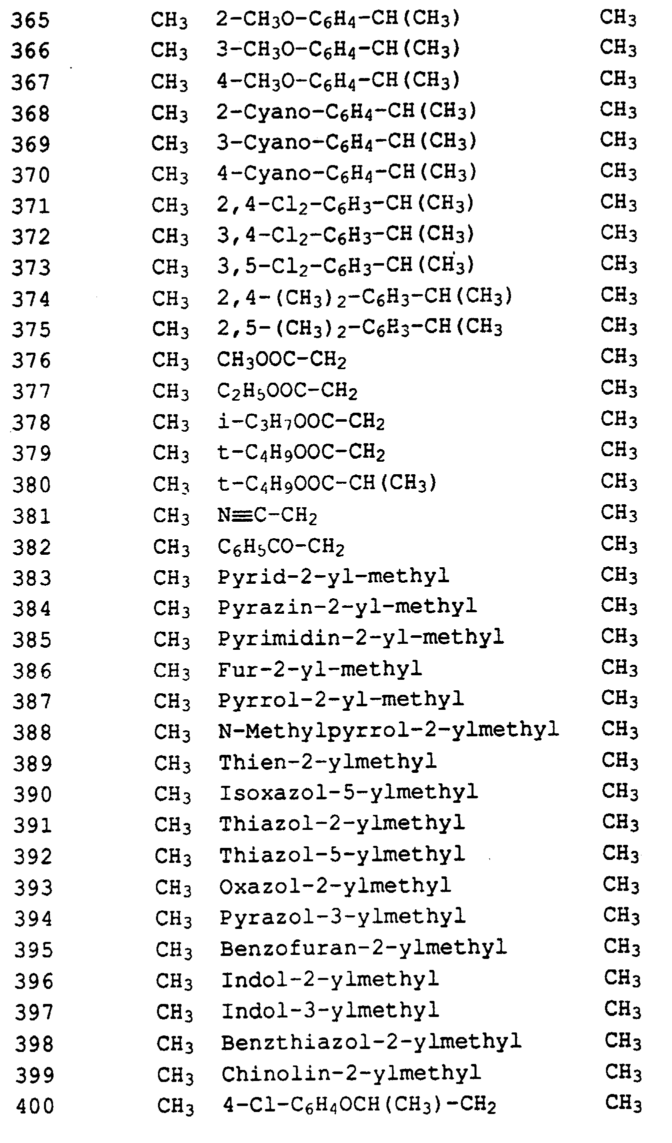

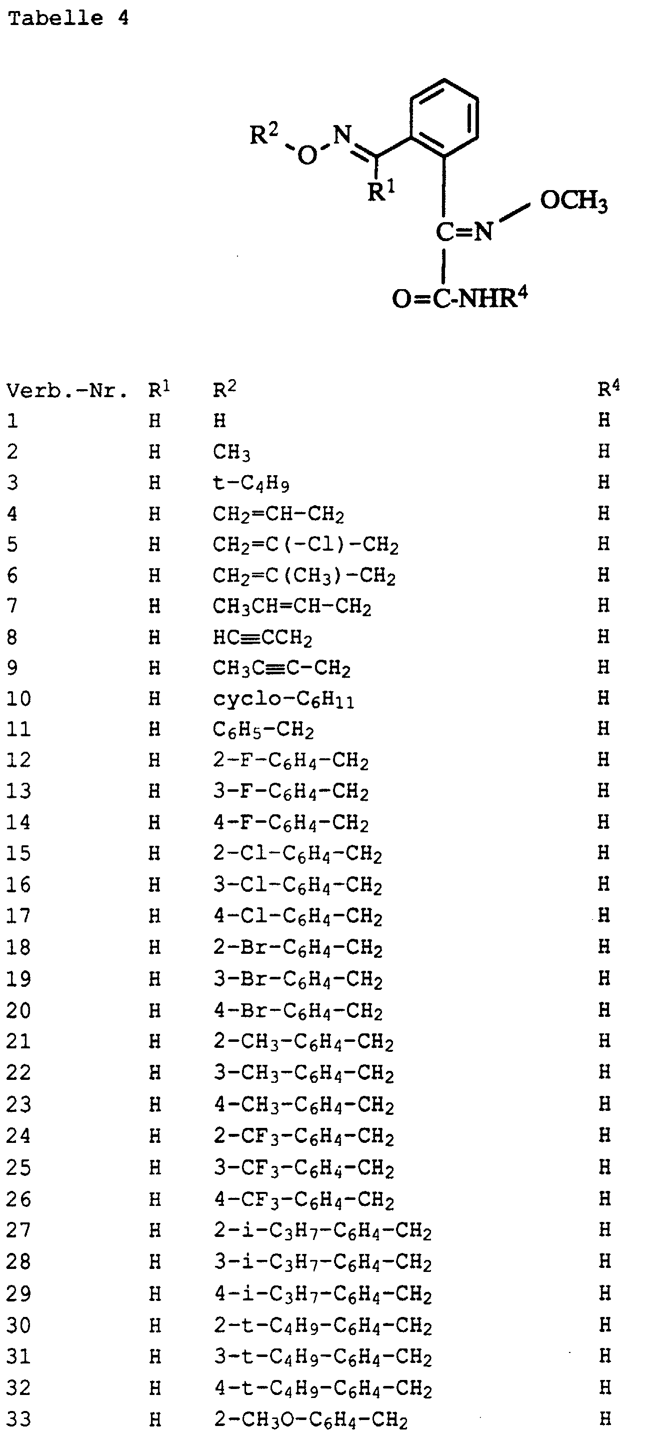

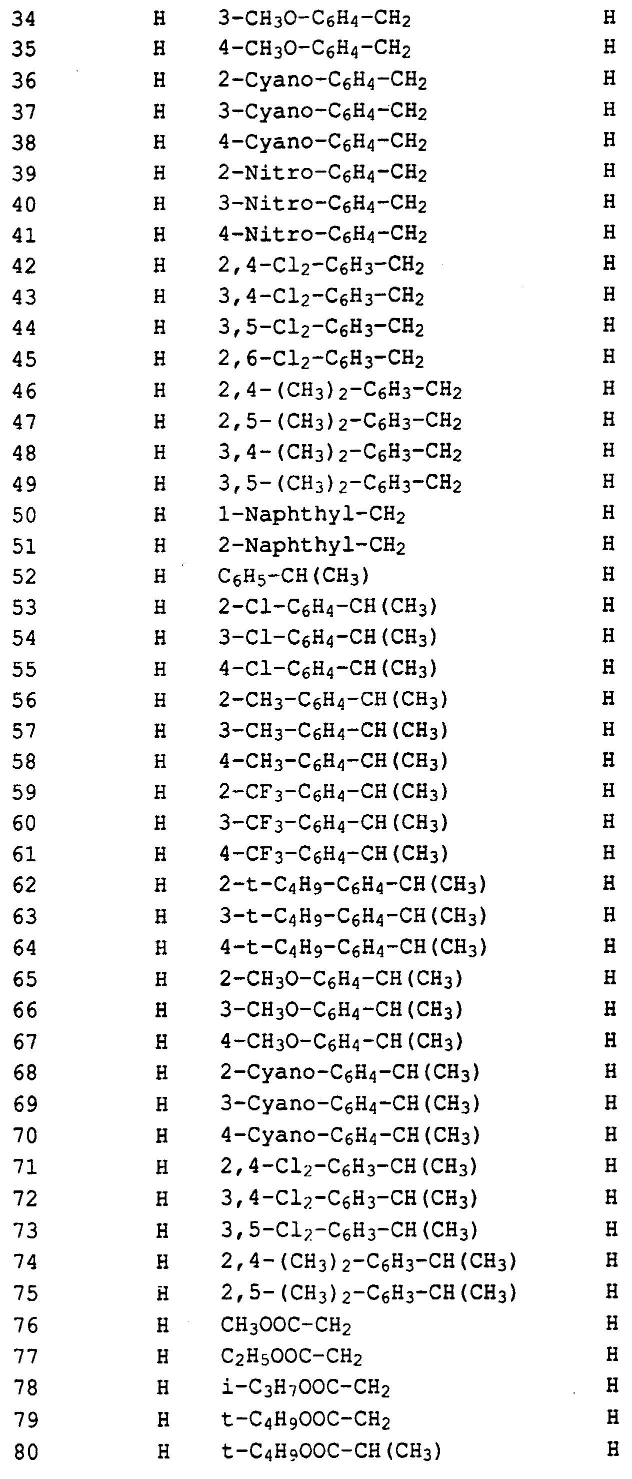

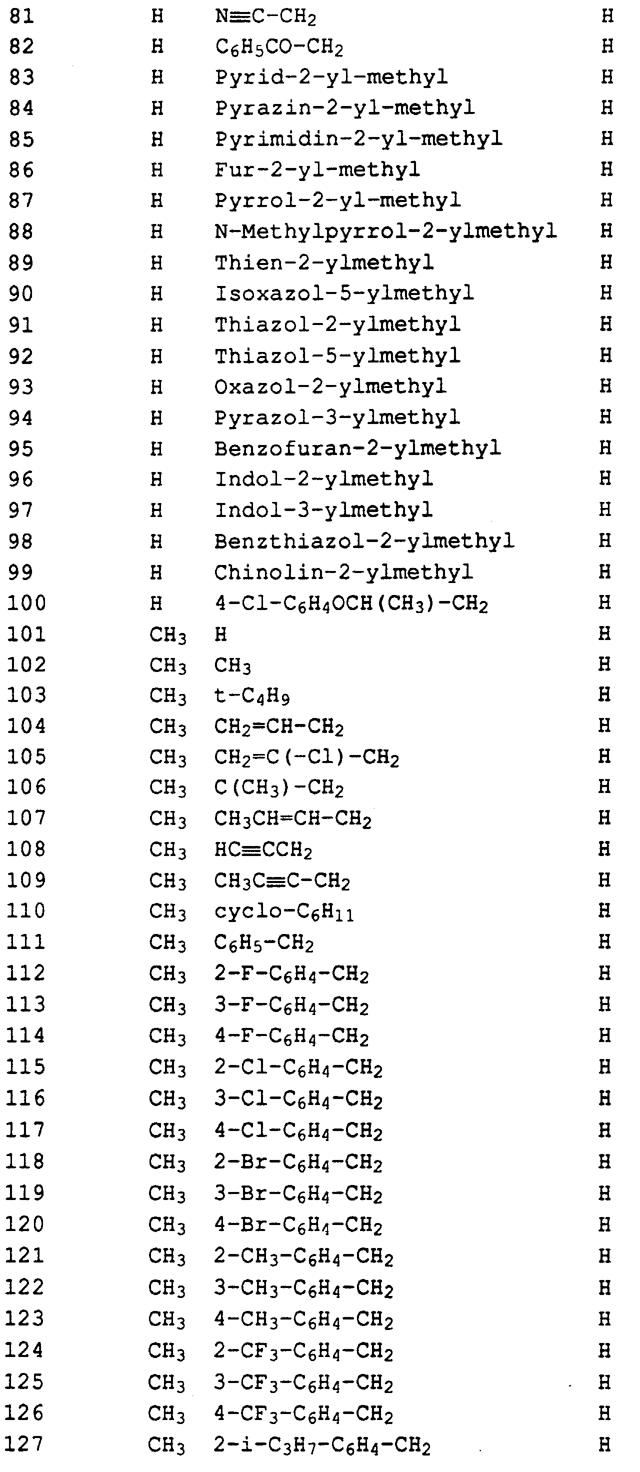

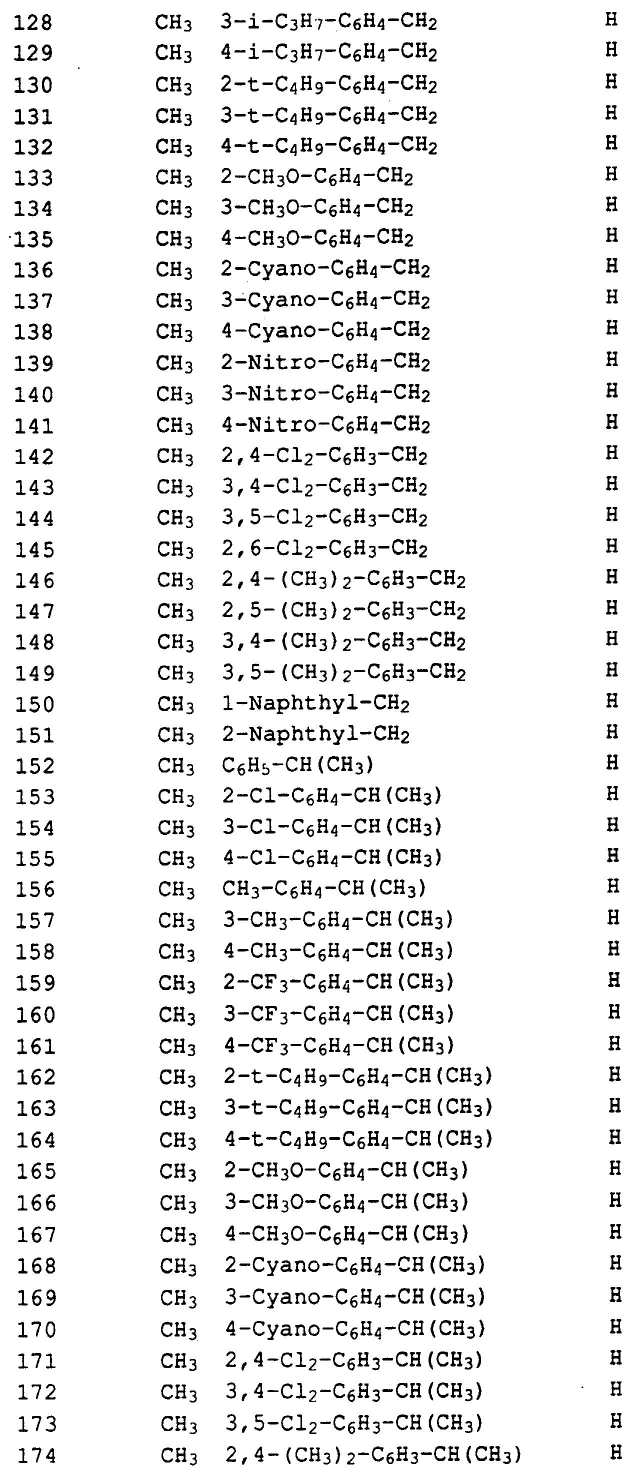

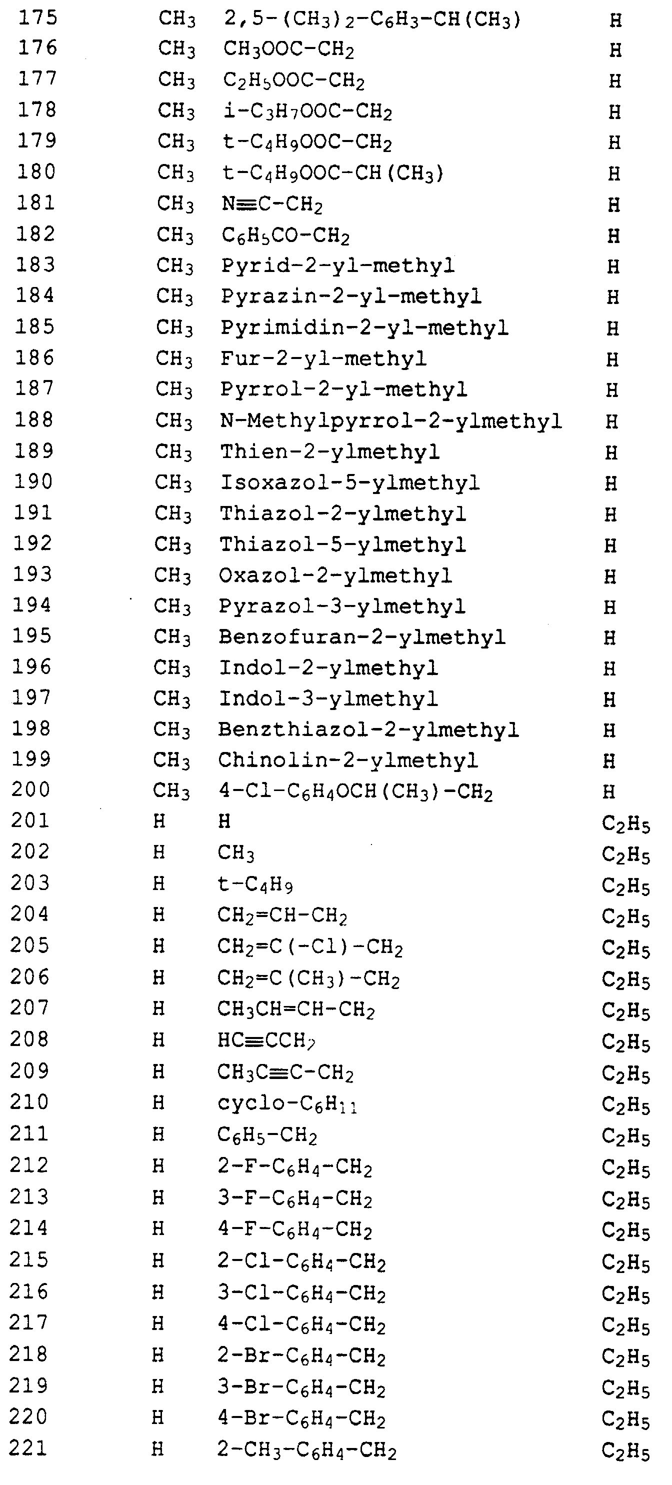

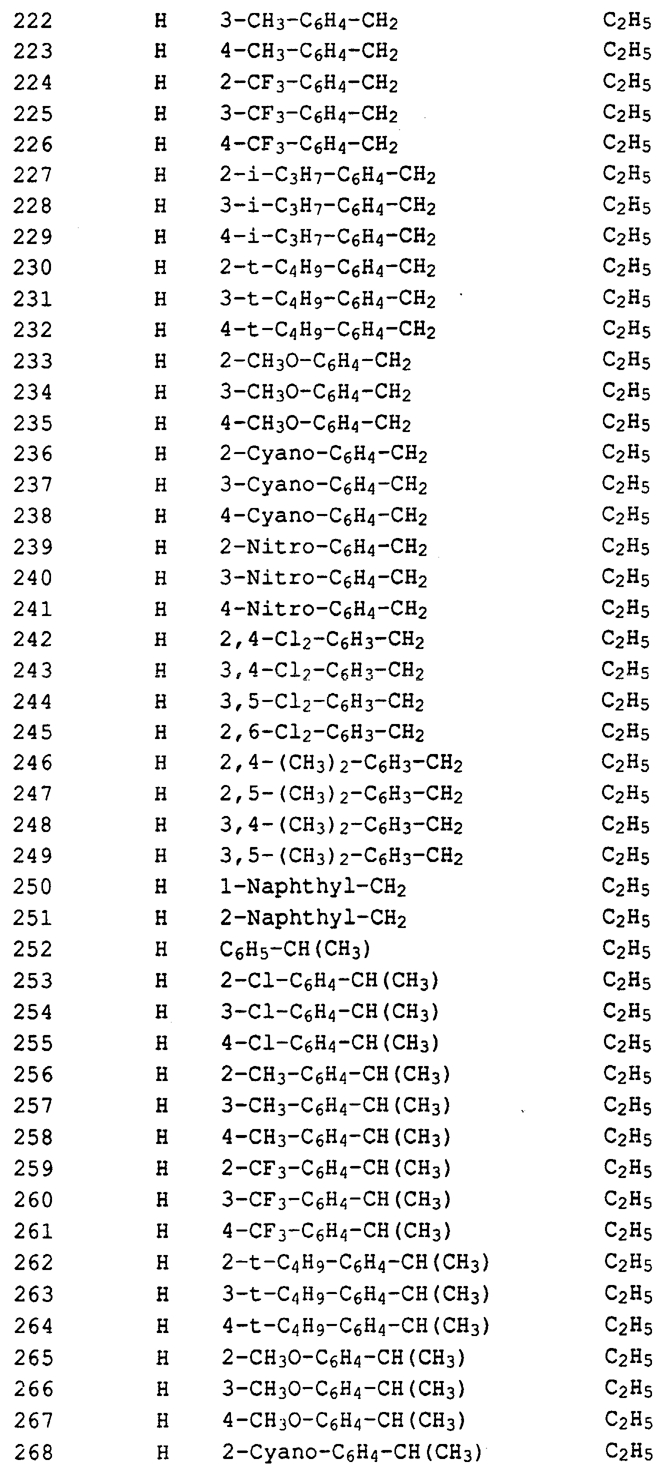

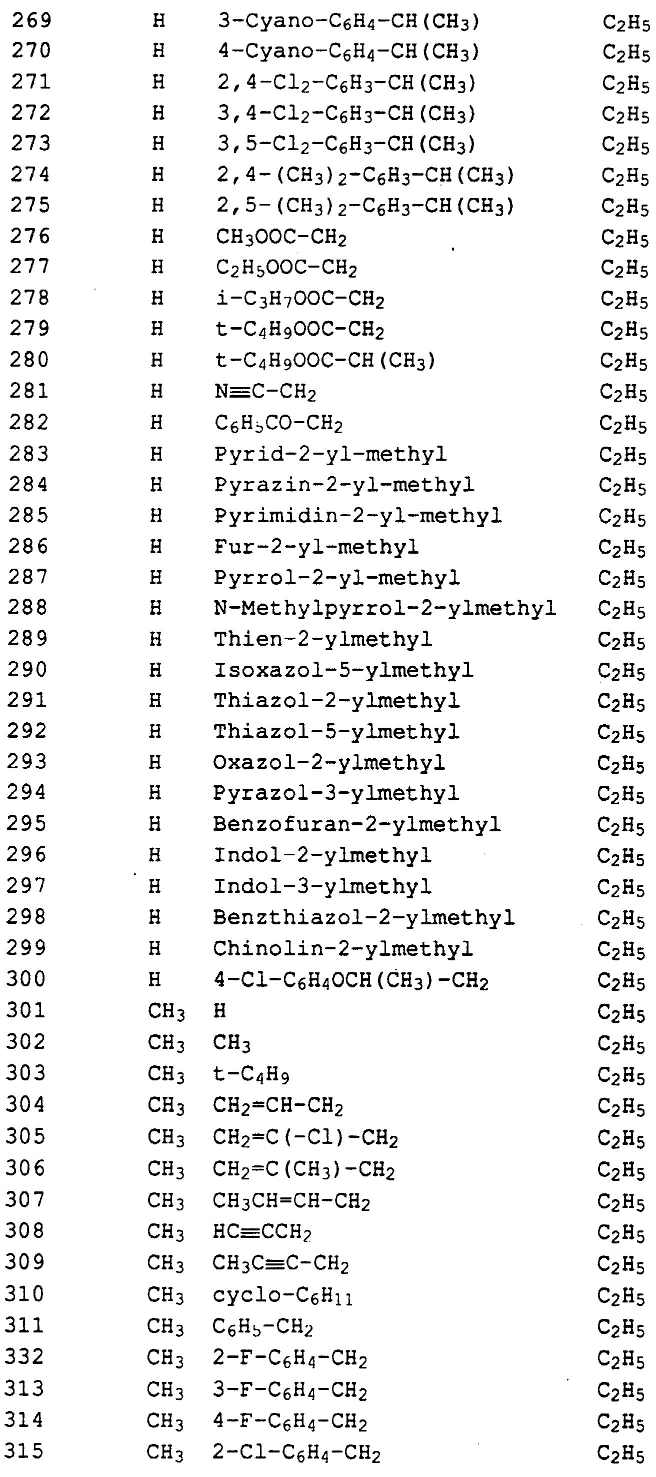

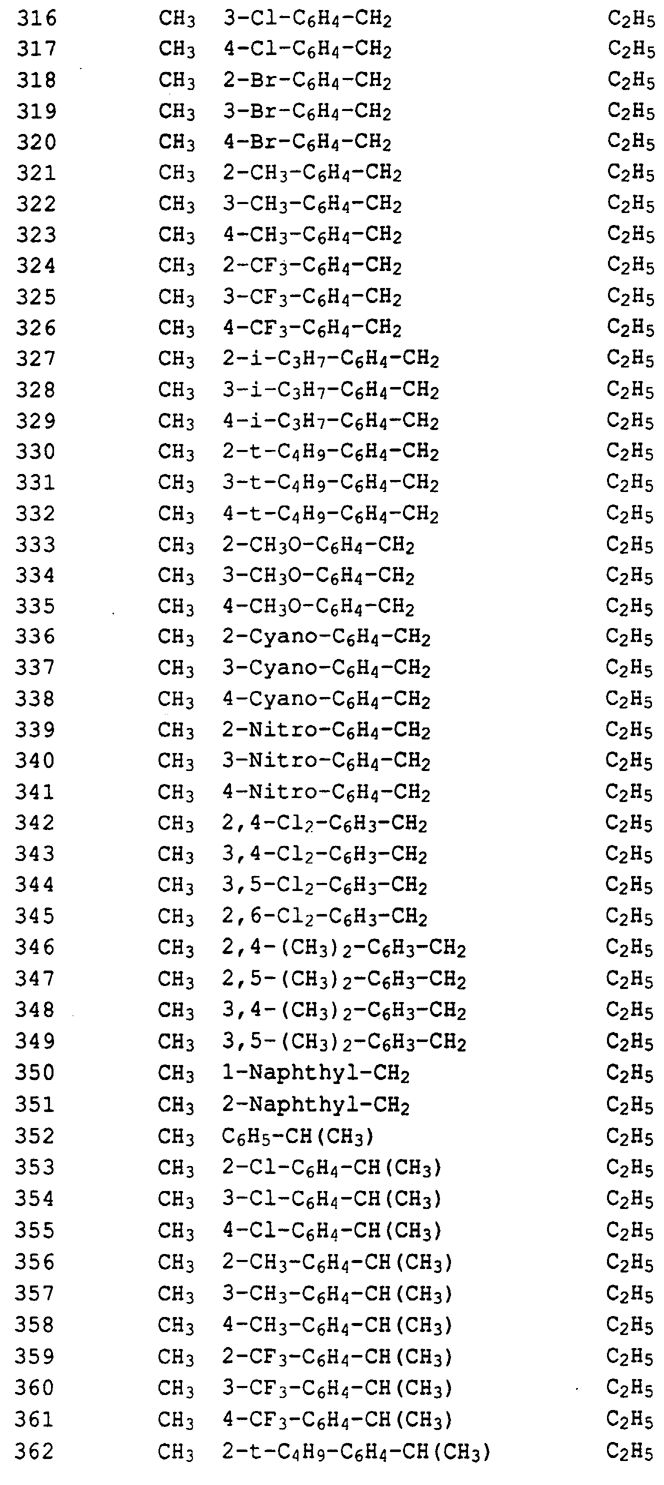

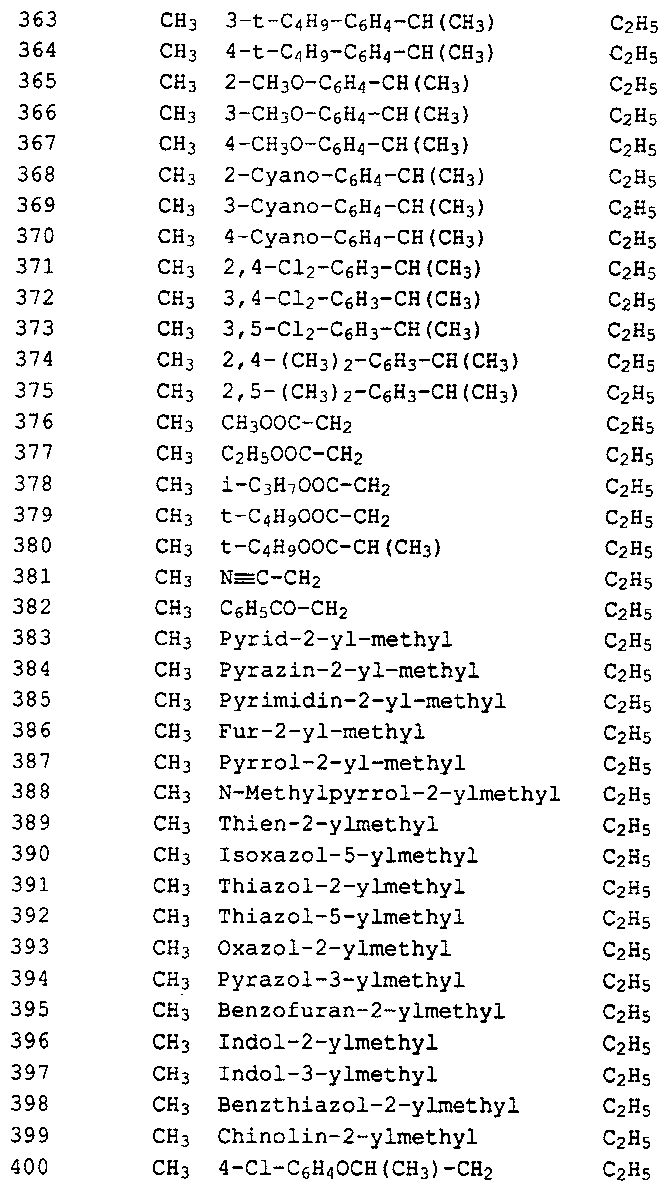

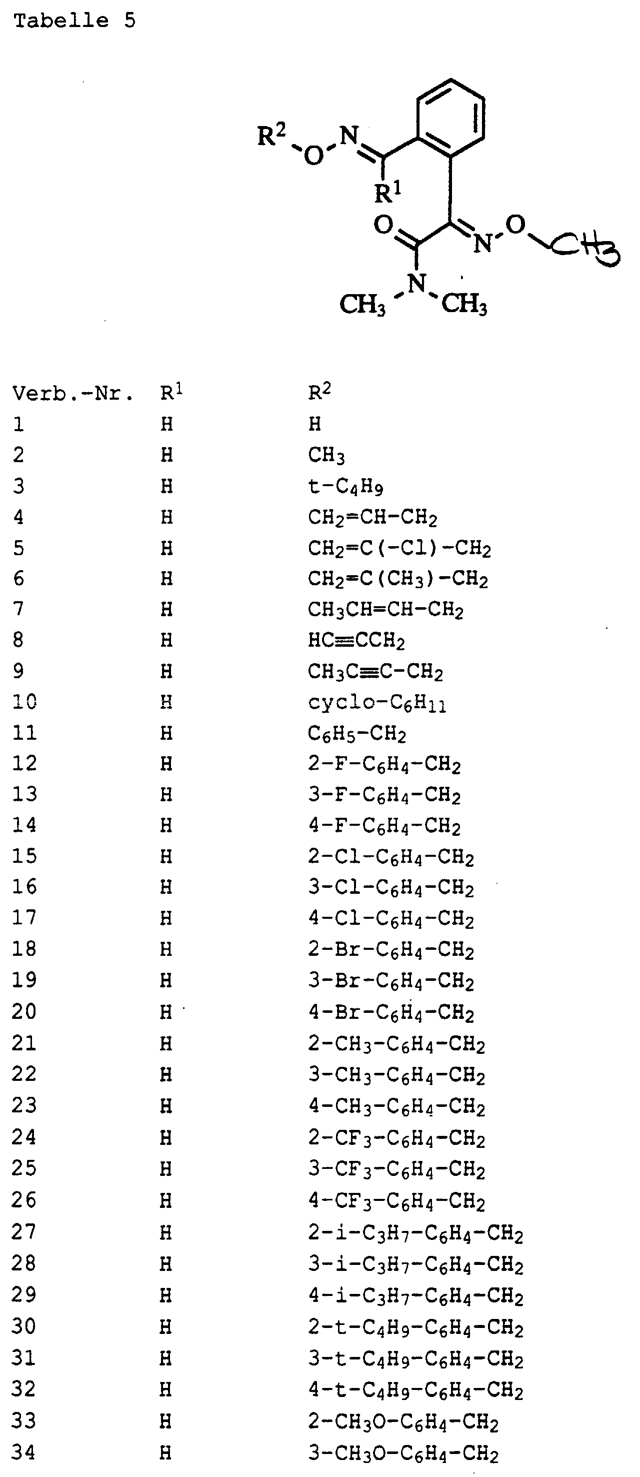

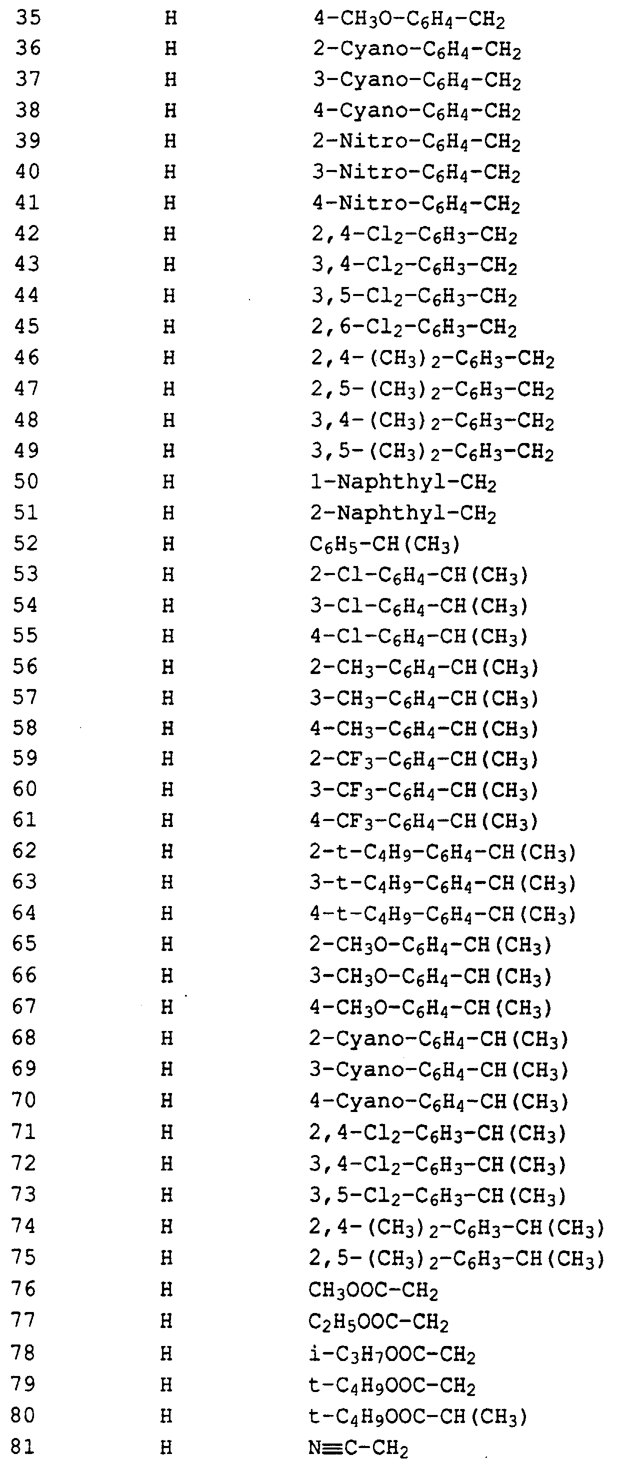

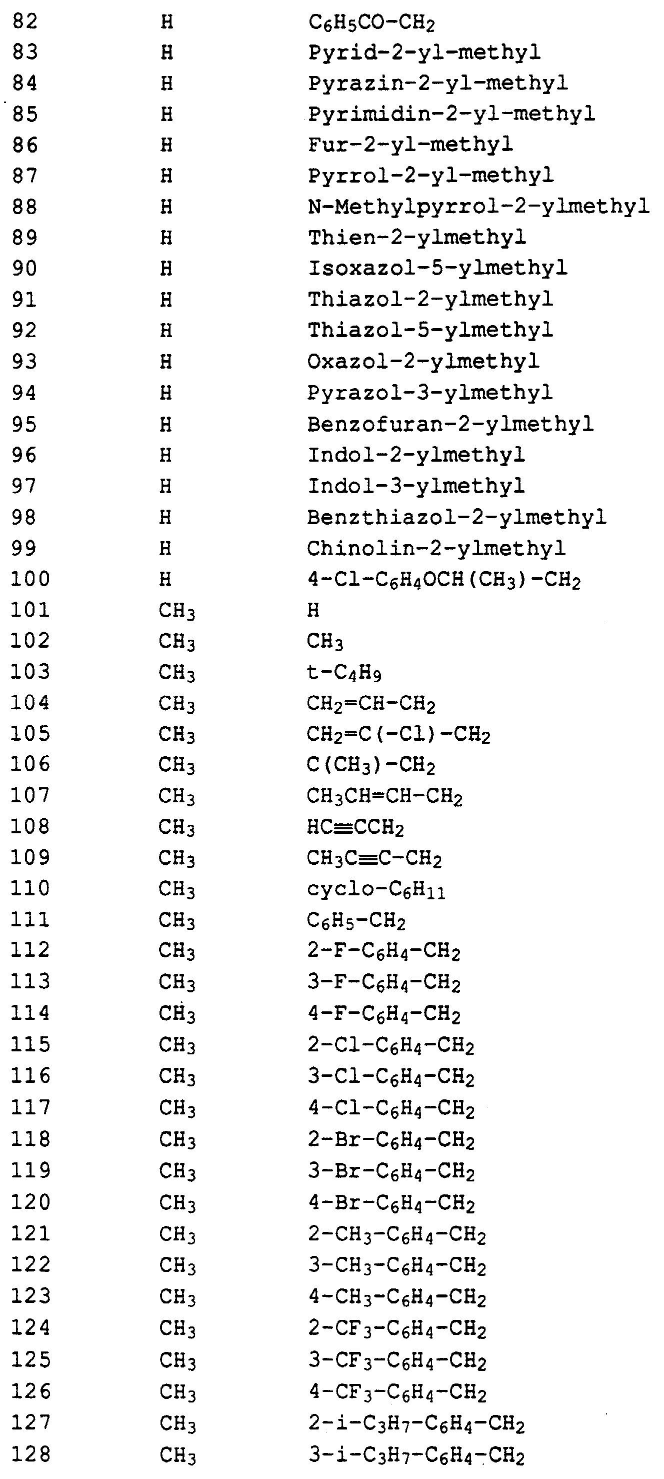

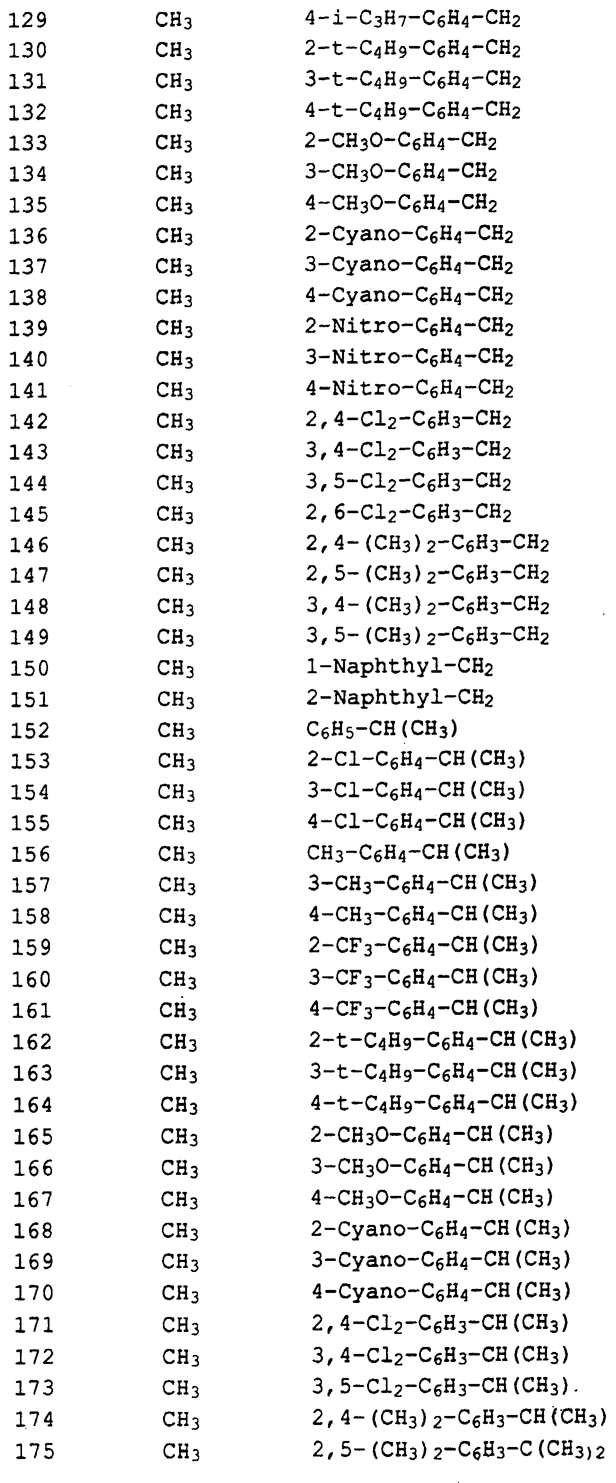

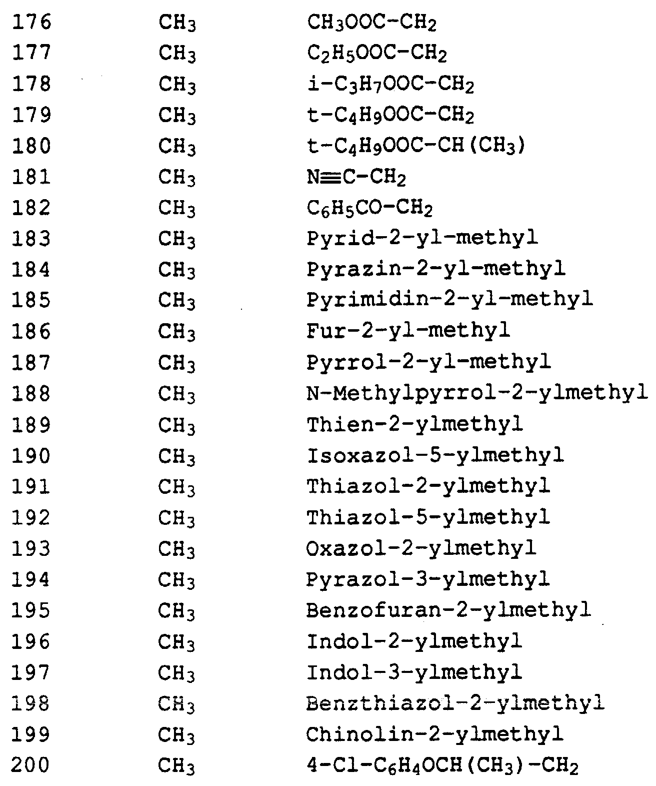

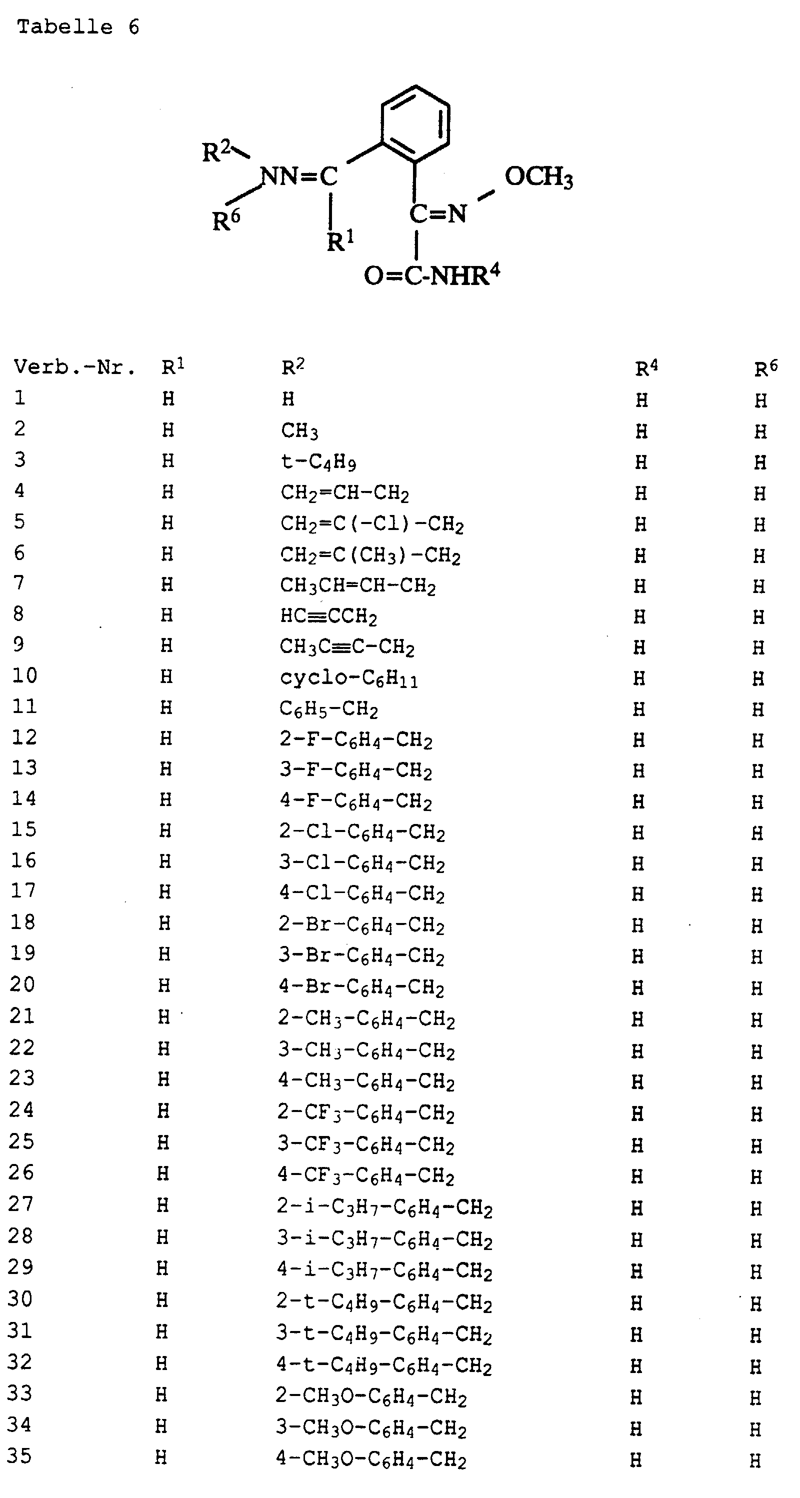

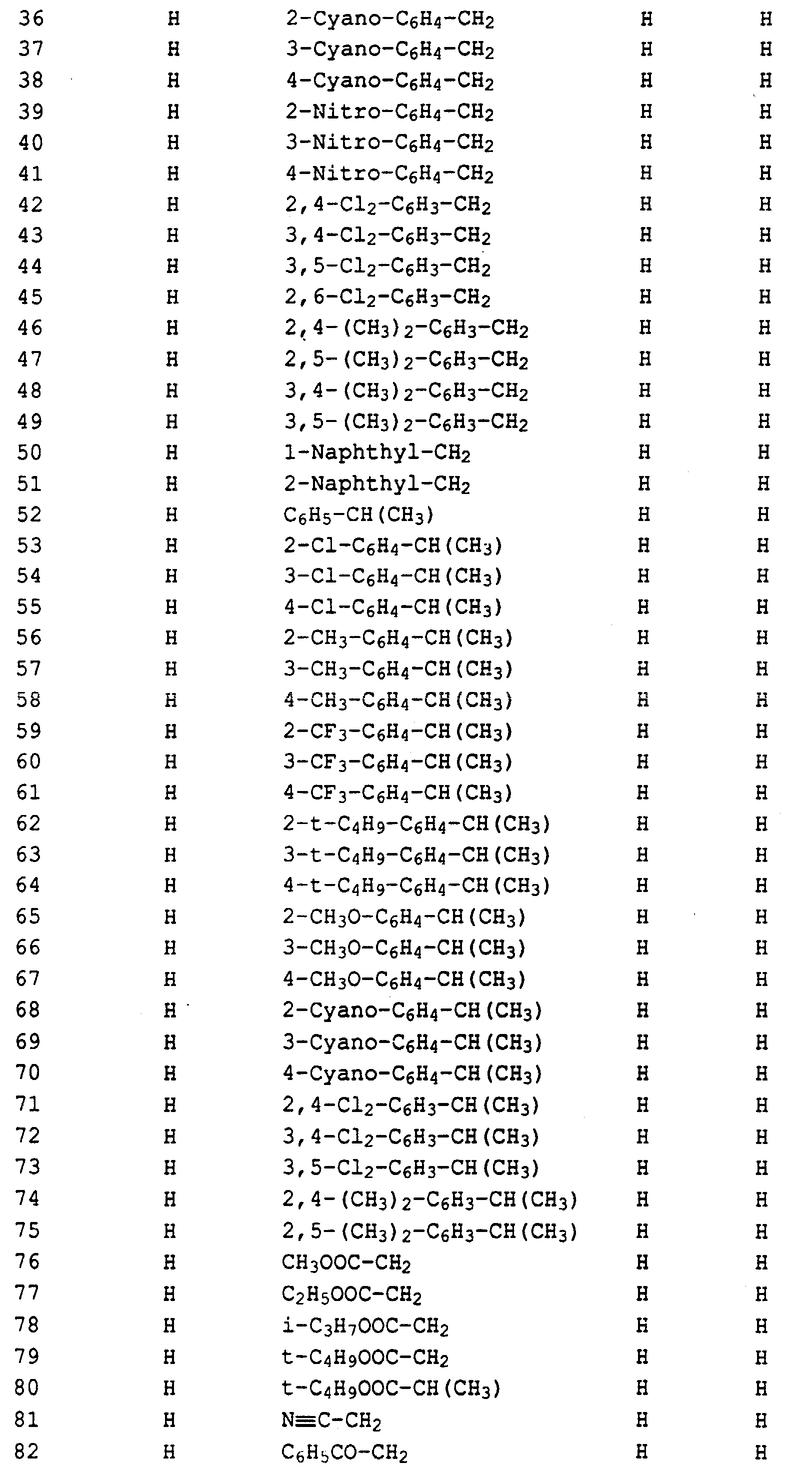

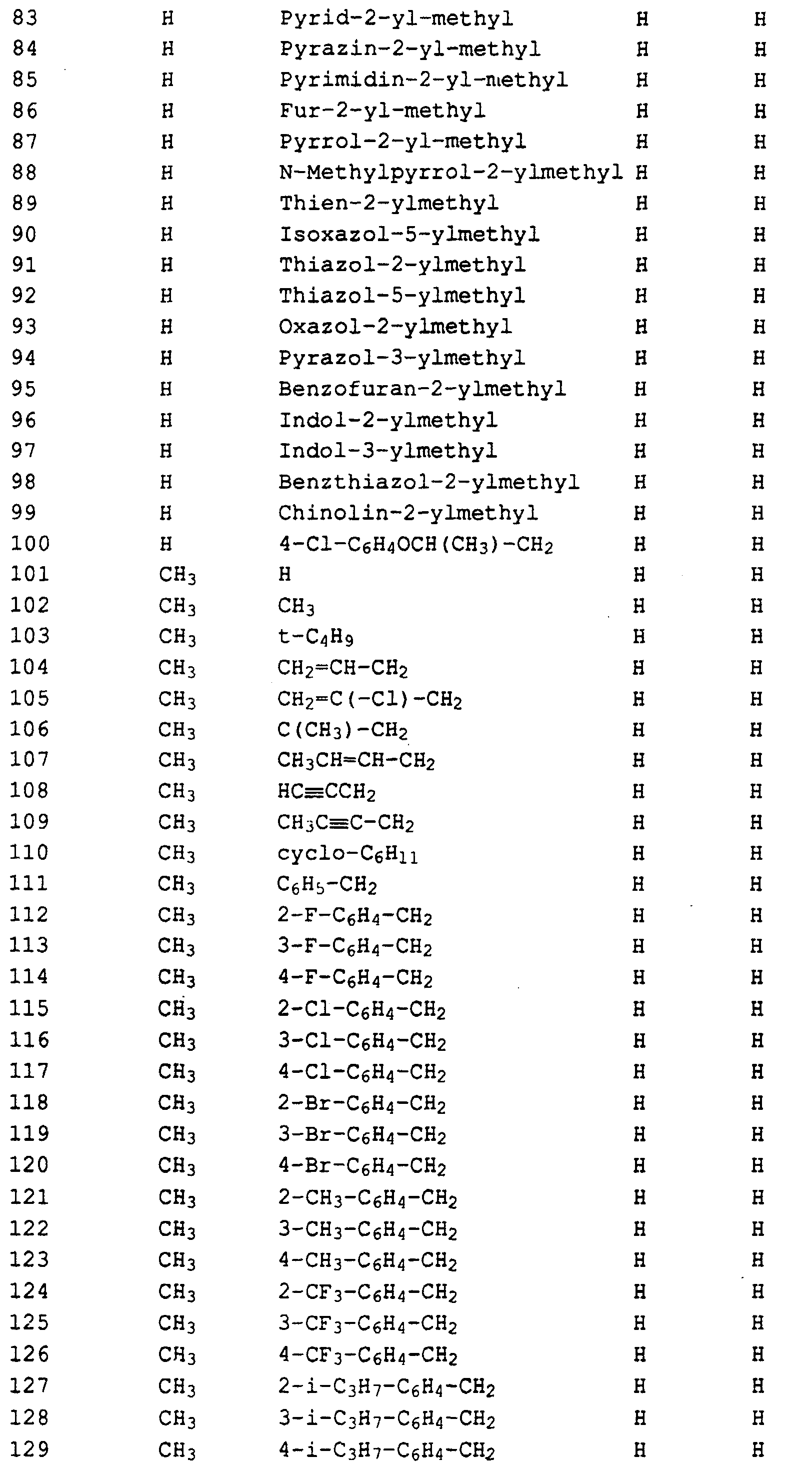

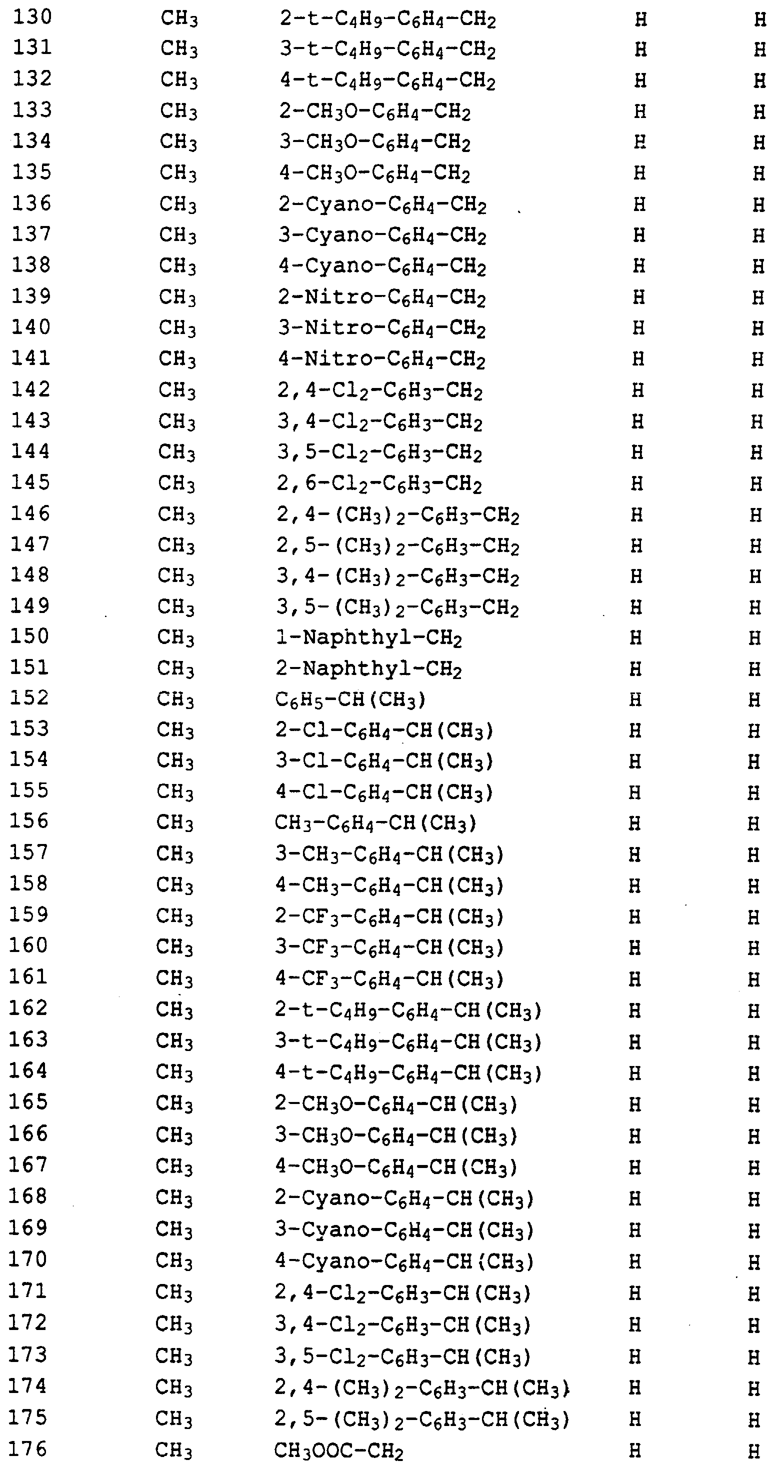

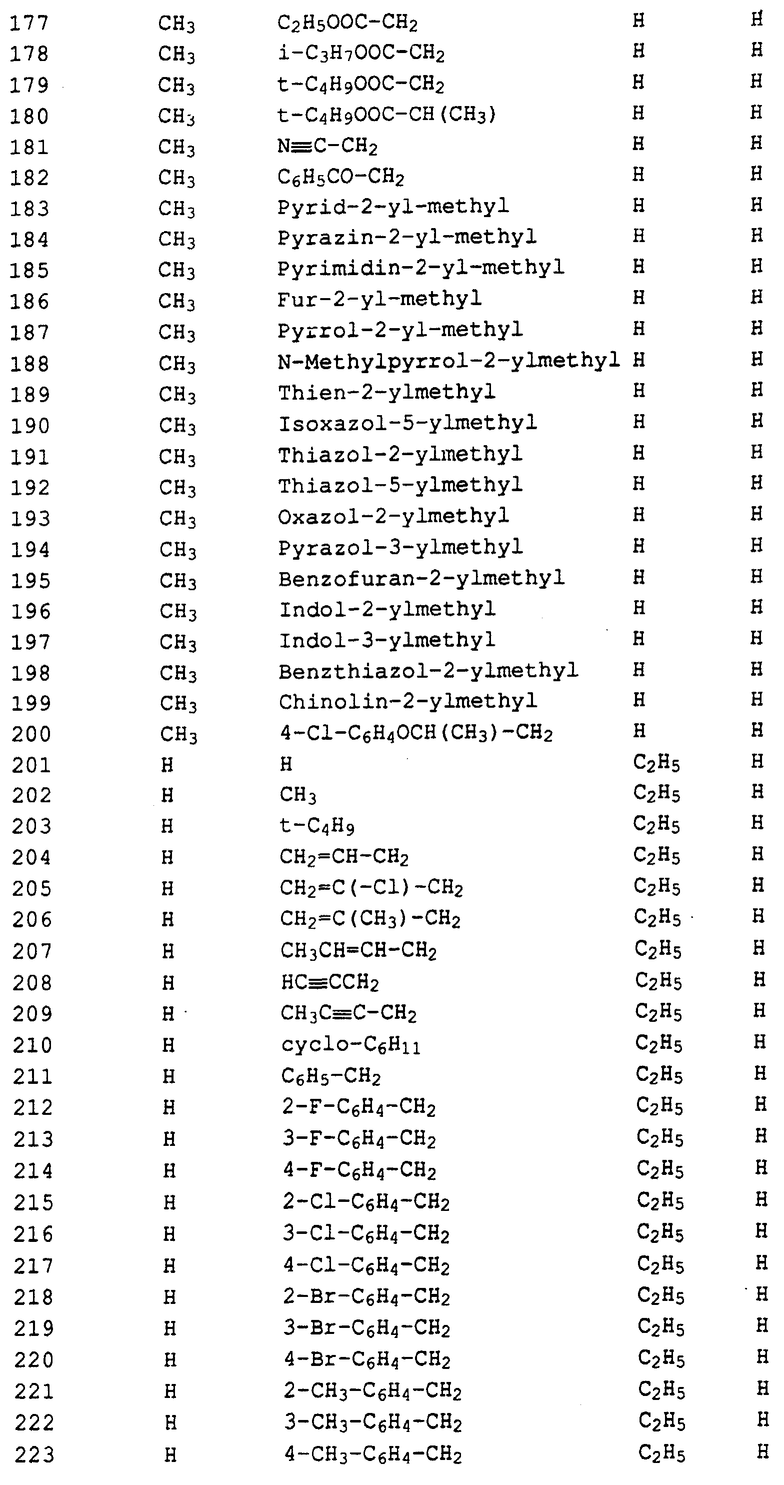

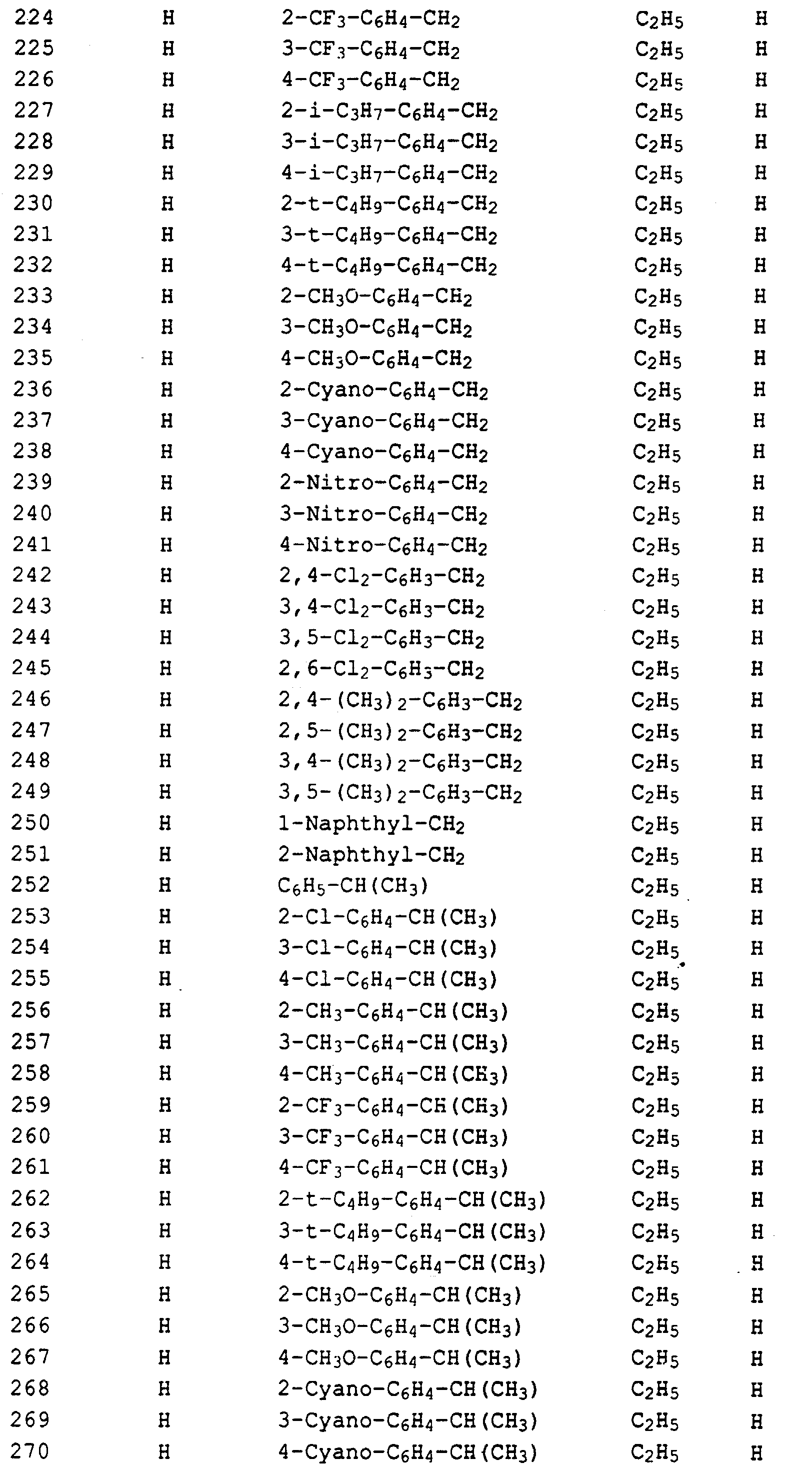

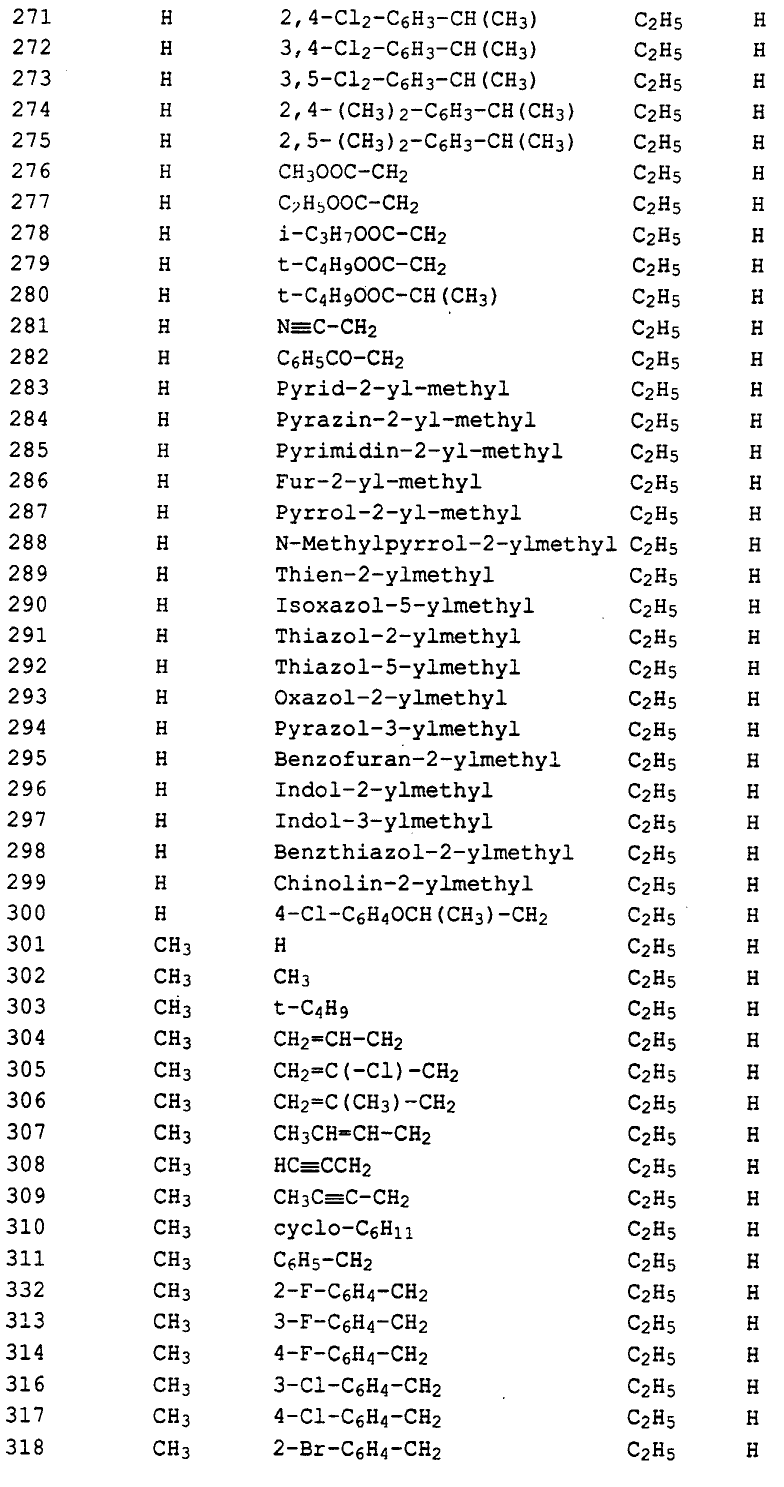

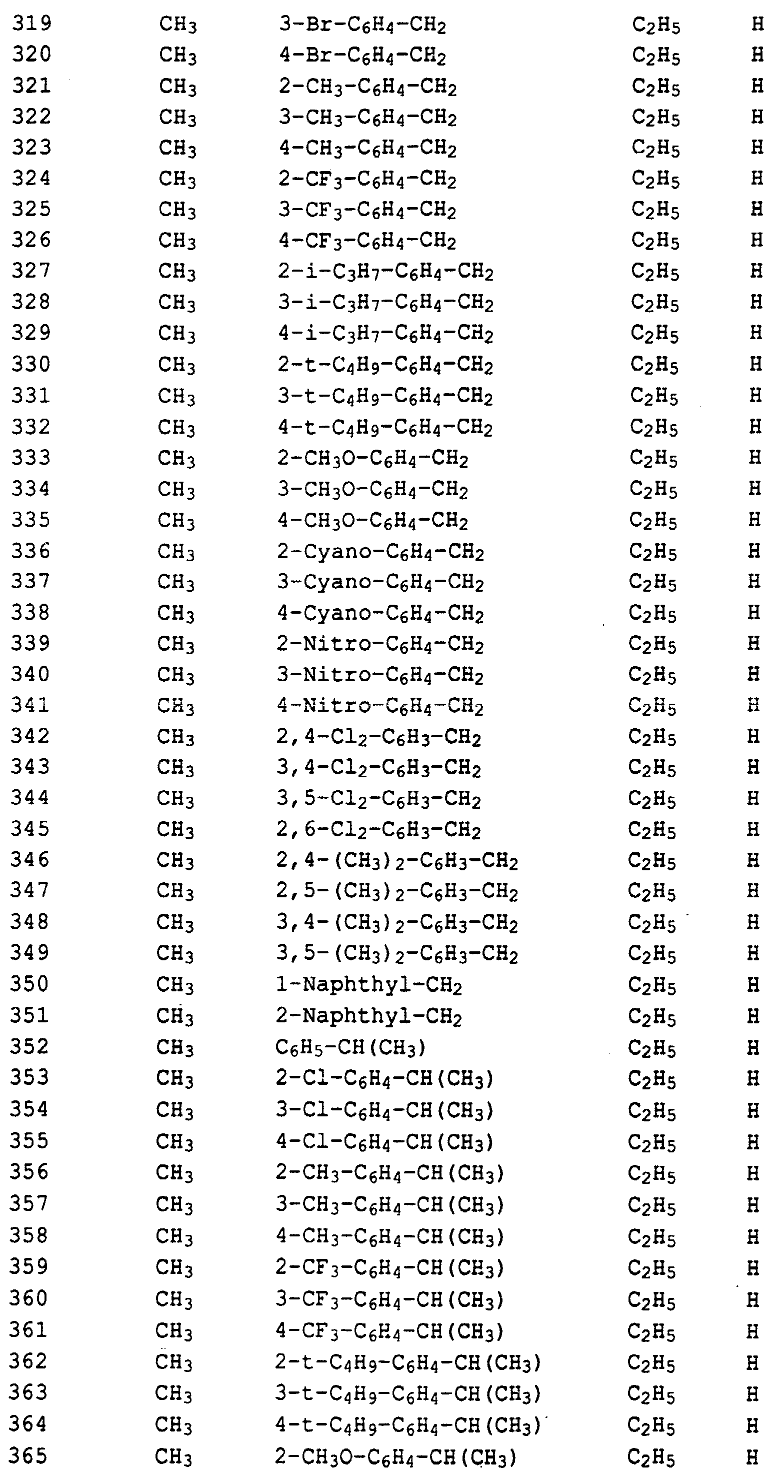

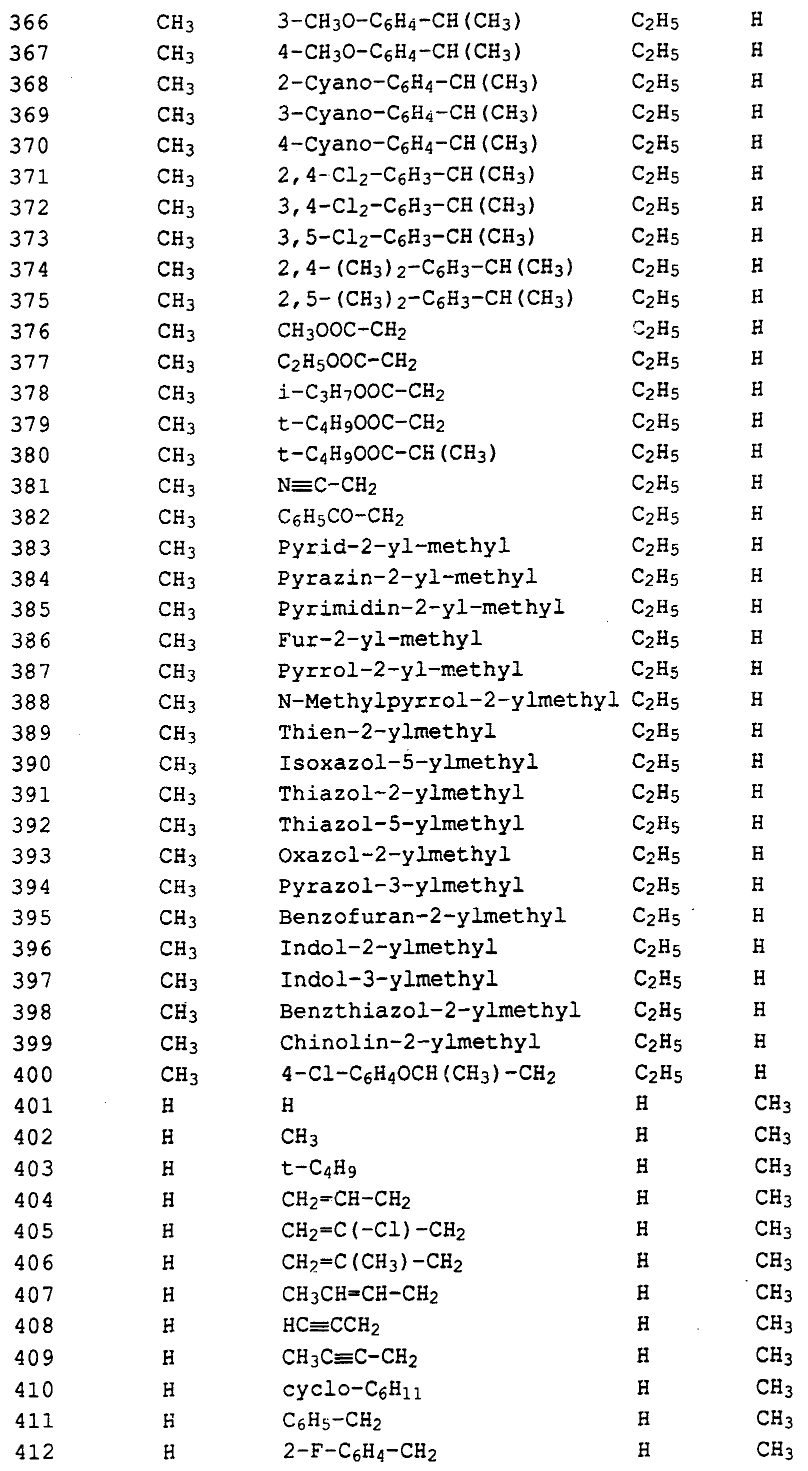

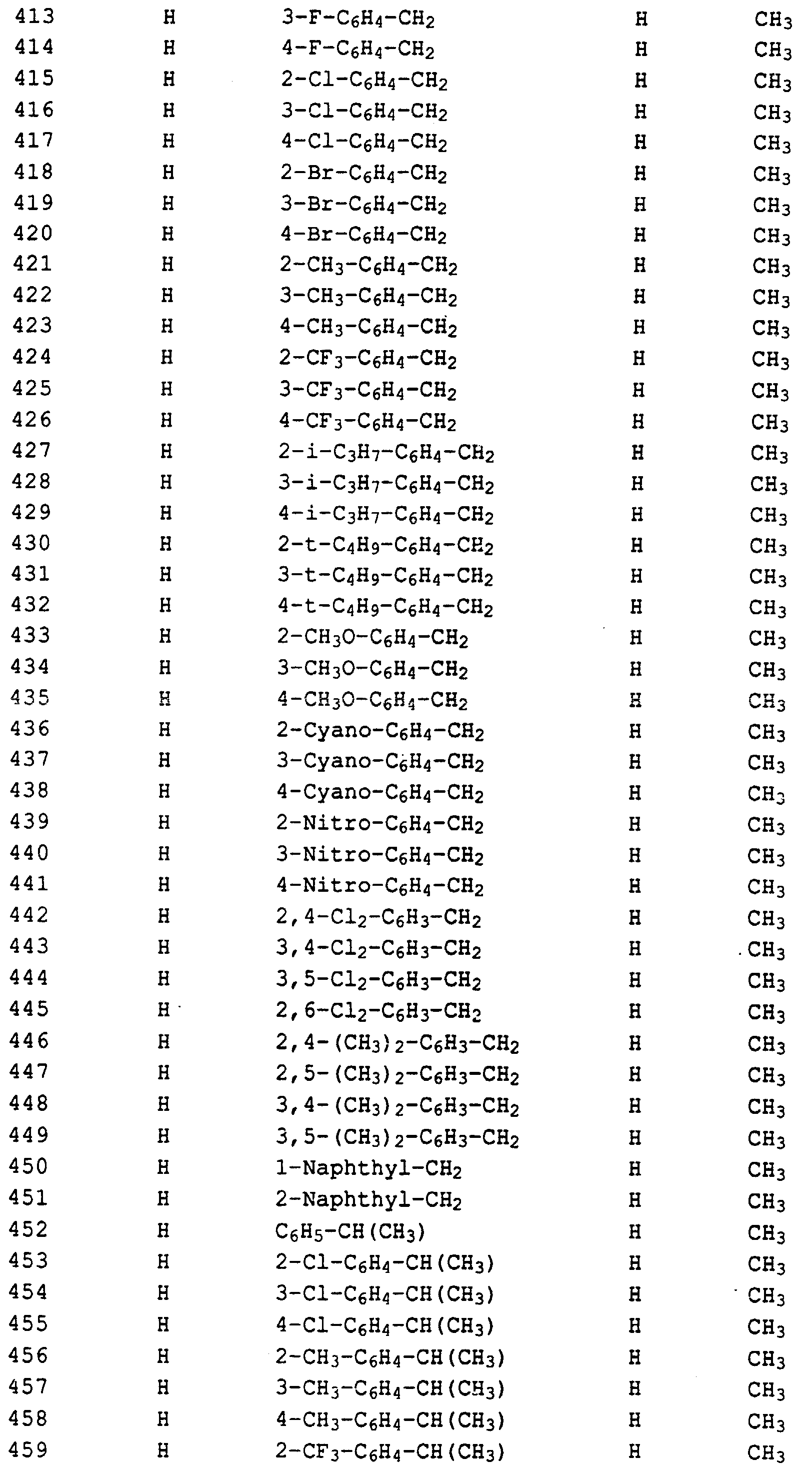

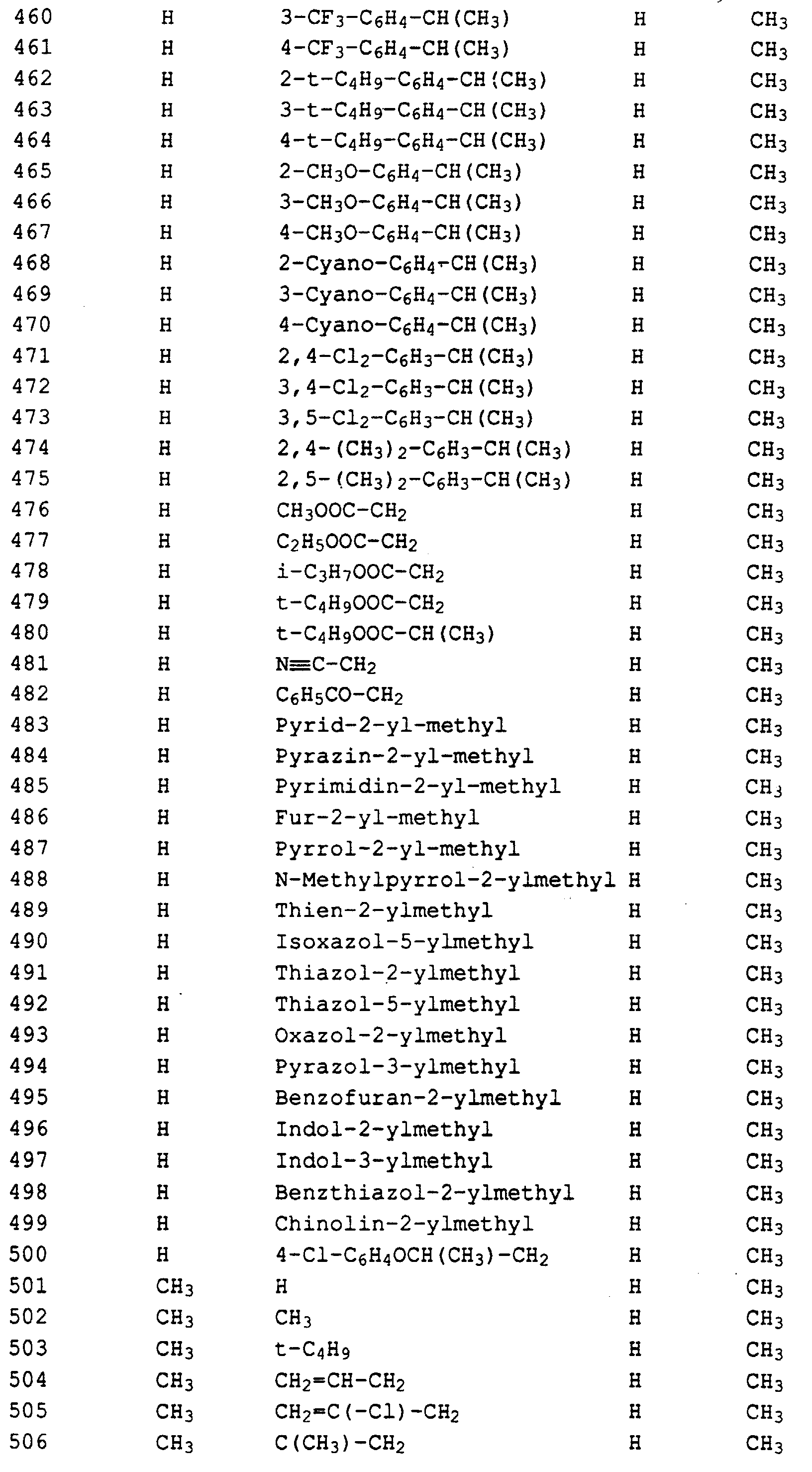

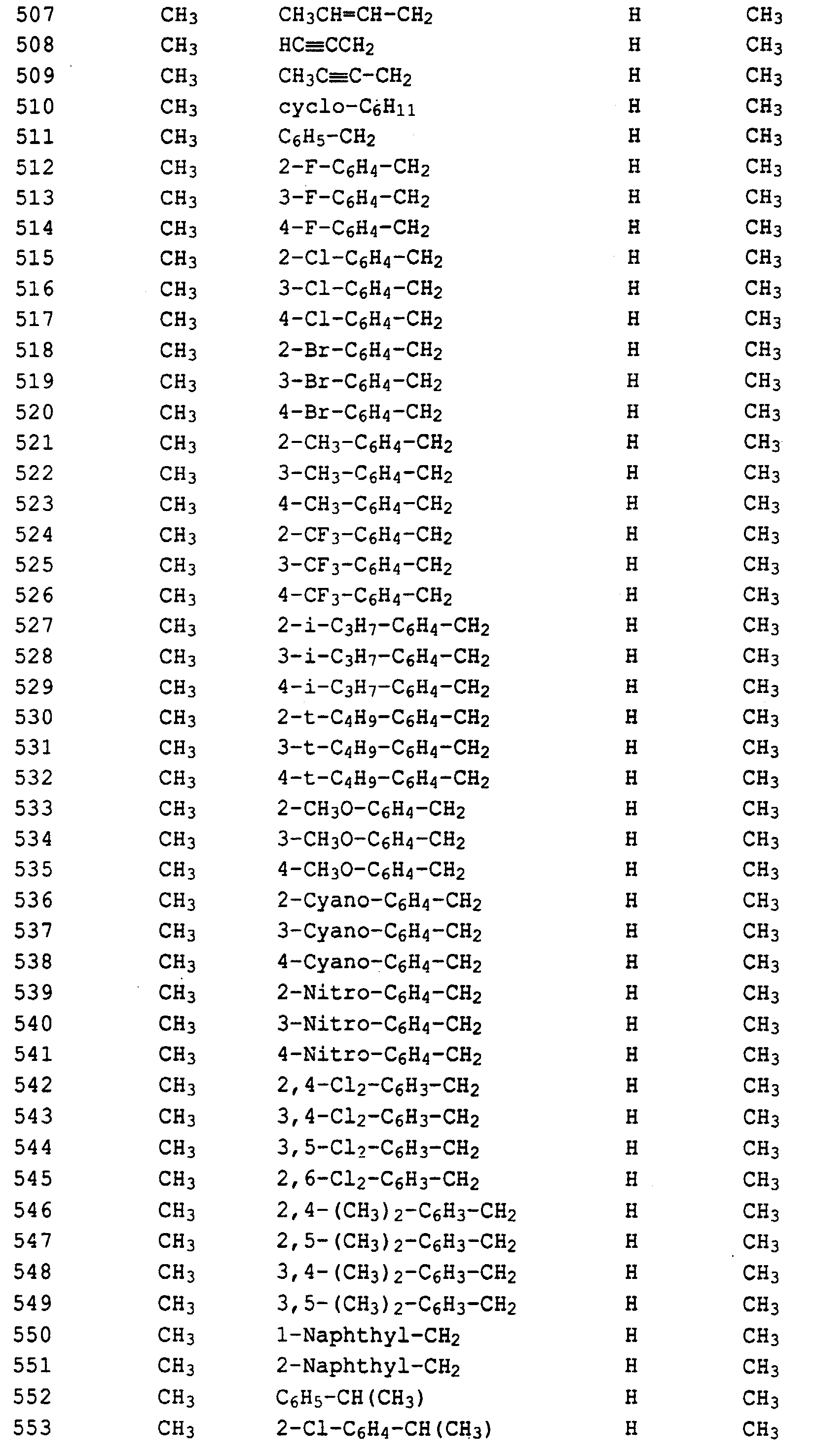

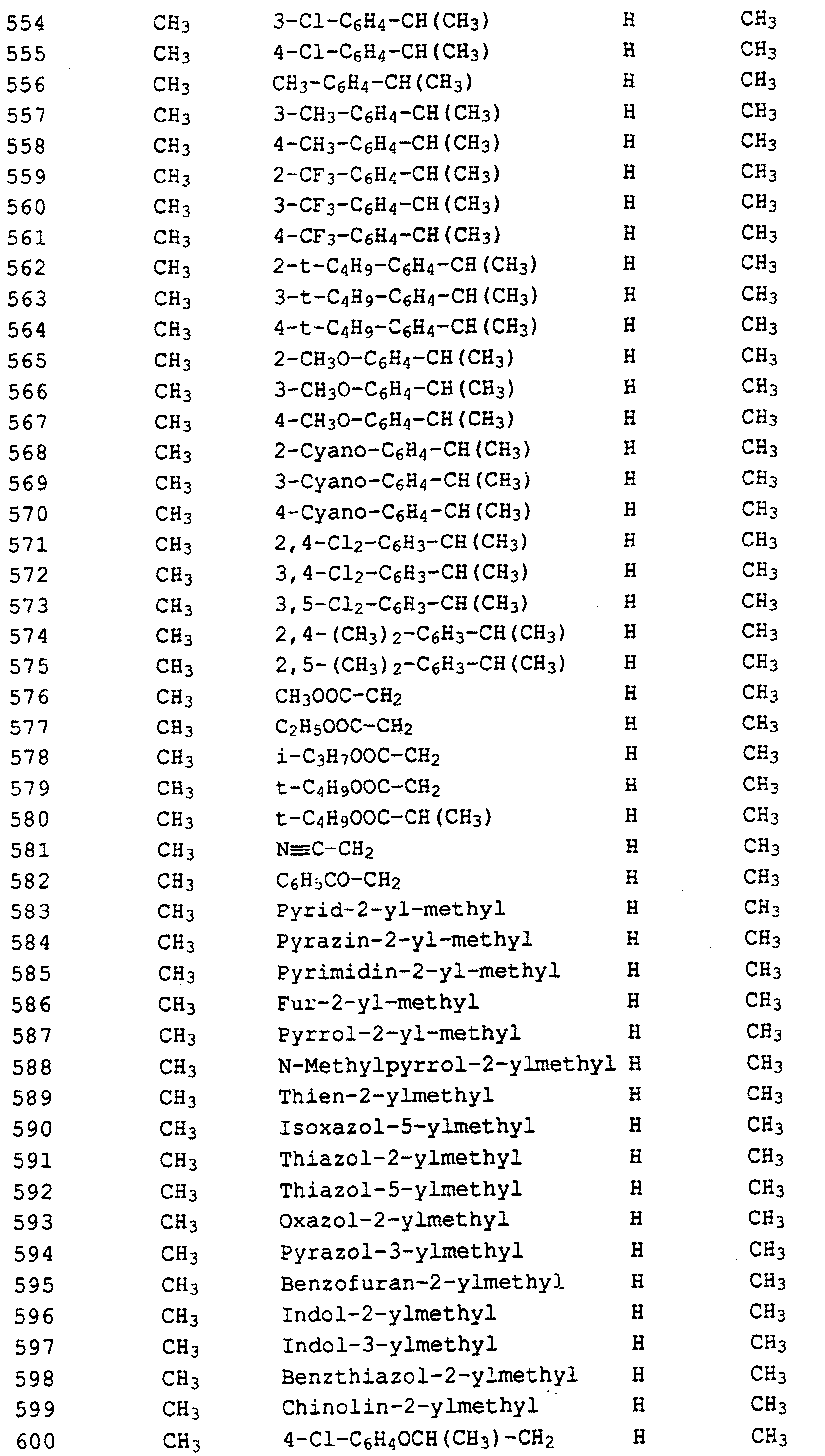

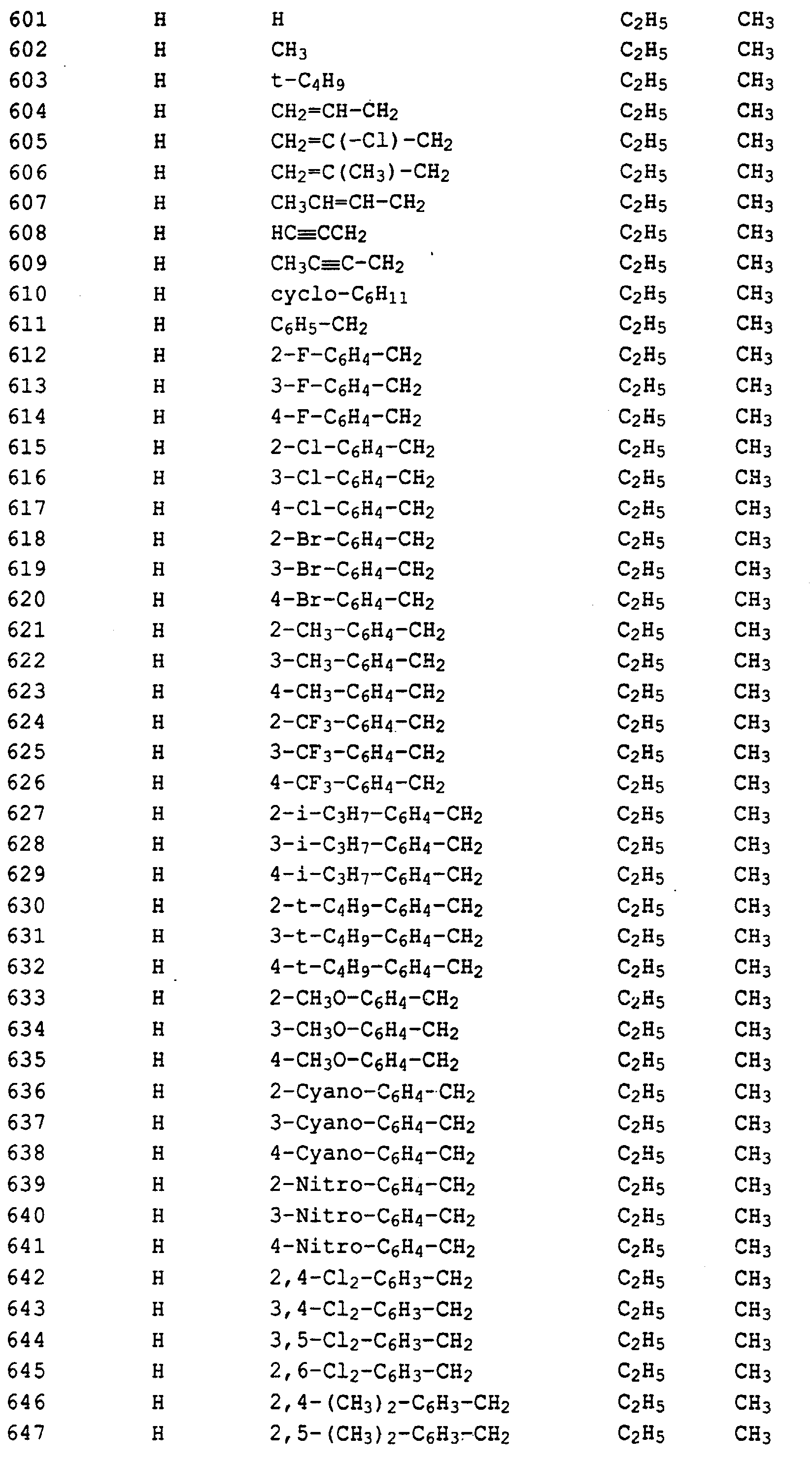

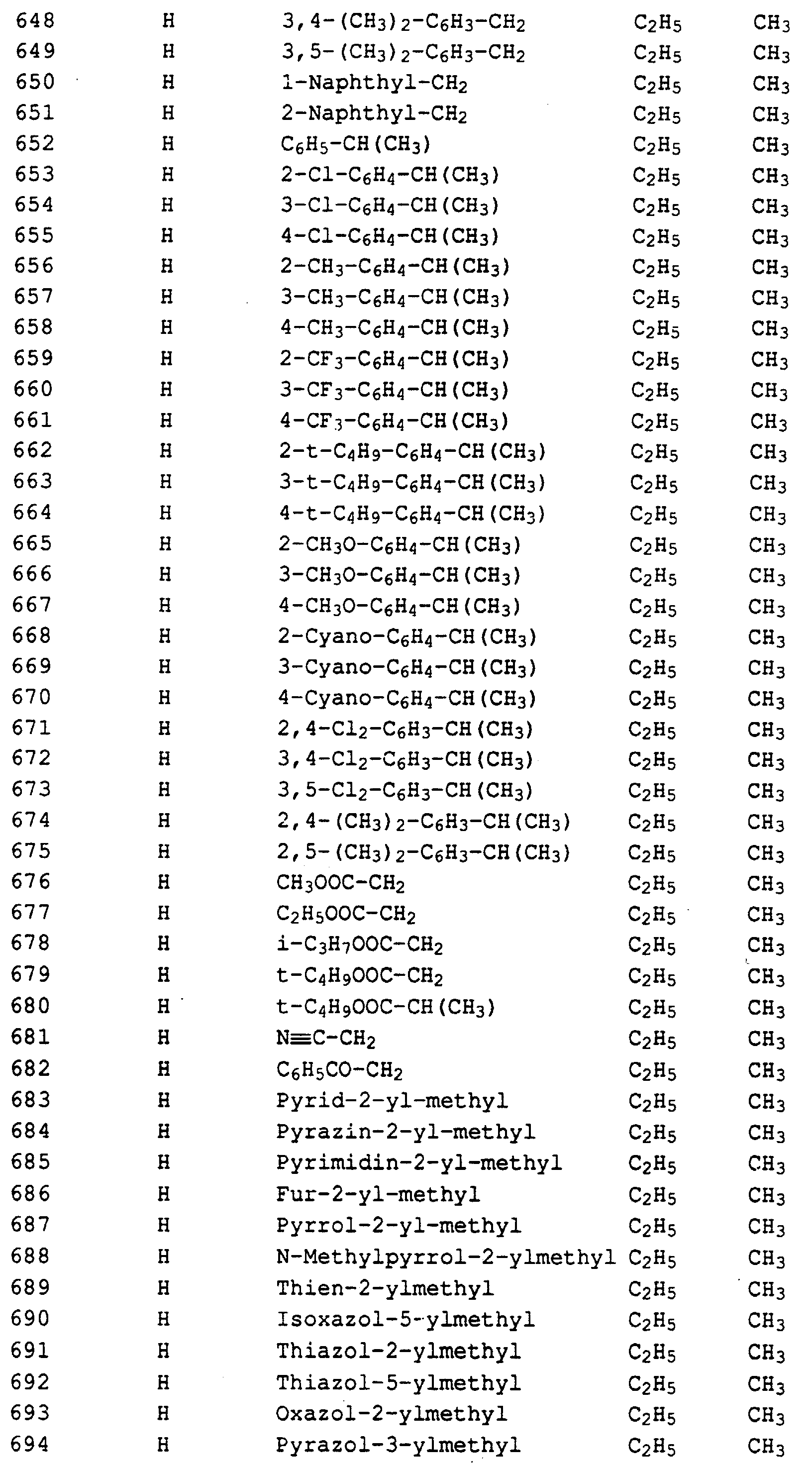

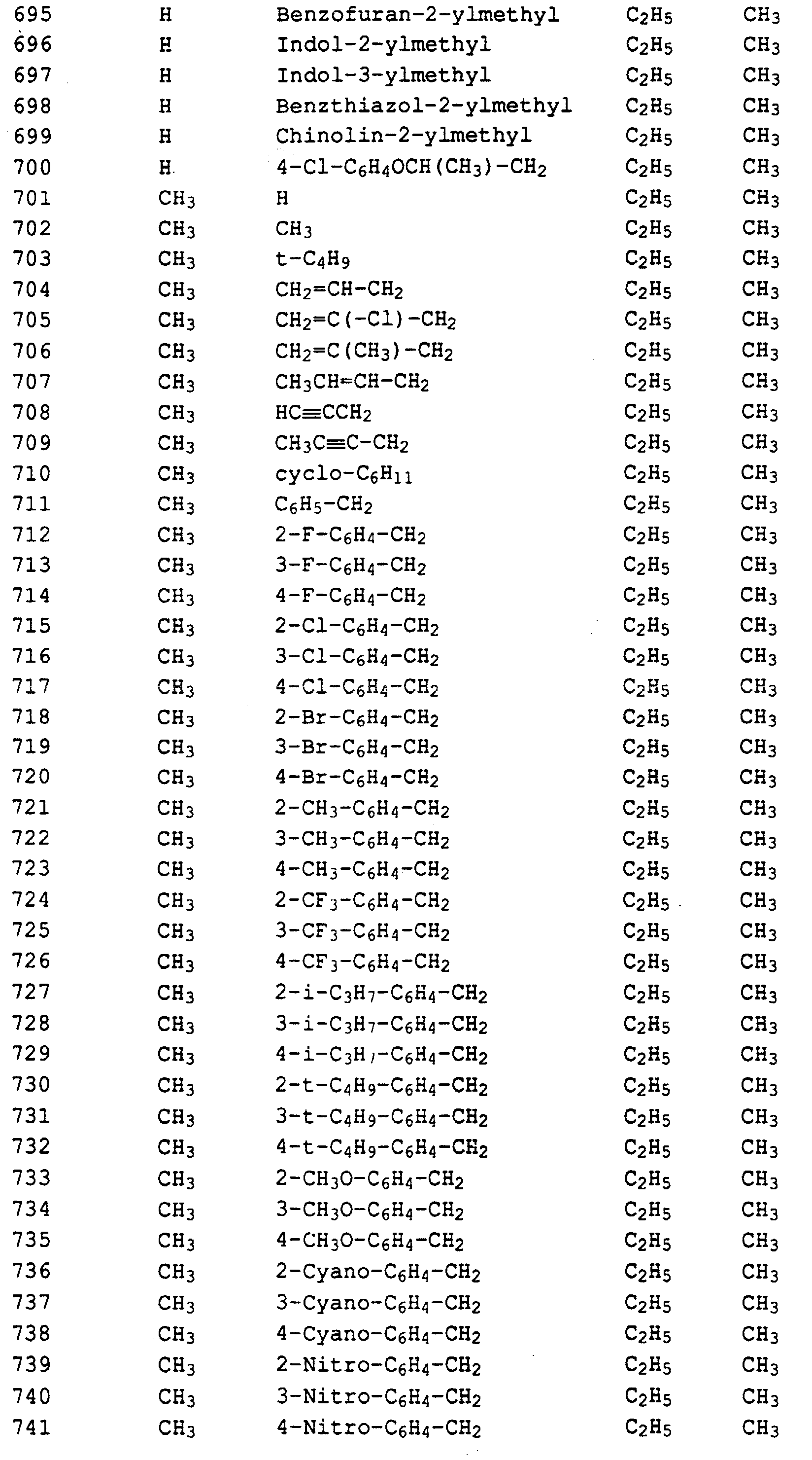

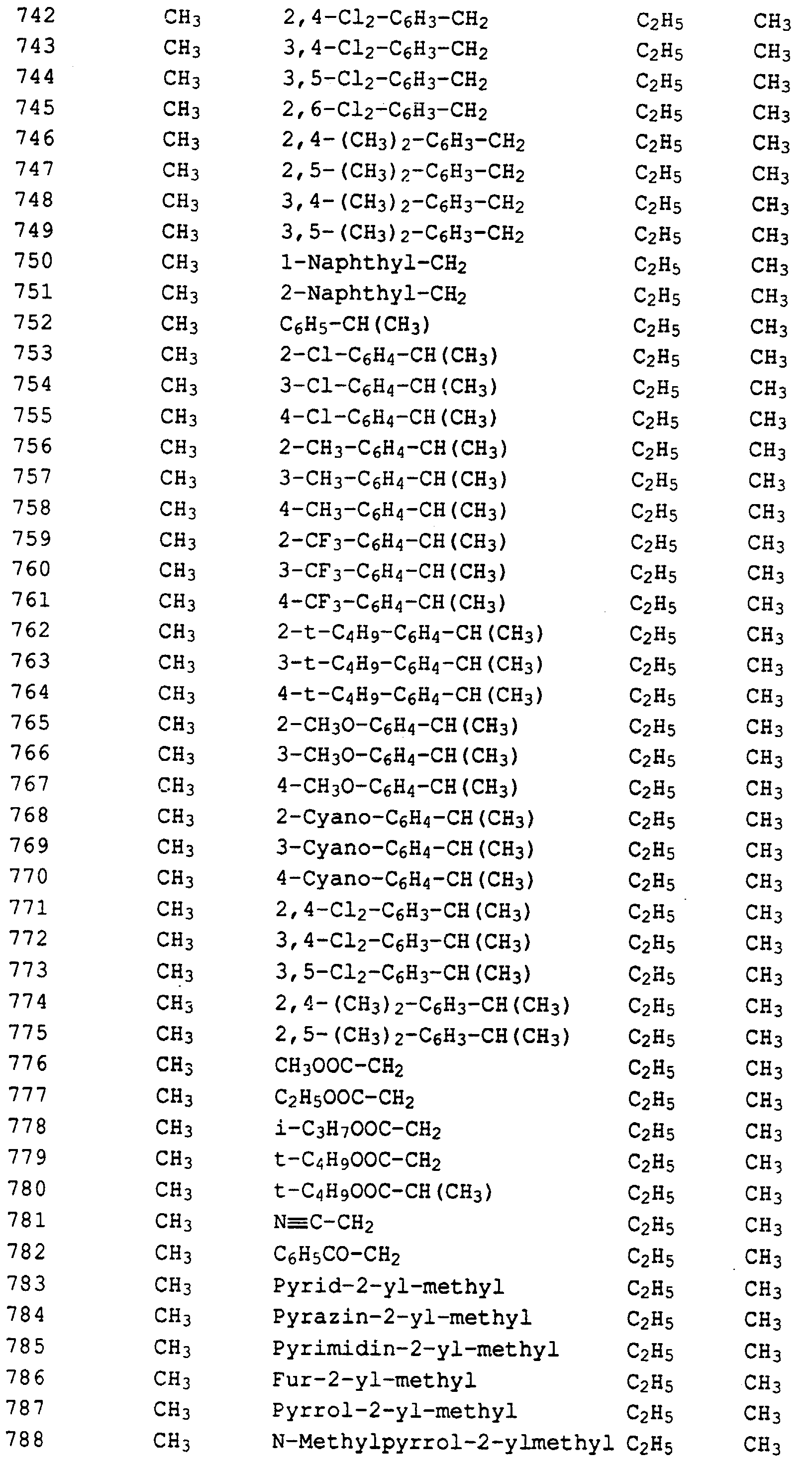

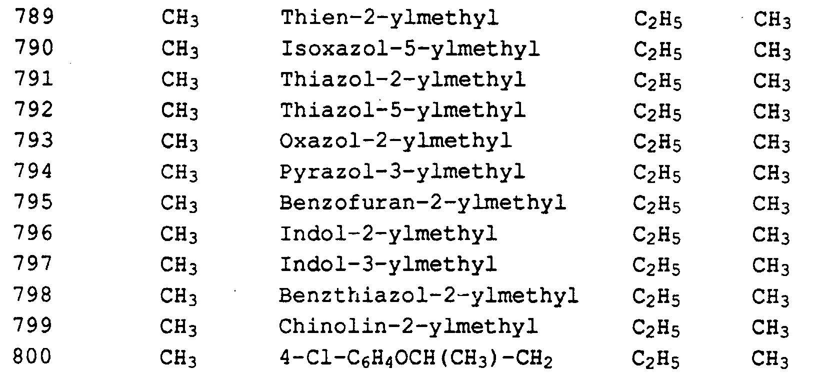

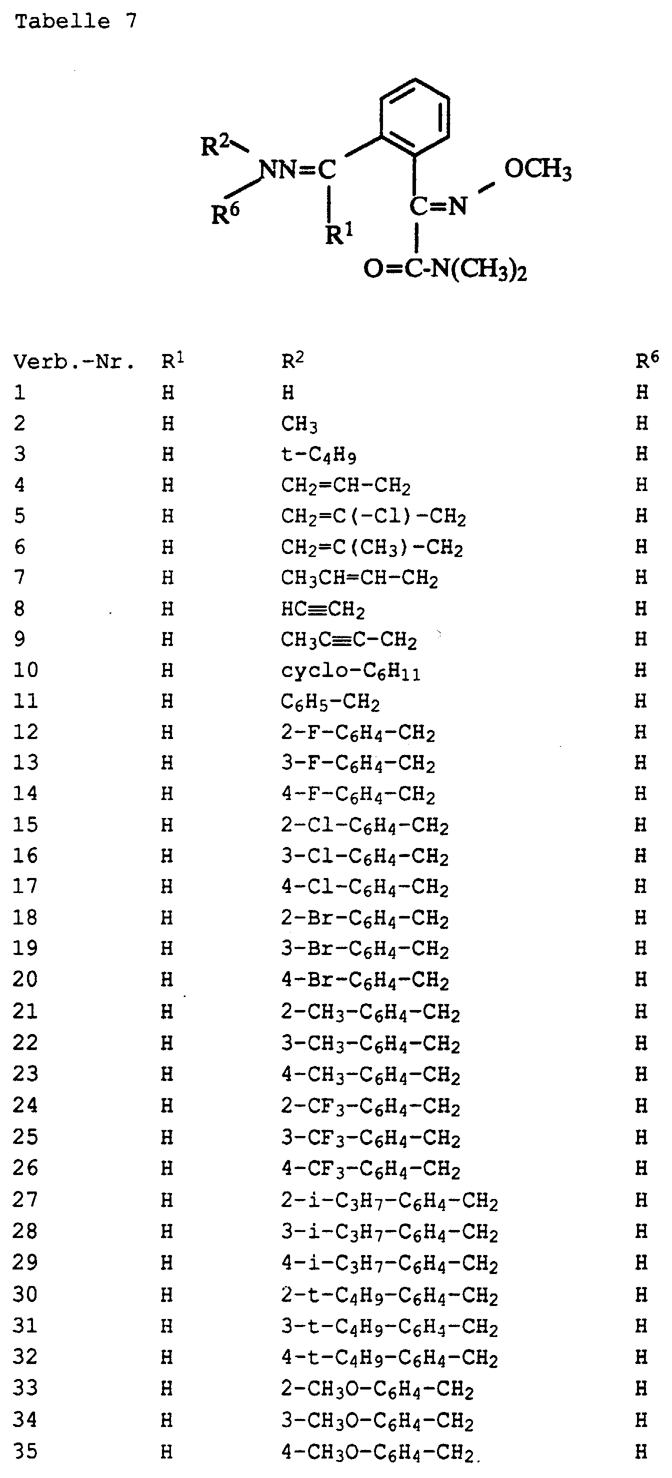

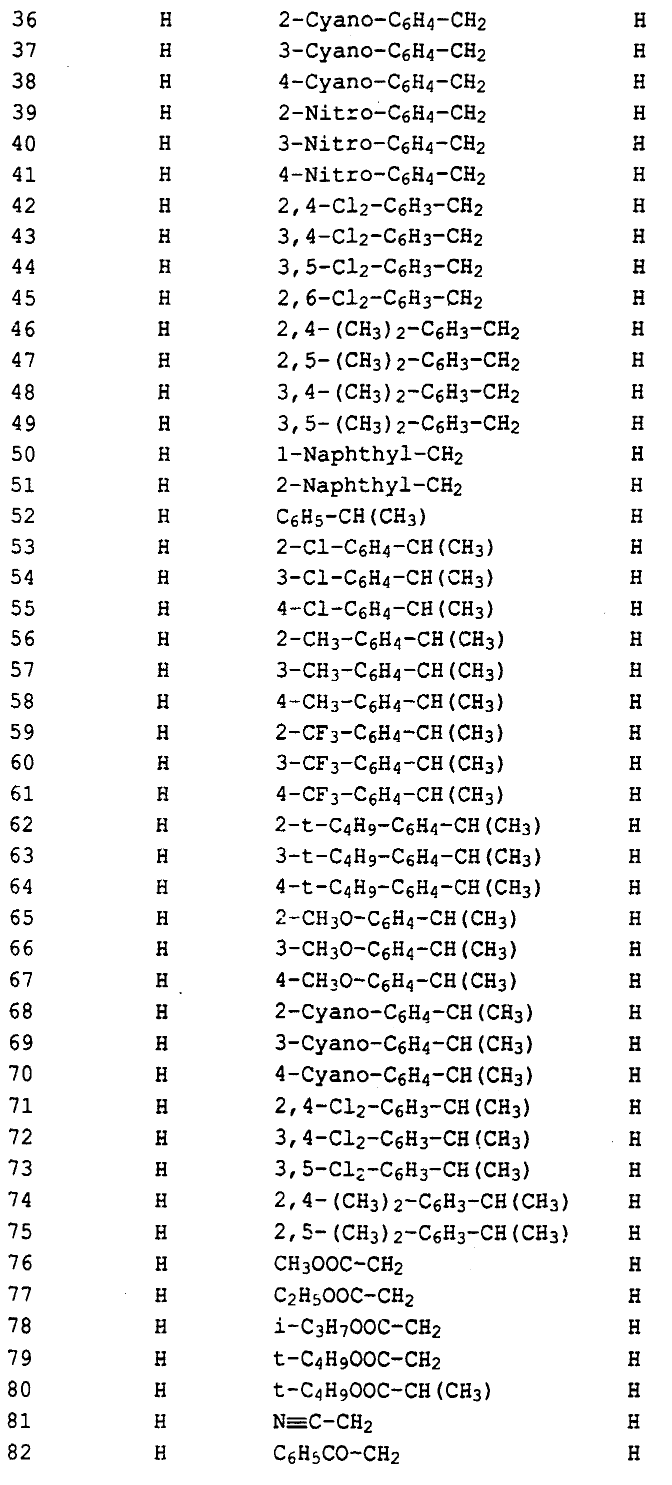

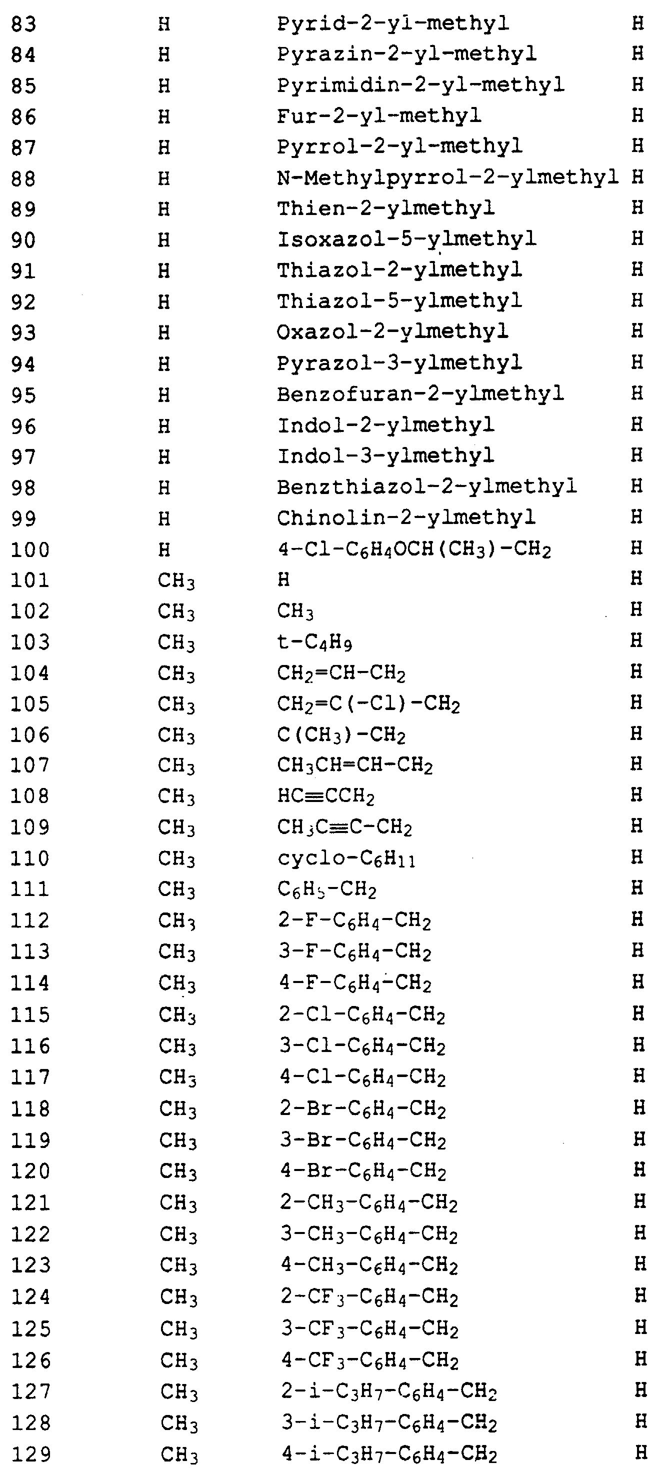

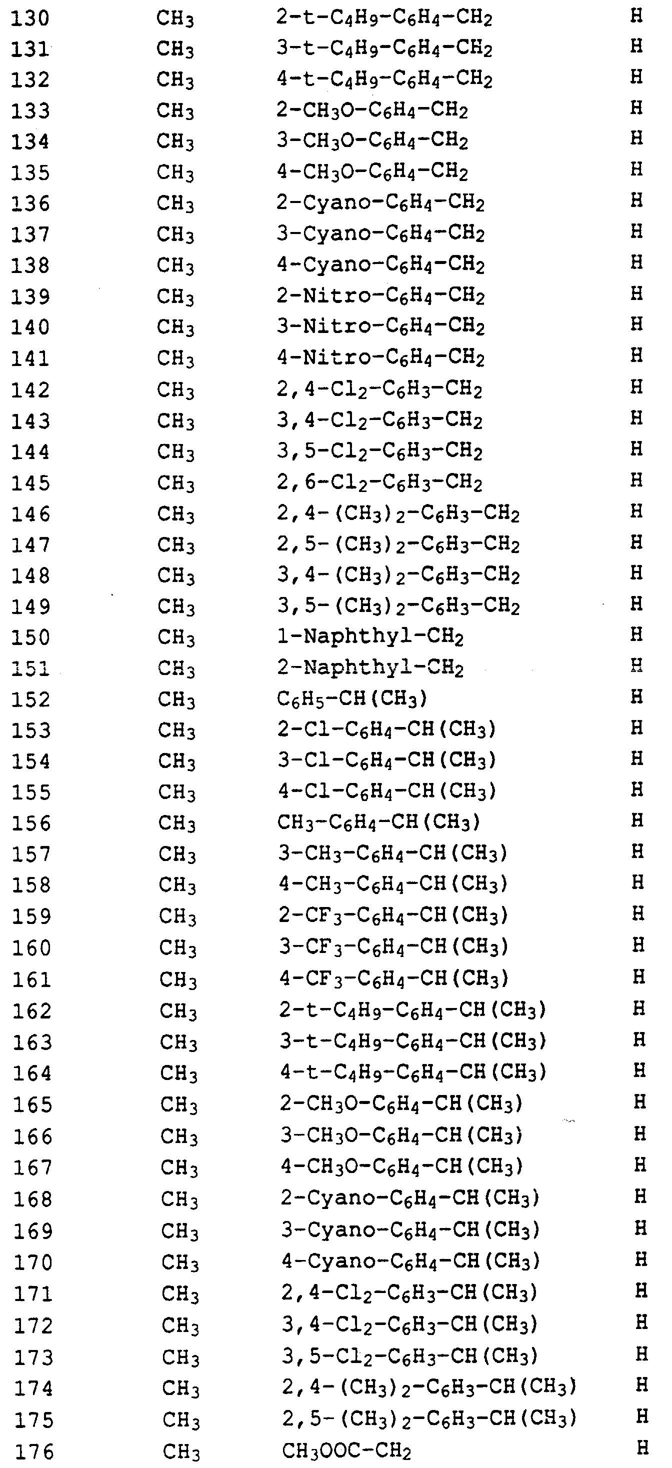

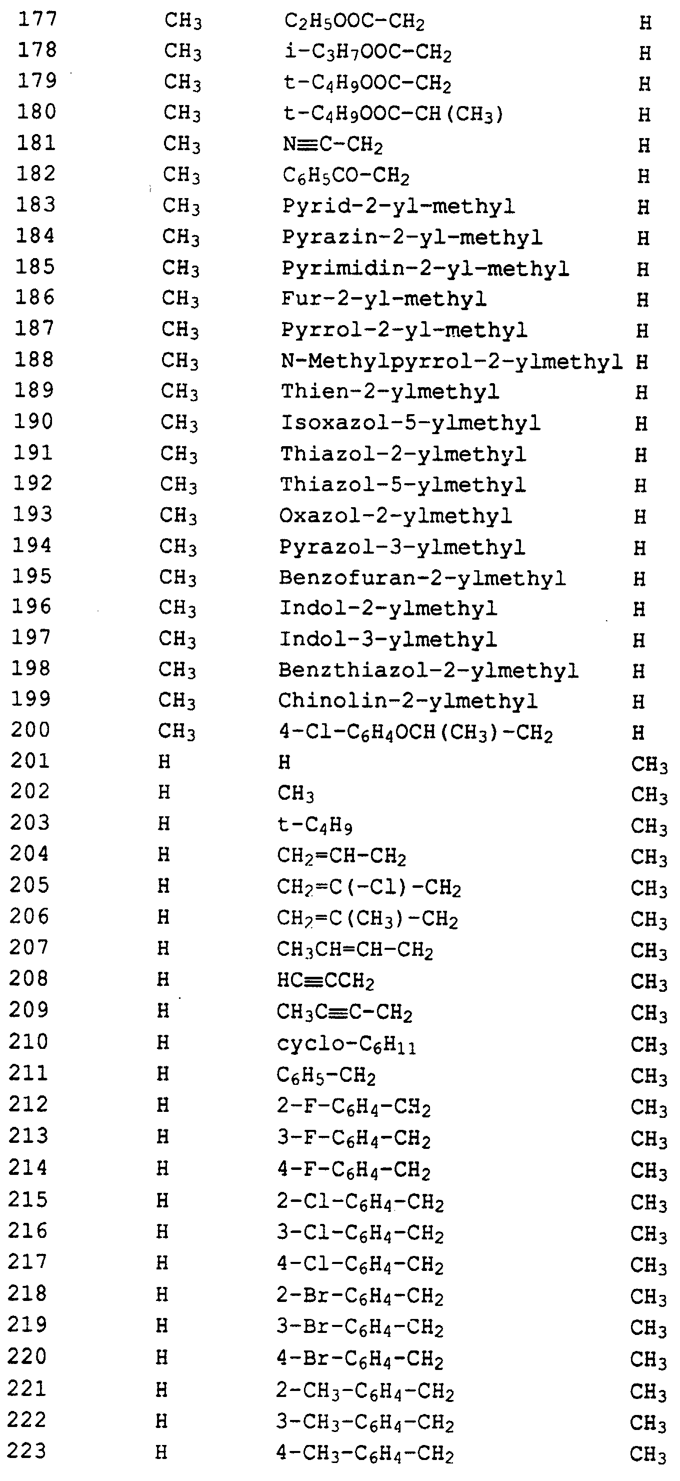

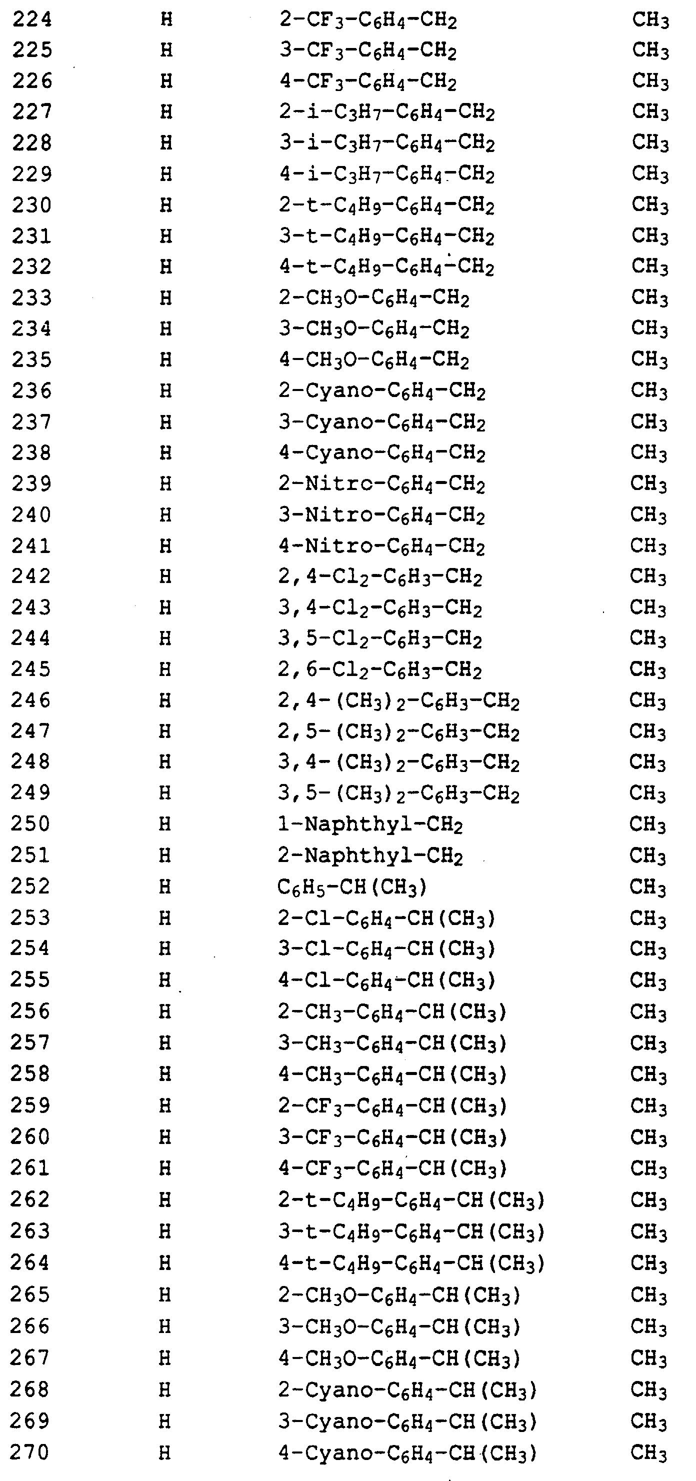

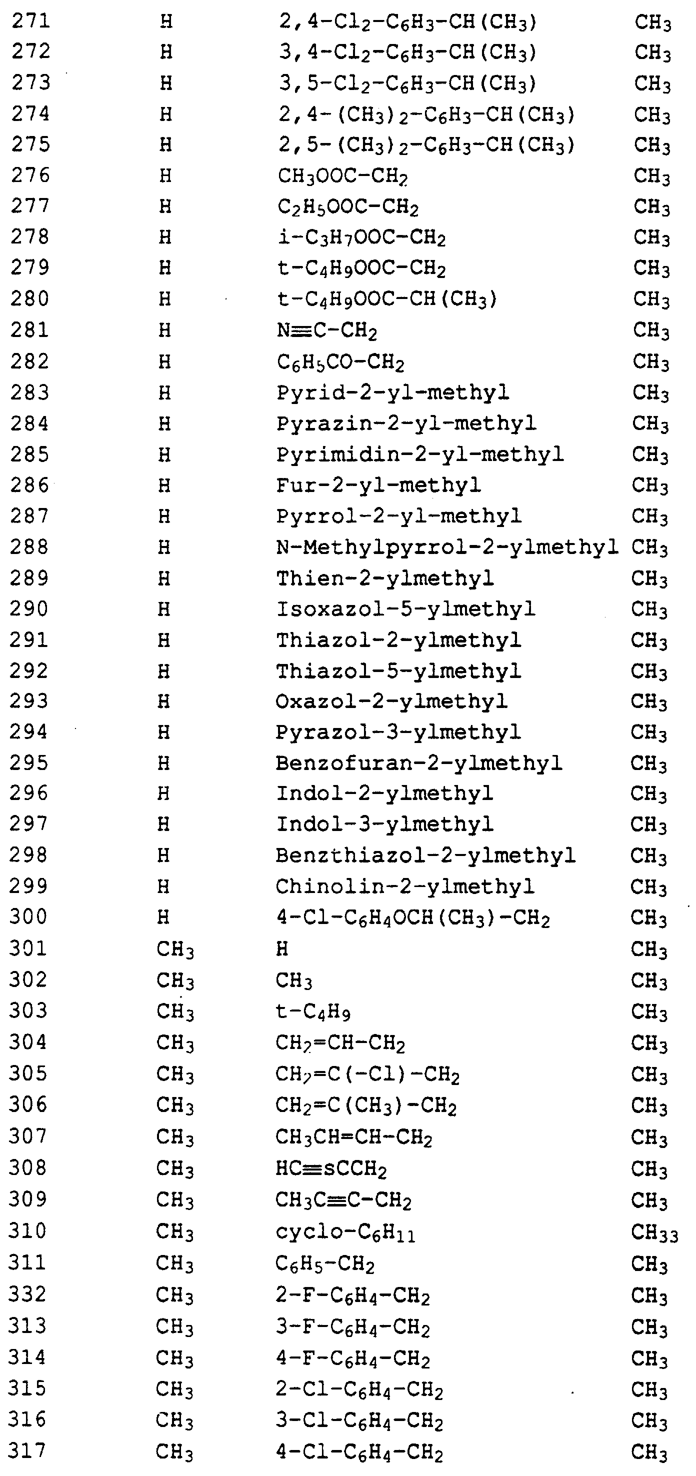

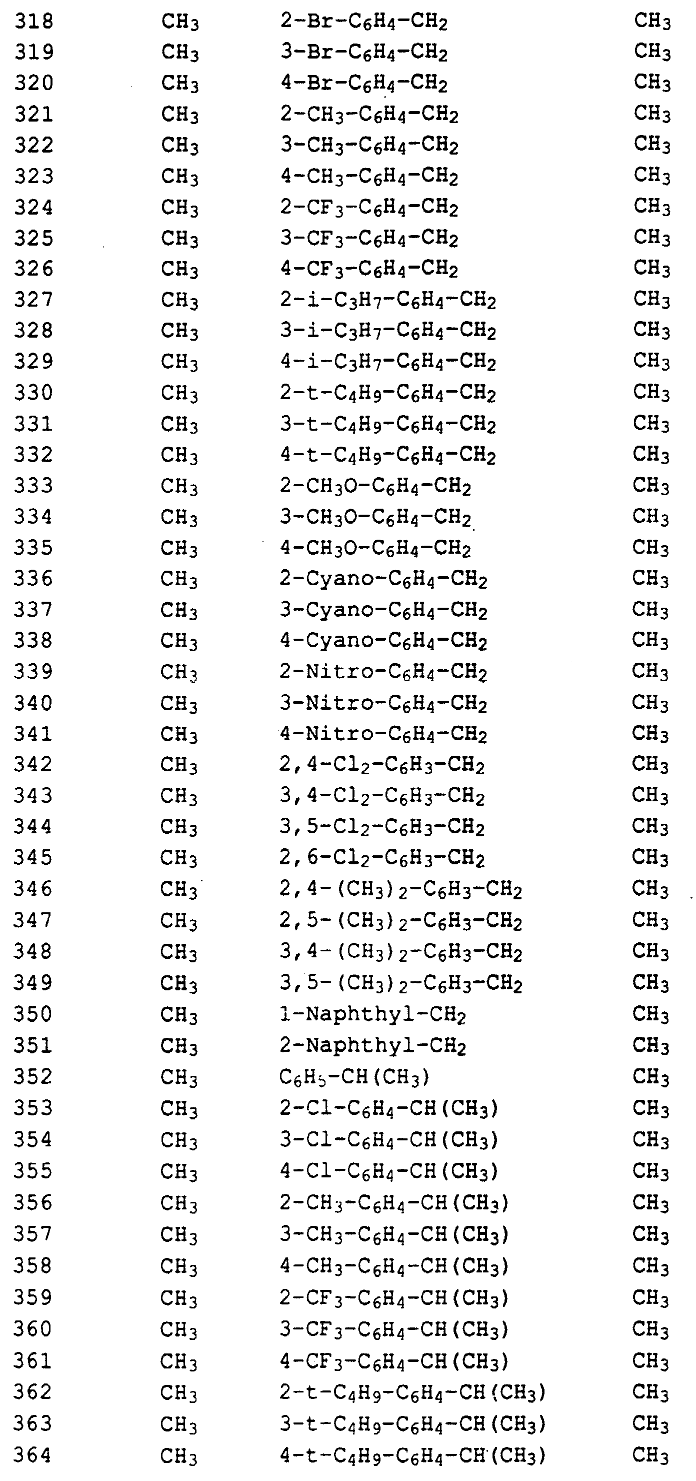

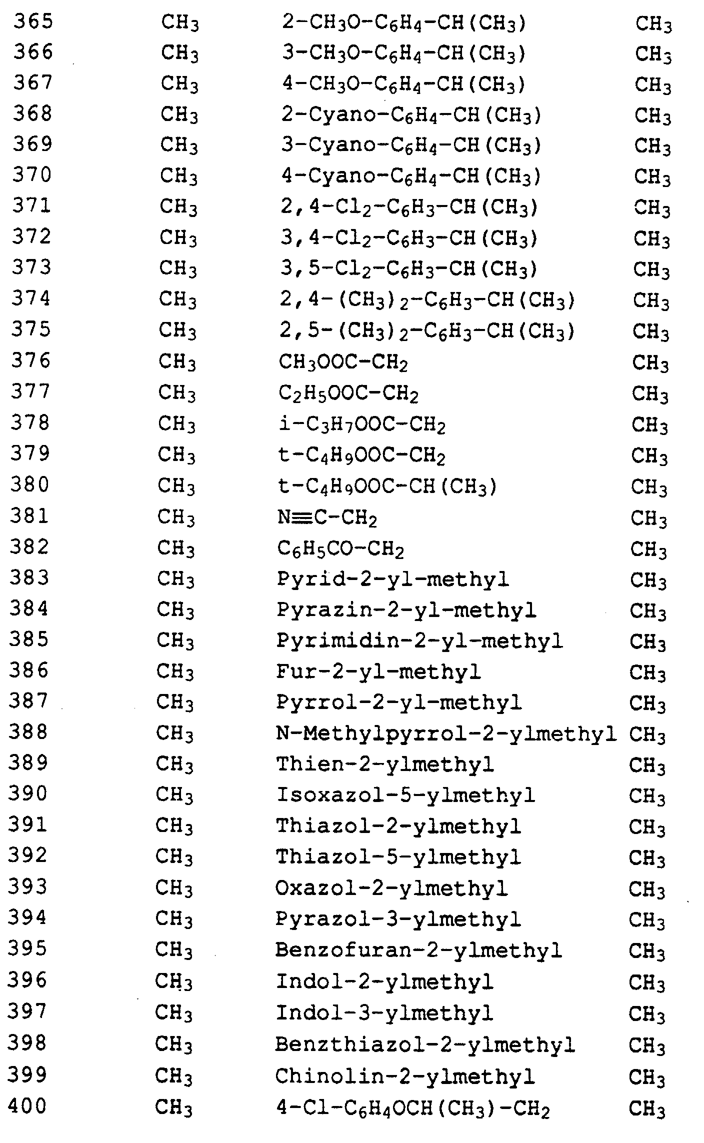

Die neuen Verbindungen der Formel I eignen sich als Fungizide.

Die neuen Verbindungen, bzw. die sie enthaltenden Mittel können beispielsweise in Form von direkt versprühbaren Lösungen, Pulvern, Suspensionen, auch hochprozentigen wäßrigen, öligen oder " sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen der Verbindung Nr. 2 aus Tabelle 1, d.h. 1/2, und 10 Gew.-Teilen N-Methyl-α-pyrrolidon, die zur Anwendung in Form kleinster Tropfen geeignet ist;
II. eine Mischung aus 20 Gew.-Teilen der Verbindung Nr. 36 aus Tabelle 1, d.h. 1/36, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung Nr. 1/49, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung Nr. 1/58, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung Nr. 1/2, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Nr. 1/36 und 97 Gew.-Teilen feinteiligem Kaolin; dieses Stäubemittel enthält 3 Gew.-% Wirkstoff;
VII. eine innige Mischung aus 30 Gew.-Teilen der Verbindung Nr. 1/49, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Nr. 1/58, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 1/2, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
   Dithiocarbamate und deren Derivate, wie
   Ferridimethyldithiocarbamat,
   Zinkdimethyldithiocarbamat,
   Zinkethylenbisdithiocarbamat,
   Manganethylenbisdithiocarbamat,
   Mangan-Zink-ethylendiamin-bis-dithiocarbamat,
   Tetramethylthiuramdisulfide,
   Ammoniak-Komplex von Zink- (N, N-ethylen-bis-dithiocarbamat),
   Ammoniak-Komplex von Zink-(N,N'-propylen-bis-dithiocarbamat),
   Zink-(N,N'-propylen-bis-dithiocarbamat),
   N,N'-Polypropylen-bis-(thiocarbamoyl)-disulfid;
Nitroderivate, wie
   Dinitro-(1-methylheptyl)-phenylcrotonat,
   2-sec-Butyl-4,6-dinitrophenyl-3,3-dimethylacrylat,
   2-sec-Butyl-4,6-dinitrophenyl-isopropylcarbonat,
   5-Nitro-isophthalsäure-di-isopropylester;
heterocyclische Substanzen, wie
   2-Heptadecyl-2-imidazolin-acetat,
   2,4-Dichlor-6-(o-chloranilino)-s-triazin,
   O,O-Diethyl-phthalimidophosphonothioat,
   5-Amino-1-βbis-(dimethylamino)-phosphinyl'-3-phenyl-1,2,4-triazol,
   2,3-Dicyano-1,4-dithioanthrachinon,
   2-Thio-1,3-dithioloβ4,5-b'chinoxalin,
   1-(Butylcarbamoyl)-2-benzimidazol-carbaminsäuremethylester,
   2-Methoxycarbonylamino-benzimidazol,
   2-(Furyl-(2))-benzimidazol,
   2-(Thiazolyl-(4))-benzimidazol,
   N-(1,1,2,2-Tetrachlorethylthio)-tetrahydrophthalimid,
   N-Trichlormethylthio-tetrahydrophthalimid,
   N-Trichlormethylthio-phthalimid,
N-Dichlorfluormethylthio-N',N'-dimethyl-N-phenyl-schwefelsäurediamid,
   5-Ethoxy-3-trichlormethyl-1,2,3-thiadiazol,
   2-Rhodanmethylthiobenzthiazol,
   1,4-Dichlor-2,5-dimethoxybenzol,
   4-(2-Chlorphenylhydrazono)-3-methyl-5-isoxazolon,
   Pyridin-2-thio-1-oxid,
   8-Hydroxychinolin bzw. dessen Kupfersalz,
   2,3-Dihydro-5-carboxanilido-6-methyl-1,4-oxathiin,
   2,3-Dihydro-5-carboxanilido-6-methyl-1,4-oxathiin-4,4-dioxid,
   2-Methyl-5,6-dihydro-4H-pyran-3-carbonsäure-anilid,
   2-Methyl-furan-3-carbonsäureanilid,
   2,5-Dimethyl-furan-3-carbonsäureanilid,
   2,4,5-Trimethyl-furan-3-carbonsäureanilid,
   2,5-Dimethyl-furan-3-carbonsäurecyclohexylamid,
   N-Cyclohexyl-N-methoxy-2,5-dimethyl-furan-3-carbonsäureamid,
   2-Methyl-benzoesäure-anilid,
   2-Iod-benzoesäure-anilid,
   N-Formyl-N-morpholin-2,2,2-trichlorethylacetal,
   Piperazin-1,4-diylbis-(1-(2,2,2-trichlor-ethyl)-formamid,
   1-(3,4-Dichloranilino)-1-formylamino-2,2,2-trichlorethan,
   2,6-Dimethyl-N-tridecyl-morpholin bzw. dessen Salze,
   2,6-Dimethyl-N-cyclododecyl-morpholin bzw. dessen Salze,
N-[3-(p-tert.-Butylphenyl)-2-methylpropyl]-cis-2,6-dimethylmorpholin,
   N-[3-(p-tert.-Butylphenyl)-2-methylpropyl]-piperidin,
   1-[2-(2,4-Dichlorphenyl)-4-ethyl-1,3-dioxolan-2-yl-ethyl]-1H-1,2,4-triazol
   1-[2-(2,4-Dichlorphenyl)-4-n-propyl-1,3-dioxolan-2-yl-ethyl]-1H-1,2,4-triazol
   N-(n-Propyl)-N-(2,4,6-trichlorphenoxyethyl)-N'-imidazol-yl-harnstoff,
   1-(4-Chlorphenoxy)-3,3-dimethyl-1-(1H-1,2,4-triazol-1-yl)-2-butanon,
   α-(2-Chlorphenyl)-α-(4-chlorphenyl)-5-pyrimidin-methanol,
   5-Butyl-2-dimethylamino-4-hydroxy-6-methyl-pyrimidin,
   Bis-(p-chlorphenyl)-3-pyridinmethanol,
   1,2-Bis-(3-ethoxycarbonyl-2-thioureido)-benzol,
   1,2-Bis-83-methoxycarbonyl-2-thioureido)-benzol,
sowie verschiedene Fungizide, wie
   Dodecylguanidinacetat,
   3-[3-(3,5-Dimethyl-2-oxycyclohexyl)-2-hydroxyethyl)]glutarimid,
   Hexachlorbenzol,
   DL-Methyl-N-(2,6-dimethyl-phenyl)-N-furoyl(2)-alaninat,
   DL-N-(2,6-Dimethyl-phenyl)-N-(2'-methoxyacetyl)-alanin-methylester,
   N-(2,6-Dimethylphenyl)-N-chloracetyl-D,L-2-aminobutyrolacton,
   DL-N-(2,6-Dimethylphenyl)-N-(phenylacetyl)-alaninmethylester,
   5-Methyl-5-vinyl-3-(3,5-dichlorphenyl)-2,4-dioxo-1,3-oxazolidin,
   3-[3,5-Dichlorphenyl(-5-methyl-5-methoxymethyl]-1,3-oxazolidin-2,4-dion,
   3-(3,5-Dichlorhenyl)-1-isopropylcarbamoylhydantoin,
   N-(3,5-Dichlorphenyl)-1,2-dimethylcyclopropan-1,2-dicarbonsäureimid,
   2-Cyano-[N-(ethylaminocarbonyl)-2-methoximino]-acetamid,
   1-[2-(2,4-Dichlorphenyl)-pentyl]-1H-1,2,4-triazol,
   2,4-Difluor-α-(1H-1,2,4-triazolyl-1-methyl)-benzhydrylalkohol,
   N-(3-Chlor-2,6-dinitro-4-trifluormethyl-phenyl)-5-trifluormethyl-3-chlor-2-aminopyridin,
   1-((bis-(4-Fluorphenyl)-methylsilyl)-methyl)-1H-1,2,4-triazol.

### Anwendungsbeispiele

Als Vergleichswirkstoffe wurden 2-(Methoximino-O-[(3-trifluormethyl)-phenylmethyl]-phenylglyoxylsäure-methylester-O-methyloxim (A) und
2-(Methoximino-O-[(3-brom)-phenylmethyl]-phenylglyoxylsäuremethylester-O-methyloxim (B) - beide bekannt aus EP 499 823 - benutzt.

### Anwendungsbeispiel 1

### Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

In diesem Test zeigten die mit 16 ppm haltiger Aufbereitung der Verbindungen (Tabelle/Nr.) 1/32, 1/34, 1/49, 1/58, 1/114, 1/128, 1/134, 1/145 und 1/155 einen Befall von 15 % und weniger, während die unbehandelten Pflanzen zu 70 % befallen waren. Die in diesen Test mit 16 ppm haltigen Aufbereitungen der bekannten Wirkstoffe A und B behandelten Pflanzen waren zu 35 % befallen.

### Anwendungsbeispiel 2

### Wirksamkeit gegen Pyrenophora teres

Gerstenkeimlinge der Sorte "Igri" wurden im Zweikeimblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 bis 22°C und 70 % relativer Luftfeuchtigkeit für weitere 5 Tage kultiviert. Dann wurde das Ausmaß des Blattbefalls ermittelt.

Das Ergebnis zeigt, daß die Wirkstoffe aus Tabelle 1, Nr. 49 und Nr. 58 bei der Anwendung als 63 ppm oder 16 ppm Wirkstoff enthaltende Spritzbrühe eine bessere fungizide Wirkung zeigen (5 oder 15 % Befall der Blätter) als die bekannten Vergleichswirkstoffe A und B (50 % Befall der Blätter).

### Anwendungsbeispiel 3

### Wirksamkeit gegen Fusarium culmorum an Weizen

Primär-Blätter von in Töpfen gewachsenem Weizen der Sorte "Kanzler" wurden mit wäßriger Spritzbrühe, die 80 % Wirkstoff und 20 % Emulgiermittel in der Trockensubstanz enthielt, tropfnaß bespritzt. Am folgenden Tage wurden sie mit einer Sporensuspension von Fusarium culmorum inokuliert und anschließend in eine Klimakammer mit hoher Luftfeuchtigkeit (>90 %) bei 22-24°C bestellt. Nach 6 Tagen wurde das Ausmaß der Symptomentwicklung visuell ausgewertet.

In diesem Test zeigten die mit 500 ppm haltiger Aufbereitung der Verbindungen (Tabelle/Nr.) 1/8, 1/23, 1/28, 1/29, 1/32, 1/34, 1/35, 1/36, 1/37, 1/58, 1/71, 1/83, 1/114 und 1/128 einen Befall von 15 % und weniger, während die unbehandelten Pflanzen zu 80 % befallen waren. Die in diesen Test mit 500 ppm haltiger Aufbereitung der bekannten Wirkstoffe A und B behandelten Pflanzen waren zu 80 % bzw. 60 % befallen.

## Patentansprüche

1. Verbindungen der Formel I, 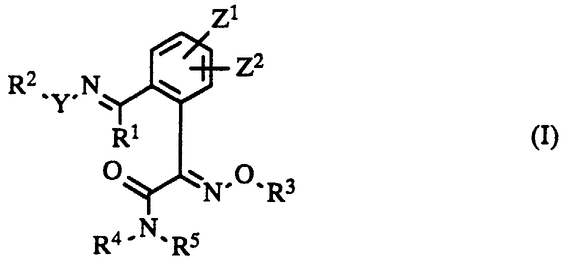 in der die Substituenten die folgende Bedeutung haben:
Y
Sauerstoff oder die Gruppe NR⁶;
Z¹ und Z²
unabhängig voneinander Wasserstoff, Halogen, Alkyl, Alkenyl, Alkinyl, Alkoxy, Alkenyloxy, Halogenalkyl, Halogenalkoxy, Halogenalkenyloxy, Cyano oder Nitro;
R¹
Wasserstoff, Alkyl, Halogenalkyl oder Aryl;
R²
Wasserstoff, oder ggf. subst. Alkyl, Alkenyl, Alkinyl, Cycloalkyl, Heteroarylalkyl, Heterocyclyl, Cycloalkenyl, Arylalkyl, Aryl, Heteroaryl, Arylalkenyl, Heteroarylalkenyl, Aryloxyalkyl, Heteroaryloxyalkyl, Acyl, Arylcarbonyl, Heteroarylcarbonyl oder Alkoxycarbonyl;
R³, R⁴, R⁵ und R⁶
unabhängig voneinander Wasserstoff oder Alkyl;
oder R⁶ und R² gemeinsam mit dem Stickstoff, dessen Substituenten sie sind, einen Ring bedeuten können.

2. Fungizid, enthaltend einen inerten Trägerstoff und eine fungizid wirksame Menge einer Verbindung der Formel I gemäß Anspruch 1.

3. Verfahren zur Bekämpfung von Pilzen, dadurch gekennzeichnet, daß man die Pilze oder die von Pilzbefall bedrohten Materialien, Pflanzen, Saatgut oder den Erdboden behandelt mit einer fungizid wirksamen Menge einer Verbindung der Formel I gemäß Anspruch 1.

4. Verfahren zur Herstellung von Verbindungen der Formel I gemäß Anspruch 1, gekennzeichnet dadurch, daß man einen Ester der Formel II (mit R⁷=Alkyl) 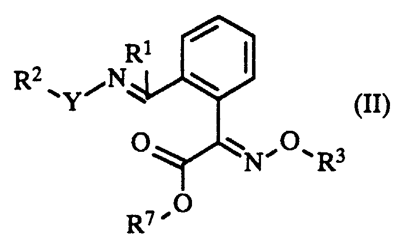 mit einem Amin der Formel III 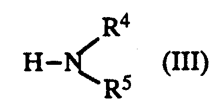 oder Ammoniak umsetzt.

5. Verfahren zur Herstellung von Verbindungen der Formel I gemäß Anspruch 1, in der R⁵ Wasserstof bedeutet, gekennzeichnet dadurch, daß man ein Gemisch aus dem cyclischen Halbaminal VI und der Carbonylverbindung VII 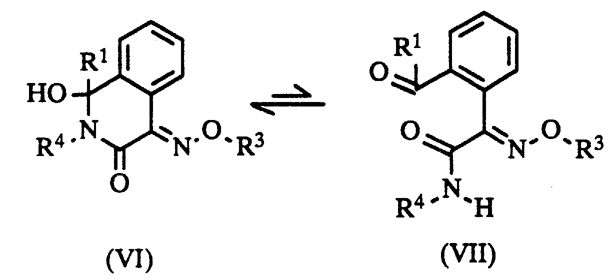 mit einem Hydroxylamin- oder Hydrazinderivat VIII (Y=O, NR⁶)
R²―Y―NH₂ (VIII)
oder einem seiner Säureadditionssalze umsetzt.

6. Verbindung der Formel VI gemäß Anspruch 5.

7. Verfahren zur Herstellung einer Verbindung der Formel VI gemäß Anspruch 5, gekennzeichnet dadurch, daß man einen Ester der Formel IV (R⁷=Alkyl) 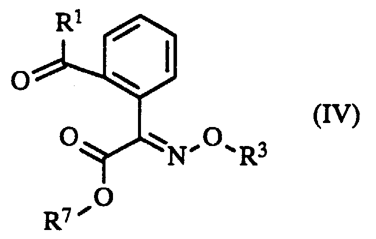 mit einem primären Amin der Formel V
H₂N-R⁴ (V)
umsetzt.

8. Verfahren zur Herstellung einer Verbindung der Formel VI gemäß Anspruch 5 mit R¹ = H, gekennzeichnet dadurch, daß man ein α-Alkoximino-phenylessigsäuremonoalkylamid der Formel X 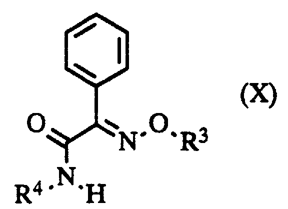 (mit R³ = Alkyl) in ein zwei Metallreste enthaltendes Metallierungsprodukt überführt und das Metallierungsprodukt mit einem Carbonsäurederivat der allgemeinen Formel XII 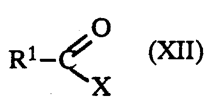 (X=Cl, OCOR⁷, OR⁷ mit R⁷= Alkyl, Aryl) umsetzt.

9. Verfahren zur Herstellung einer Verbindung der Formel VI gemäß Anspruch 5, gekennzeichnet dadurch, daß man ein α-Alkoximino-phenylessigsäure-monoalkylamid der Formel X gemäß Anspruch 7 (mit R³ = Alkyl) in ein zwei Metallreste enthaltendes Metallierungsprodukt überführt, das Metallierungsprodukt mit dem Aldehyd XIII 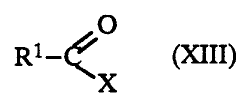 umsetzt und das entstandene Carbinol XIV 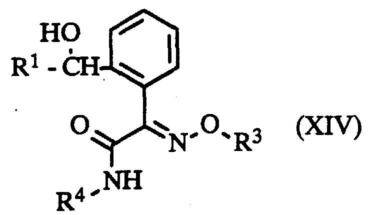 oxidiert.

10. Verbindung der Formel X gemäß Anspruch 8, in der R³ und R⁴ Alkyl bedeuten.

11. Verbindung der Formel XIV gemäß Anspruch 9, in der R³ und R⁴ Alkyl bedeuten und R¹ die in Anspruch 1 genannten Bedeutungen hat.

## Claims

1. A compound of the formula I 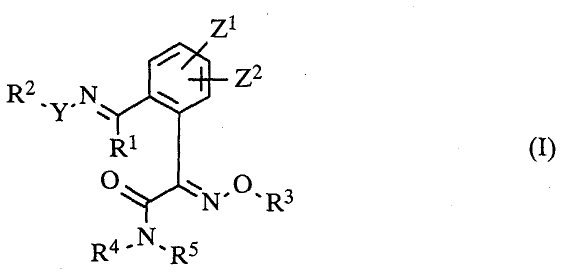 where the substituents have the following meanings:
Y
is oxygen or the group NR⁶;
Z¹ and Z²
are independently of one another hydrogen, halogen, alkyl, alkenyl, alkynyl, alkoxy, alkenyloxy, haloalkyl, haloalkoxy, haloalkenyloxy, cyano or nitro;
R¹
is hydrogen, alkyl, haloalkyl or aryl;
R²
is hydrogen, or unsubstituted or substituted alkyl, alkenyl, alkynyl, cycloalkyl, heteroarylalkyl, heterocyclyl, cycloalkenyl, arylalkyl, aryl, heteroaryl, arylalkenyl, heteroarylalkenyl, aryloxyalkyl, heteroaryloxyalkyl, acyl, arylcarbonyl, heteroarylcarbonyl or alkoxycarbonyl;
R³, R⁴, R⁵ and R⁶
are independently of one another hydrogen or alkyl;
or R⁶ and R², together with the nitrogen whose substituents they are, can be a ring.

2. A fungicide, containing an inert carrier and a fungicidally active amount of a compound of the formula I as claimed in claim 1.

3. A process for the control of fungi, which comprises treating the fungi or the materials, plants, seeds or the soil threatened by fungal attack with a fungicidally active amount of a compound of the formula I as claimed in claim 1.

4. A process for preparing compounds of the formula I as claimed in claim 1, which comprises reacting an ester of the formula II (where R⁷=alkyl) 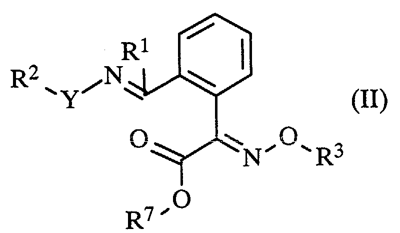 with an amine of the formula III 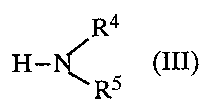 or ammonia.

5. A process for preparing compounds of the formula I as claimed in claim 1, in which R⁵ is hydrogen, which comprises reacting a mixture of the cyclic hemiaminal VI and the carbonyl compound VII 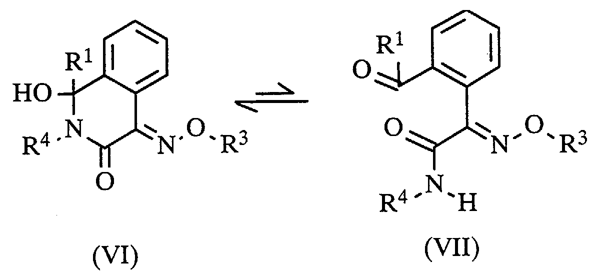 with a hydroxylamine or hydrazine derivative VIII (Y=O, NR⁶)
R²―Y―NH₂ (VIII)
or one of its acid addition salts.

6. A compound of the formula VI as in claim 5.

7. A process for preparing a compound of the formula VI as in claim 5, which comprises reacting an ester of the formula IV (R⁷=alkyl) 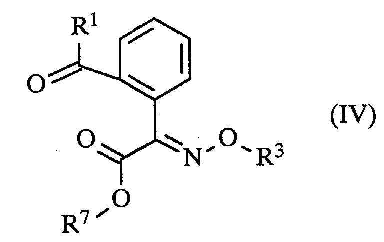 with a primary amine of the formula V
H₂N-R⁴ (V).

8. A process for preparing a compound of the formula VI as in claim 5 where R¹ = H, which comprises converting a monoalkyl α-alkoximinophenylacetamide of the formula X 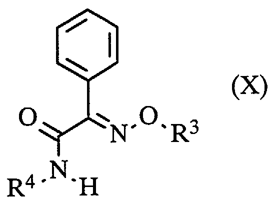 (where R³ = alkyl) into a metalation product containing two metal radicals and reacting the metalation product with a carboxylic acid derivative of the general formula XII 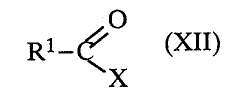 (X=Cl, OCOR⁷ or OR⁷ where R⁷= alkyl or aryl).

9. A process for preparing a compound of the formula VI as in claim 5, which comprises converting a monoalkyl α-alkoximinophenylacetamide of the formula X as in claim 8 (where R³ = alkyl) into a metalation product containing two metal radicals, reacting the metalation product with the aldehyde XIII 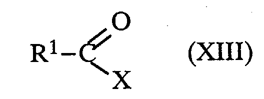 and oxidizing the resultant carbinol XIV

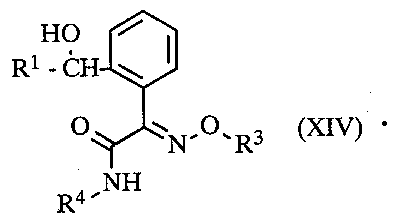

10. A compound of the formula X as in claim 8, where R³ and R⁴ are alkyl.

11. A compound of the formula XIV as in claim 9, where R³ and R⁴ are alkyl and R¹ has the meanings mentioned in claim 1.

## Revendications

1. Composés de formule I 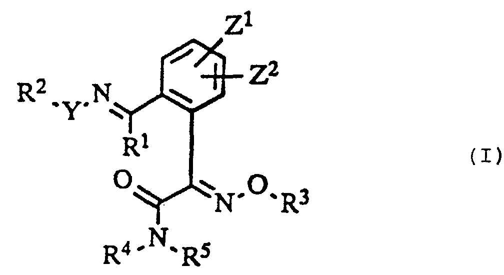 dans laquelle les symboles ont les significations suivantes :
Y
l'oxygène ou le groupe NR⁶ ;
Z¹ et Z²,
indépendamment l'un de l'autre, l'hydrogène, un halogène, un groupe alkyle, alcényle, alcynyle, alcoxy, alcényloxy, halogénoalkyle, halogénoalcoxy, halogénoalcényloxy, cyano ou nitro ;
R¹
l'hydrogène, un groupe alkyle, halogénoalkyle ou aryle ;
R²
l'hydrogène ou un groupe alkyle, alcényle, alcynyle, cycloalkyle, hétéroarylalkyle, hétérocyclyle, cycloalcényle, arylalkyle, aryle, hétéroaryle, arylalcényle, hétéroarylalcényle, aryloxyalkyle, hétéroaryloxyalkyle, acyle, arylcarbonyle, hétéroarylcarbonyle ou alcoxycarbonyle éventuellement substitué ;
R³, R⁴, R⁵ et R⁶,
indépendamment les uns des autres, l'hydrogène ou des groupes alkyle ;
ou bien R⁶ et R² peuvent former un cycle avec l'azote dont ils sont substituants.

2. Produit fongicide contenant un véhicule inerte et une quantité fongicide efficace d'un composé de formule I selon la revendication 1.

3. Procédé pour combattre les mycètes, caractérisé par le fait que l'on traite les mycètes ou les matières, végétaux, semences ou sols menacés d'une attaque par les mycètes par une quantité fongicide efficace d'un composé de formule I selon la revendication 1.

4. Procédé de préparation des composés de formule I selon revendication 1, caractérisé par le fait que l'on fait réagir un ester de formule II (dans laquelle R⁷ = alkyle) 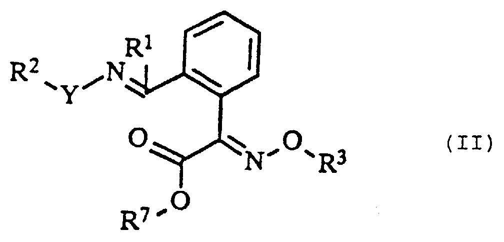 avec une amine de formule III 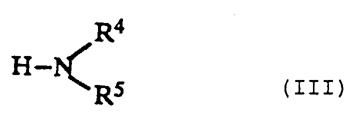 ou avec l'ammoniac.

5. Procédé de préparation des composés de formule I selon la revendication 1 dans laquelle R⁵ représente l'hydrogène, caractérisé par le fait que l'on fait réagir un mélange de l'hémi-aminal cyclique VI et du dérivé carbonylé VII 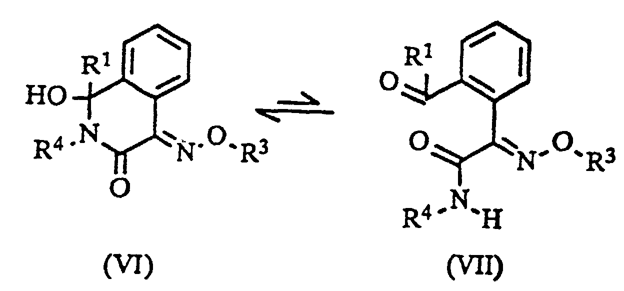 avec un dérivé de l'hydroxylamine ou de l'hydrazine VIII (Y=0, NR⁶)
R²―Y―NH₂ (VIII)
ou l'un de ses sels formés par addition avec un acide.

6. Composé de formule VI selon la revendication 5.

7. Procédé de préparation d'un composé de formule VI selon la revendication 5, caractérisé par le fait que l'on fait réagir un ester de formule IV (R⁷=alkyle) 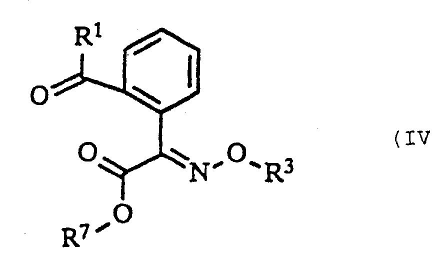 avec une amine primaire de formule V
H₂N-R⁴ (V)

8. Procédé de préparation d'un composé de formule VI selon la revendication 5, pour lequel R¹ = H, caractérisé par le fait que l'on convertit un monoalkylamide d'acide alpha-alcoxyimino-phénylacétique de formule X 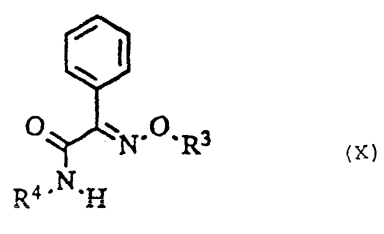 (dans laquelle R³ = alkyle) en un produit de métallisation contenant deux groupes métalliques, et on fait réagir le produit de métallisation avec un dérivé d'acide carboxylique de formule générale XII 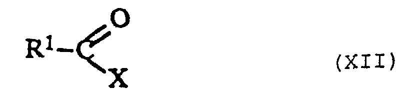 (dans laquelle X=Cl, OCOR⁷, OR⁷ et R⁷ = alkyle, aryle).

9. Procédé de préparation d'un composé de formule VI selon la revendication 5, caractérisé par le fait que l'on convertit un monoalkylamide d'acide alphaalcoxyiminophénylacétique de formule X selon la revendication 7 (dans laquelle R³ = alkyle) en un produit de métallisation contenant deux groupes métalliques, on fait réagir le produit de métallisation avec l'aldéhyde XIII 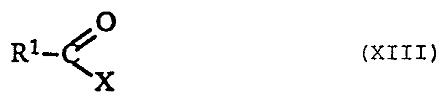 ce qui donne le carbinol XIV 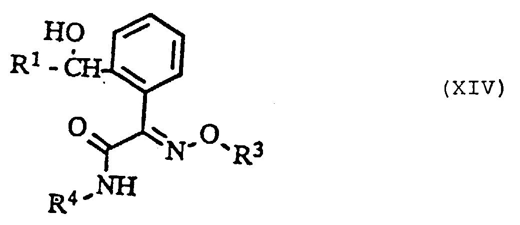 qu'on oxyde.

10. Composé de formule X selon la revendication 8, dans laquelle R³ et R⁴ représentent des groupes alkyle.

11. Composé de formule XIV selon la revendication 9, dans laquelle R³ et R⁴ représentent des groupes alkyle et R¹ a les significations indiquées dans la revendication 1.
